# EUROPEAN PATENT SPECIFICATION

(11) **EP 3 383 372 B1**
(45) Date of publication and mention of the grant of the patent: **01.11.2023**
(21) Application number: 16816486.1
(22) Date of filing: 22.11.2016
(51) Int. Cl.: A61K 9/16, A61K 9/20, A61K 31/506, A61P 9/00

(54) **SOLID DISPERSIONS COMPRISING A SGC STIMULATOR**
FESTE DISPERSIONEN MIT EINEM SGC-STIMULATOR
DISPERSIONS SOLIDES COMPRENANT UN STIMULATEUR DE GUANYLATE CYCLASE SOLUBLE (SGC)

(30) Priority: 30.11.2015 US 201562260910 P; 07.07.2016 US 201662359440 P
(43) Date of publication of application: 10.10.2018
(73) Proprietor: Cyclerion Therapeutics, Inc., Cambridge, MA 02142 (US)
(72) Inventor: DUNBAR, Craig Anthony, Needham Massachusetts 02494 (US); SETHURAMAN, Vasu, Waltham Massachusetts 02451 (US); HASHASH, Ahmad, Southborough Massachusetts 01772 (US)
(74) Representative: Elkington and Fife LLP
(86) International application number: PCT/US2016/063312
(87) International publication number: WO 2017/095697

(56) References cited:
- WO-A2-2014/144100
- TIWARI R ET AL: "Solid dispersions: An overview to modify bioavailability of poorly water soluble drugs", INTERNATIONAL JOURNAL OF PHARMTECH RESEARCH, SHPINX KNOWLEDGE HOUSE, IN, vol. 1, no. 4, 1 October 2009 (2009-10-01) , pages 1338-1349, XP009137553, ISSN: 0974-4304
- LEUNER C ET AL: "Improving drug solubility for oral delivery using solid dispersions", EUROPEAN JOURNAL OF PHARMACEUTICS AND BIOPHARMACEUTICS, ELSEVIER SCIENCE PUBLISHERS B.V., AMSTERDAM, NL, vol. 50, no. 1, 3 July 2000 (2000-07-03), pages 47-60, XP004257179, ISSN: 0939-6411, DOI: 10.1016/S0939-6411(00)00076-X
- DWAYNE T. FRIESEN ET AL: "Hydroxypropyl Methylcellulose Acetate Succinate-Based Spray-Dried Dispersions: An Overview", MOLECULAR PHARMACEUTICS, vol. 5, no. 6, 1 December 2008 (2008-12-01), pages 1003-1019, XP055056966, ISSN: 1543-8384, DOI: 10.1021/mp8000793
- BROADHEAD J ET AL: "THE SPRAY DRYING OF PHARMACEUTICALS", DRUG DEVELOPMENT AND INDUSTRIAL PHARMACY, NEW YORK, NY, US, vol. 18, no. 11/12, 1 January 1992 (1992-01-01), pages 1169-1206, XP009030193, ISSN: 0363-9045, DOI: 10.3109/03639049209046327

## Description

### TECHNICAL FIELD OF THE INVENTION

The present invention relates to solid dispersions of amorphous 1,1,1,3,3,3-hexafluoro-2-(((5-fluoro-2-(1-(2-fluorobenzyl)-5-(isoxazol-3-yl)-1H-pyrazol-3-yl)pyrimidin-4-yl)amino)methyl)propan-2-ol (**Compound I**) or a pharmaceutically acceptable salt thereof, as described in present claim 1.

It also relates to pharmaceutical compositions and pharmaceutical oral dosage unit forms comprising them, and their uses thereof.

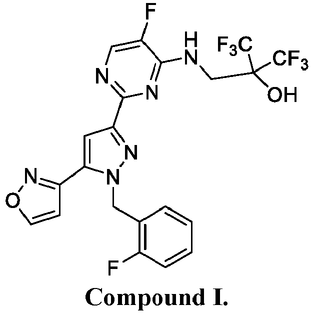

### BACKGROUND OF THE INVENTION

The heme-containing enzyme soluble guanylate cyclase (sGC) is the primary receptor for nitric oxide (NO) *in vivo.* sGC can be activated *via* both NO-dependent and NO-independent mechanisms. In response to this activation, sGC converts guanosine triphosphate (GTP) into the secondary messenger cyclic guanosine mono phosphate (cGMP). The increased level of cGMP, in turn, modulates the activity of downstream effectors including protein kinases, phosphodiesterases (PDEs) and ion channels.

Experimental and clinical evidence indicate that reduced bioavailability of NO and/or diminished responsiveness to endogenously produced NO contributes to the development of vascular, endothelial, inflammatory, fibrotic and metabolic processes or diseases. Impairment of sGC and/or reduced NO bioavailability have been implicated in the pathogenesis of, for example, cardiovascular, pulmonary, renal and hepatic diseases.

In the body, NO is synthesized from arginine and oxygen by various nitric oxide synthase enzymes (NOS) and by sequential reduction of inorganic nitrate. Three distinct isoforms of NOS have been identified: inducible NOS (i-NOS or NOS II) found in activated macrophage cells; constitutive neuronal NOS (nNOS or NOS I), involved in neurotransmission and long term potentiation; and constitutive endothelial NOS (eNOS or NOS III) which regulates smooth muscle relaxation and blood pressure.

NO-independent, heme-dependent, sGC stimulators, such as Compound I, are molecules that stimulate soluble guanylate cyclase activity and have several important differentiating characteristics, compared to activators, including crucial dependency on the presence of the reduced prosthetic heme moiety for their activity, strong synergistic enzyme activation when combined with NO and stimulation of the synthesis of cGMP by direct stimulation of sGC, independent of NO. The benzylindazole compound YC-1 was the first sGC stimulator to be identified. Additional sGC stimulators with improved potency and specificity for sGC have since been developed.

Adempas^{®} (riociguat) was the first sGC stimulator drug to receive FDA regulatory approval. It is currently approved for the treatment of chronic thromboembolic pulmonary hypertension (CTEPH) and pulmonary arterial hypertension (PAH). Adempas^{®} is taken orally three times a day and has to be very carefully titrated due to the variable risk of serious adverse events such as hypotension (low blood pressure), depending on the patient.

Accordingly, there is a need for new and improved sGC stimulators and their pharmaceutical compositions and dosage unit forms that circumvent some of these issues. Furthermore, there is a need for methods of treatment of NO and cGMP related diseases or disorders using said stimulators, their pharmaceutical compositions and dosage unit forms.

### SUMMARY OF THE INVENTION

In a first aspect, the present invention relates to a solid dispersion of amorphous 1,1,1,3,3,3-hexafluoro-2-(((5-fluoro-2-(1-(2-fluorobenzyl)-5-(isoxazol-3-yl)-1H-pyrazol-3-yl)pyrimidin-4-yl)amino)methyl)propan-2-ol (Compound I) or a pharmaceutically acceptable salt thereof, as described in present claim 1.

Compound I has the structure depicted below and is a sGC stimulator useful for treating or lessening the severity of a number of NO and/or cGMP related disorders.

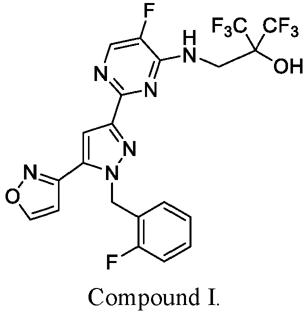

In a second aspect, the present invention relates to a pharmaceutical composition comprising at least one pharmaceutically acceptable excipient and a solid dispersion of amorphous 1,1,1,3,3,3-hexafluoro-2-(((5-fluoro-2-(1-(2-fluorobenzyl)-5-(isoxazol-3-yl)-1H-pyrazol-3-yl)pyrimidin-4-yl)amino)methyl)propan-2-ol (Compound I), or a pharmaceutically acceptable salt thereof, as described in present claim 1.

In a third aspect, the present invention relates to a dosage unit form, comprising a solid dispersion of amorphous 1,1,1,3,3,3-hexafluoro-2-(((5-fluoro-2-(1-(2-fluorobenzyl)-5-(isoxazol-3-yl)-1H-pyrazol-3-yl)pyrimidin-4-yl)amino)methyl)propan-2-ol (Compound I), or a pharmaceutically acceptable salt thereof, as described in present claim 1.

In a fourth aspect, the present invention relates to a dosage unit form, comprising a pharmaceutical composition described herein.

In a fifth aspect, the present invention relates to methods of treating sGC and/or cGMP related disorders by administering one of said solid amorphous dispersion, pharmaceutical composition or dosage unit form to a patient in need thereof.

Any references in the description to methods of treatment refer to the compounds, pharmaceutical compositions and medicaments of the present invention for use in a method for treatment of the human (or animal) body by therapy.

### BRIEF DESCRIPTION OF THE DRAWINGS

FIG. 1 shows an XRPD pattern for a spray-dried dispersion of Compound I of the invention with no polymer after two hours of production superposed over an XRPD pattern of neat crystalline Compound I.
FIG. 2 illustrates an XRPD pattern for a spray-dried dispersion embodiment of the invention with CAP polymer under various conditions.
FIG. 3 shows an XRPD pattern for a spray-dried dispersion embodiment of the invention with HRMCAS-M polymer under various conditions.
FIG. 4 illustrates XRPD patterns for various spray-dried dispersions of the invention at the time of preparation.
FIG. 5 illustrates XRPD patterns for a spray-dried dispersion embodiment of the invention at various time points under various conditions.
FIG. 6 illustrates XRPD patterns for a spray-dried dispersion embodiment of the invention at various time points under various conditions.
FIG. 7 illustrates XRPD patterns for various spray-dried dispersions of the invention at the time of production (time 0).
FIG. 8 is a chart showing the kinetic solubility of various dispersions of the invention in FaSSIF.
FIG. 9 is a chart showing the kinetic solubility of various dispersions of the invention in FeSSIF.
FIG. 10 illustrates the dissolution of various tablets containing amorphous spray dried dispersions of Compound I in FaSSIF.
FIG. 11 shows the dissolution of a batch of 5 mg tablets produced as described in an embodiment of the invention.

### DETAILED DESCRIPTION OF THE INVENTION

In a first aspect, the present invention relates to a solid dispersion of amorphous 1,1,1,3,3,3-hexafluoro-2-(((5-fluoro-2-(1-(2-fluorobenzyl)-5-(isoxazol-3-yl)-1H-pyrazol-3-yl)pyrimidin-4-yl)amino)methyl)propan-2-ol (Compound I) or a pharmaceutically acceptable salt thereof, as described in present claim 1.

Compound I has the structure depicted below and is a sGC stimulator useful for treating or lessening the severity of a number of NO and/or cGMP related disorders.

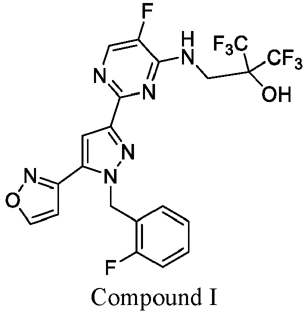

For purposes of this disclosure, "compound" includes pharmaceutically acceptable salts of the compound, even if the term "pharmaceutically acceptable salt" is not explicitly noted.

The phrase "pharmaceutically acceptable salt," as used herein, refers to pharmaceutically acceptable organic or inorganic salts of Compound I. The pharmaceutically acceptable salts of Compound I are used in medicine. Salts that are not pharmaceutically acceptable may, however, be useful in the preparation of Compound I or of its pharmaceutically acceptable salts. A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ions.

Pharmaceutically acceptable salts of Compound I include those derived from the compounds with inorganic or organic acids or bases. In some embodiments, the salts can be prepared in situ during the final isolation and purification of the compounds. In other embodiments the salts can be prepared from the free form of the compound in a separate synthetic step.

Salts of Compound I may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Particular embodiments include citric, hydrobromic, hydrochloric, maleic, phosphoric, sulfuric and tartaric acids. Other exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)) salts.

The preparation of the pharmaceutically acceptable salts described above and other typical pharmaceutically acceptable salts is more fully described by Berg et al., "Pharmaceutical Salts," J. Pharm. Sci., 1977:66:1-19.

As used herein, the term "dispersion" refers to a system in which one substance, the "dispersed phase", is distributed in discrete units, throughout a second substance, "the continuous phase" or "the vehicle" or "the dispersant" or "the dispersion medium" or "the matrix" or "the carrier". The dispersed and the continuous phases may be in the same or different physical state and thus may be solids, liquids or gases. The size of the particles of the dispersed phase can vary and, accordingly, dispersions are classified as solutions, colloids or suspensions depending on the size of the dispersed phase particles. Some common types of dispersions are, for instance, solutions, aerosols, solid aerosols, gels, emulsions, foams and solid foams.

A "solid dispersion" is a dispersion in which both the dispersed phase and the matrix are solids. Usually, a solid active pharmaceutical ingredient (API), for example Compound I, is dispersed in a second, inert, carrier or vehicle, usually a polymer, forming a homogeneous mixture in the solid state. In some cases, the solid dispersion comprises a polymer as the dispersed phase and the drug or API as the continuous phase. More typically, the API is the dispersed phase and the polymer is the matrix. Solid dispersions have successfully been applied to improve the solubility, dissolution rates and consequently the bioavailability of poorly soluble drugs.

Solid dispersions are usually prepared by forming a homogeneous solution or melt of the two solid components, either alone or in the presence of other additives (i.e., pharmaceutically acceptable additives or excipients), and then solidifying the mixture by either cooling or removing the solvent. Different techniques may be used to achieve this step. For instance, the solvent may be removed by vacuum-drying, spray-drying, tray-drying, freeze-drying, lyophilization or similar procedures. In the case of melts, hot-melt extrusion may be used to prepare solid dispersions.

A solid dispersion of Compound I could, for example, be a co-precipitate or a co-melt of Compound I with at least one polymer carrier. A "co-precipitate" is the solid dispersion obtained after dissolving the drug (e.g., Compound I) and a polymer carrier in a solvent or solvent mixture followed by the removal of the solvent or solvent mixture. The solvent or solvent mixture can include organic solvents and super-critical fluids. A "co-melt" is the solid dispersion obtained after heating a drug (e.g., Compound I) and a polymer carrier to a melt, optionally in the presence of a solvent or solvent mixture, followed by mixing, removal of at least a portion of the solvent if applicable, and cooling to room temperature at a selected rate.

A "crystalline" solid is one in which the structural units are arranged in fixed geometric patterns or lattices, so that crystalline solids have rigid long range order. The structural units that constitute the crystal could be atoms, molecules or ions. Crystalline solids show definite melting points. A crystalline material displays sharp characteristic crystalline peak(s) in its X-ray powder diffraction (XRPD) pattern.

Other analytical techniques that can be used to determine the level of crystallinity of a solid (e.g., a solid dispersion) or the stability of its amorphous form comprise, for example, Differential Scanning Calorimetry (DSC) or microscopy.

The term "amorphous", as used herein, refers to a solid material having no long range order in the position of its ions, atoms or molecules characteristic of a crystal. In the case of an amorphous material, instead of sharp peaks, one or more broad peaks (or "halos") appear in its XRPD pattern.

In pharmaceutical applications, a solid dispersion may comprise a crystalline drug or API (dispersed phase) dispersed in an amorphous continuous phase (e.g., a polymer), or alternatively, an amorphous drug or API dispersed in an amorphous continuous phase (e.g., a polymer). An "amorphous solid dispersion" is one in which the dispersed phase is amorphous. Dispersions of the invention are solid dispersions of amorphous Compound I.

Amorphous solid dispersions of the invention comprise Compound I in its amorphous state. Compound I has low solubility in aqueous media and readily crystallizes out of solution. This effect is pH independent. It has been found that certain solid dispersions of amorphous Compound I, can maintain Compound I in a stable amorphous state, preventing crystallization and displaying improved solubility in aqueous media and other use environments compared to Compound I itself (i.e., neat Compound I). Solid amorphous dispersions of Compound I display enhanced physical stability and/or dissolution and/or solubility when compared with Compound I in the absence of the dispersion polymer.

In some embodiments, solid dispersions of amorphous Compound I of the invention comprise substantially amorphous Compound I and a polymer carrier as described in present claim 1. As used herein, the term "solid dispersion of amorphous Compound I" is used interchangeably with the term "solid amorphous dispersion of Compound I".

As used herein, the term "substantially amorphous Compound I" is used interchangeably with the term "amorphous Compound I substantially free of crystalline Compound I". In some embodiments, substantially amorphous Compound I comprises less than about 20 % crystalline Compound I, less than about 15 % crystalline Compound I, less than about 10 % crystalline Compound I, less than about 8 % crystalline Compound I, less than about 5 % crystalline Compound I, less than about 2 % crystalline Compound I, less than about 1% crystalline Compound I, or less than about 0.5 % crystalline Compound I. In other embodiments, amorphous Compound I is substantially free of crystalline Compound I as determined by XRPD, DSC or microscopy.

As used herein, the term "substantially free" is used to describe amorphous Compound I that contains less than about 20 % crystalline Compound I. In some embodiments, "substantially free" is used to describe amorphous Compound I that contains less than about 15 % crystalline Compound I. In some embodiments, "substantially free" is used to describe amorphous Compound I that contains less than about 10 % crystalline Compound I. In some embodiments, "substantially free" is used to describe amorphous Compound I that contains less than about 8 % crystalline Compound I. In some embodiments, "substantially free" is used to describe amorphous Compound I that contains less than about 5 % crystalline Compound I. In some embodiments, "substantially free" is used to describe amorphous Compound I that contains less than about 2 % crystalline Compound I. In some embodiments, "substantially free" is used to describe amorphous Compound I that contains less than about 1% crystalline Compound I. In some embodiments, "substantially free" is used to describe amorphous Compound I that contains less than about 0.5 % crystalline Compound I.

In some embodiments, solid dispersions of amorphous Compound I of the invention comprise less than about 20 % crystalline Compound I, less than about 15 % crystalline Compound I, less than about 10 % crystalline Compound I, less than about 8 % crystalline Compound I, less than about 5 % crystalline Compound I, less than about 2 % crystalline Compound I, less than about 1% crystalline Compound I, or less than about 0.5 % crystalline Compound I. In other embodiments, solid dispersions of amorphous Compound I are substantially free of crystalline Compound I as determined by XRPD, DSC or microscopy. In some embodiments, the solid dispersion of amorphous Compound I is a stable solid dispersion of amorphous Compound I.

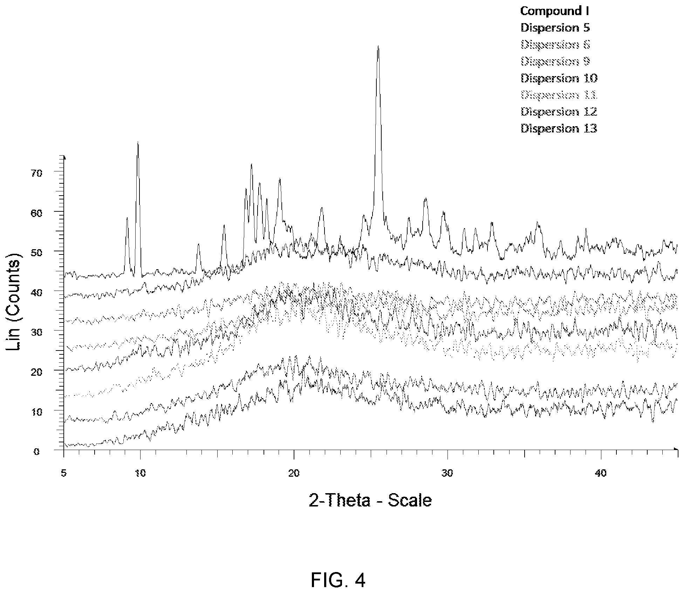

In some embodiments, solid dispersions of amorphous Compound I are characterized by an X-ray powder diffraction pattern substantially similar to that shown in Figure 4, for any one of dispersions 5 to 13 at time zero.

In some embodiments, solid amorphous dispersions of Compound I are characterized by the absence of any defined crystalline morphology when analyzed by microscopy.

In some embodiments, solid amorphous dispersions of Compound I are characterized by the absence of a recrystallization event or a melting event when analyzed by DSC.

The term "stable solid dispersion of amorphous Compound I", as used herein, refers to a solid dispersion of amorphous Compound I (i.e., substantially amorphous Compound I) that does not substantially increase the amount of crystalline Compound I when kept under certain storage conditions over a given period or length of time.

In some embodiments, these storage conditions involve a temperature of 25°C and a relative humidity (RH) of 60 %. Throughout this disclosure, these storage conditions are referred to as conditions of Stability Chamber 1. In other embodiments, storage conditions involve a temperature of 40°C and a relative humidity (RH) of 75 %. Throughout this disclosure, these storage conditions are referred to as conditions of Stability Chamber 2. In some embodiments, solid amorphous dispersions of Compound I are placed under the stability conditions of Stability Chamber 1 or Stability Chamber 2 in an unsealed vial (also referred to as open tray conditions). In other embodiments, solid amorphous dispersions of Compound I are placed under the stability conditions of Stability Chamber 1 or Stability Chamber 2 in a sealed bottle (for example a HDPE (high density polyethylene) bottle). In other embodiments, solid amorphous dispersions of Compound I are placed under the stability conditions of Stability Chamber 1 or Stability Chamber 2 in a sealed bottle (for example, a HDPE bottle) either in the presence or in the absence of a desiccant. In some embodiments, said desiccant is selected from silica gel. In other embodiments, the desiccant is selected from molecular sieves.

In some embodiments, stable solid dispersions of amorphous Compound I remain substantially free of crystalline Compound I for at least 12 months in Stability Chamber 1, when stored in a vial. In some embodiments, stable solid dispersions of amorphous Compound I remain substantially free of crystalline Compound I for at least 6 months in Stability Chamber 1 when stored in a vial. In other embodiments, stable solid dispersions of amorphous Compound I remain substantially free of crystalline Compound I for at least 4 months in Stability Chamber 1 when stored in a vial. In still other embodiments, stable solid dispersions of amorphous Compound I remain substantially free of crystalline Compound I for at least 3 months in Stability Chamber 1 when stored in a vial. In yet other embodiments, stable solid dispersions of amorphous Compound I remain substantially free of crystalline Compound I for at least 2 months in Stability Chamber 1 when stored in a vial. In some embodiments, stable solid dispersions of amorphous Compound I remain substantially free of crystalline Compound I for at least 1 month in Stability Chamber 1 when stored in a vial.

In some embodiments, stable solid dispersions of amorphous Compound I remain substantially free of crystalline Compound I for at least 12 months in Stability Chamber 1, when stored in a sealed bottle. In some embodiments, stable solid dispersions of amorphous Compound I remain substantially free of crystalline Compound I for at least 6 months in Stability Chamber 1 when stored in a sealed bottle. In other embodiments, stable solid dispersions of amorphous Compound I remain substantially free of crystalline Compound I for at least 4 months in Stability Chamber 1 when stored in a sealed bottle. In still other embodiments, stable solid dispersions of amorphous Compound I remain substantially free of crystalline Compound I for at least 3 months in Stability Chamber 1 when stored in a sealed bottle. In yet other embodiments, stable solid dispersions of amorphous Compound I remain substantially free of crystalline Compound I for at least 2 months in Stability Chamber 1 when stored in a sealed bottle. In some embodiments, stable solid dispersions of amorphous Compound I remain substantially free of crystalline Compound I for at least 1 month in Stability Chamber 1 when stored in a sealed bottle. In some embodiments, the solid amorphous dispersion of Compound I is stored in the presence of a desiccant. In other embodiments, it is stored in the absence of a desiccant.

In some embodiments, stable solid dispersions of amorphous Compound I remain substantially free of crystalline Compound I for at least 12 months in Stability Chamber 2, when stored in a vial. In some embodiments, stable solid dispersions of amorphous Compound I remain substantially free of crystalline Compound I for at least 6 months in Stability Chamber 2, when stored in a vial. In other embodiments, stable solid dispersions of amorphous Compound I remain substantially free of crystalline Compound I for at least 4 months in Stability Chamber 2, when stored in a vial. In still other embodiments, stable solid dispersions of amorphous Compound I remain substantially free of crystalline Compound I for at least 3 months in Stability Chamber 2, when stored in a vial. In some embodiments, stable solid dispersions of amorphous Compound I remain substantially free of crystalline Compound I for at least 2 months in Stability Chamber 2, when stored in a vial. In some embodiments, stable solid dispersions of amorphous Compound I remain substantially free of crystalline Compound I for at least 1 month in Stability Chamber 2, when stored in a vial.

In some embodiments, stable solid dispersions of amorphous Compound I remain substantially free of crystalline Compound I for at least 12 months in Stability Chamber 2, when stored in a sealed bottle. In some embodiments, stable solid dispersions of amorphous Compound I remain substantially free of crystalline Compound I for at least 6 months in Stability Chamber 2, when stored in a sealed bottle. In other embodiments, stable solid dispersions of amorphous Compound I remain substantially free of crystalline Compound I for at least 4 months in Stability Chamber 2, when stored in a sealed bottle. In still other embodiments, stable solid dispersions of amorphous Compound I remain substantially free of crystalline Compound I for at least 3 months in Stability Chamber 2, when stored in a sealed bottle. In yet other embodiments, stable solid dispersions of amorphous Compound I remain substantially free of crystalline Compound I for at least 2 months in Stability Chamber 2, when stored in a sealed bottle. In some embodiments, stable solid dispersions of amorphous Compound I remain substantially free of crystalline Compound I for at least 1 month in Stability Chamber 2, when stored in a sealed bottle. In some embodiments, the dispersion is stored in the absence of a desiccant. In other embodiments, it is stored in the presence of a desiccant.

As used herein, "use environment" or "use medium" is defined as the "in vivo" environment of the gastrointestinal tract of an animal, particularly a human, or the "in vitro" environment of a test solution, examples being FaSSIF (Fasted State Simulated Intestinal Fluid) and FeSSIF (Fed State Simulated Intestinal Fluid). FaSSIF is characterized by a pH of 6.5, and imitates the pH that would be characteristic of the small intestine of an animal, particularly a human, in the fasted state. FeSSIF is characterized by a pH of 5.8, and imitates the pH that would be characteristic of the small intestine of an animal, particularly a human, in the fed state. A solid dispersion of Compound I can therefore be tested *in vivo* or, more conveniently, *in vitro* as described in the Example section, to ascertain whether it is within the scope of the invention.

"Solubility testing of a solid amorphous dispersion of Compound I" in a use environment is carried out by introducing the solid dispersion of Compound I into a use environment (for instance an *in-vitro* solution such as those described in the previous paragraph) at given conditions of temperature, atmospheric pressure, stirring speed, etc., removing aliquots of these solutions at pre-determined times and analyzing said aliquots (for example, using HPLC or some other analytical technique known in the art) to determine the concentration of Compound I that is present in solution at each time point. Throughout this disclosure the term "solubility testing" or "kinetic solubility testing" (or "kinetic solubility") is usually reserved for the study of the solid dispersions of Compound I.

As used herein, a "supersaturated solution of Compound I" is a solution that contains more of Compound I than could be dissolved by the given solvent under normal circumstances, if Compound I was dissolved by itself in the absence of the other components of the solid dispersion. Solutions of solid dispersions of amorphous Compound I of the invention are super saturated solutions of Compound I in a use environment (e.g., FaSSIF or FeSSIF). A solid dispersion of amorphous Compound I is within the scope of the invention if, when said solid dispersion is solubility tested, the maximum super saturated concentration of Compound I achievable with said solid dispersion is higher by a factor of at least 1.5 relative to the equilibrium concentration achieved by solubility testing a composition comprising an equivalent quantity of Compound I in the absence of the dispersion polymer (for example, neat Compound I, either amorphous or crystalline, for example as spray dried neat Compound I). In some embodiments, a solid dispersion of Compound I, when solubility tested in FaSSIF or FeSSIF achieves a maximum super saturated concentration (MSSC) of Compound I that is higher by a factor of at least 20, relative to a composition comprising an equivalent quantity of Compound I in the absence of the dispersion polymer. In other embodiments, it is higher by a factor of at least 15. In other embodiments, it is higher by a factor of at least 10. In other embodiments it is higher by a factor of at least 5.

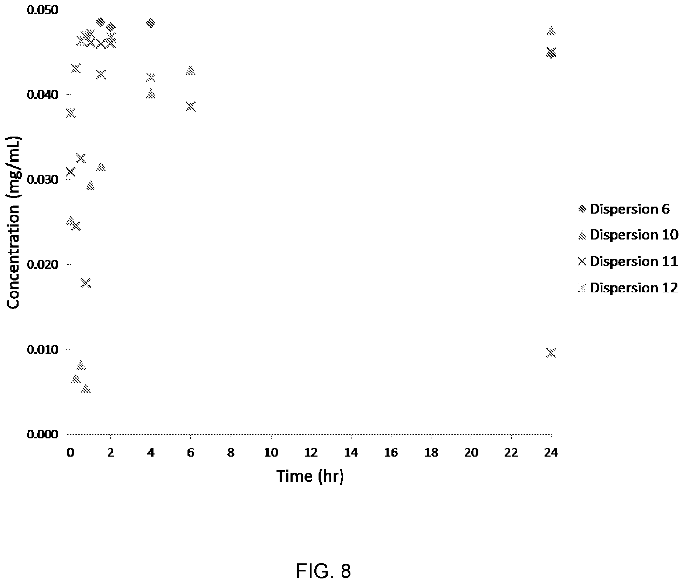

When carrying out solubility testing of a solid amorphous dispersion of Compound I, the maximum super saturated concentration of Compound I in solution is achieved soon after the dispersion is dissolved in the use environment and then it diminishes as Compound I slowly precipitates out of solution or it stays the same if the given dispersion is able to maintain Compound I in a supersaturated state for a longer period of time than the time of the test. An example of this type of behavior is displayed in Figure 8.

Polymers that are able to maintain Compound I in its super saturated state in solution in a use environment for long periods of time are said to be better "stabilizers of amorphous Compound I," and solid amorphous dispersions of Compound I comprising said polymers are preferred embodiments of the invention.

In some embodiments, a solid dispersion of amorphous Compound I of the invention is characterized by a maximum super saturated concentration of Compound I above 0.040 mg/mL when solubility tested in FaSSIF. In other embodiments, it is characterized by a maximum super saturated concentration between 0.040 mg/mL and 0.050 mg/mL when solubility tested in FaSSIF.

In other embodiments, a solid dispersion of amorphous Compound I of the invention is characterized by a super saturated concentration of Compound I between 0.040 mg/mL and 0.050 mg/mL after 2 hours of solubility testing in FaSSIF. In other embodiments it is characterized by a super saturated concentration of Compound I between 0.040 mg/mL and 0.050 mg/mL after 4 hours of solubility testing in FaSSIF. In other embodiments it is characterized by a super saturated concentration of Compound I between 0.040 mg/mL and 0.050 mg/mL after 24 hours of solubility testing in FaSSIF.

In some embodiments, a solid dispersion of amorphous Compound I of the invention is characterized by a maximum super saturated concentration of Compound I above 0.250 mg/mL when solubility tested in FeSSIF. In other embodiments, it is characterized by a maximum super saturated concentration between 0.250 mg/mL and 0.450 mg/mL when solubility tested in FeSSIF. In other embodiments, it is characterized by a maximum super saturated concentration between 0.250 mg/mL and 0.350 mg/mL when solubility tested in FeSSIF. In other embodiments, it is characterized by a maximum super saturated concentration between 0.350 mg/mL and 0.450 mg/mL when solubility tested in FeSSIF.

In other embodiments, a solid dispersion of amorphous Compound I of the invention is characterized by a super saturated concentration of Compound I between 0.250 mg/mL and 0.450 mg/mL after 1 hours of solubility testing in FeSSIF. In other embodiments it is characterized by a super saturated concentration of Compound I between 0.250 mg/mL and 0.450 mg/mL after 3 hours of solubility testing in FeSSIF In other embodiments it is characterized by a super saturated concentration of Compound I between 0.250 mg/mL and 0.450 mg/mL after 4 hours of solubility testing in FeSSIF In other embodiments it is characterized by a super saturated concentration of Compound I between 0.250 mg/mL and 0.450 mg/mL after 6 hours of solubility testing in FeSSIF.

In some embodiments of the above solid dispersions of amorphous Compound I, the polymer carrier is a water soluble or partially water soluble polymer as claimed.

The polymer carrier is selected from hydroxypropyl methyl cellulose acetate succinate (HPMCAS), hydroxypropyl methyl cellulose phthalate (HPMCP) or cellulose acetate phthalate (CAP). In some embodiments, the polymer carrier is selected from HPMCAS or CAP. In other embodiments, the at least one polymer carrier is selected from polyvinyl pyrrolidone (PVP) or a co-polymer of polyvinyl pyrrolidone.

The above polymer comprises between about 60 % and about 95 % of the total weight of the solid dispersion of Compound I. In other embodiments, the polymer comprises between about 70 % and about 95 % of the total weight. In other embodiments, it comprises at between about 75 % and about 95 % of the total weight. In other embodiments, it comprises at between about 80 % and about 95 %.

In other embodiments, the polymer comprises between about 70 % and about 90 % of the total weight of the solid dispersion of amorphous Compound I. In some embodiments, the polymer comprises between about 75 % and about 90 % of the total weight of the solid dispersion of amorphous Compound I. In other embodiments, the polymer comprises between about 60 % and about 95 % of the total weight of the solid dispersion of amorphous Compound I. In still other embodiments, the polymer comprises between about 60 % and about 90 % of the total weight of the solid dispersion of amorphous Compound I.

The amorphous solid dispersion of Compound I of the invention comprises between about 5 % and about 40 % by weight of Compound I. In other embodiments, the amorphous solid dispersion of Compound I of the invention comprises between about 10 % and about 30 %. In other embodiments, the amorphous solid dispersion of Compound I of the invention comprises between about 10 % and about 25%. In still other embodiments, the amorphous solid dispersion of Compound I of the invention comprises between about 15 % and about 40 %. In yet other embodiments, the amorphous solid dispersion of Compound I of the invention comprises between about 20 % and about 30%.

The ratio of Compound I to polymer is between about 20:80 and about 40:60. In some embodiments, the ratio of Compound I to polymer is between about 20:80 and about 50:50. In still other embodiments, the ratio of Compound I to polymer is about 25:75. In some embodiments, the ratio of Compound I to polymer is about 50:50.

In some embodiments, the solid dispersions of amorphous Compound I are obtained by spray-drying. In other embodiments, the solid dispersions of amorphous Compound I are obtained by hot melt extrusion. Any spray drying or hot melt extrusion can be used that results in the desired solid dispersion of amorphous Compound I.

In some embodiments, solid dispersions of amorphous Compound I optionally comprise one or more excipients or pharmaceutically acceptable excipients.

Additives in the solid dispersion may be added directly to the spray-drying solution when forming the mixture. For instance, the additive may be dissolved or suspended in the solution as a slurry which can then be spray dried. Alternatively, the additives may be added following the spray-drying process to aid in the forming of the final formulated product.

In a second aspect, the invention relates to pharmaceutical compositions comprising a solid dispersion of amorphous Compound I and at least one pharmaceutical excipient. In some embodiments, the pharmaceutical compositions comprise a filler. In some embodiments the pharmaceutical compositions comprise a disintegrant. In other embodiments they comprise a lubricant. In some embodiments, the pharmaceutical compositions of the invention may optionally comprise a binder and/or a glidant. In some embodiments, the pharmaceutical compositions of the invention comprise a glidant.

Non-limiting examples of fillers include microcrystalline cellulose, lactose, mannitol, maltodextrins, other compressible sugars, dicalcium phosphate, calcium sulfate, other salts or a starch. In some embodiments, the filler is selected from microcrystalline cellulose, lactose, mannitol, maltodextrins, other compressible sugars, dicalcium phosphate, calcium sulfate, other salts or a starch. In some embodiments the filler is selected from microcrystalline cellulose.

Non-limiting examples of disintegrants include polyvinylpyrrolidone (PVP), croscarmellose sodium or sodium starch glycolate. In some embodiments, the disintegrant is selected from polyvinylpyrrolidone (PVP), croscarmellose sodium or sodium starch glycolate. In some embodiments it is selected from croscarmellose sodium.

Non-limiting examples of lubricants include magnesium stearate, calcium stearate, stearic acid or glyceryl behenate. In some embodiments, the lubricant is selected from magnesium stearate, calcium stearate, stearic acid or glyceryl behenate. In some embodiments, it is selected from magnesium stearate.

Non limiting examples of glidants include silica, hydrophilic fuming silica or Aerosil200.

In some embodiments, pharmaceutical compositions comprising solid dispersions of amorphous Compound I of the invention comprise less than about 20 % crystalline Compound I, less than about 15 % crystalline Compound I, less than about 10 % crystalline Compound I, less than about 8 % crystalline Compound I, less than about 5 % crystalline Compound I, less than about 2 % crystalline Compound I, less than about 1% crystalline Compound I, or less than about 0.5 % crystalline Compound I. In other embodiments, pharmaceutical compositions of the invention are substantially free of crystalline Compound I as determined by XRPD, DSC or microscopy.

The term "stable pharmaceutical compositions comprising a solid dispersion of amorphous Compound I", as used herein, refers to pharmaceutical compositions comprising a solid dispersion of amorphous Compound I (i.e., substantially amorphous Compound I) that do not substantially increase the amount of crystalline Compound I when kept under certain storage conditions over a given period or length of time. In some embodiments, pharmaceutical compositions of the invention are stable under the storage conditions of Stability Chamber 1 for a period of up to two years, when stored in a vial. In other embodiments, pharmaceutical compositions of the invention are stable under the storage conditions of Stability Chamber 1 for a period of up to 12 months, when stored in a vial. In still other embodiments, pharmaceutical compositions of the invention are stable under the storage conditions of Stability Chamber 1 for a period of up to 6 months, when stored in a vial. In some embodiments, pharmaceutical compositions of the invention are stable under the storage conditions of Stability Chamber 1 for a period of up to 4 months, when stored in a vial. In other embodiments, pharmaceutical compositions of the invention are stable under the storage conditions of Stability Chamber 1 for a period of up to 3 months, when stored in a vial. In still other embodiments, pharmaceutical compositions of the invention are stable under the storage conditions of Stability Chamber 1 for a period of up to 2 months, when stored in a vial. In yet other embodiments, pharmaceutical compositions of the invention are stable under the storage conditions of Stability Chamber 1 for a period of up to 1 month, when stored in a vial.

In some embodiments, pharmaceutical compositions of the invention are stable under the storage conditions of Stability Chamber 1 for a period of up to two years, when stored in a sealed bottle. In other embodiments, pharmaceutical compositions of the invention are stable under the storage conditions of Stability Chamber 1 for a period of up to 12 months, when stored in a sealed bottle. In still other embodiments, pharmaceutical compositions of the invention are stable under the storage conditions of Stability Chamber 1 for a period of up to 6 months, when stored in a sealed bottle. In some embodiments, pharmaceutical compositions of the invention are stable under the storage conditions of Stability Chamber 1 for a period of up to 4 months, when stored in a sealed bottle. In other embodiments, pharmaceutical compositions of the invention are stable under the storage conditions of Stability Chamber 1 for a period of up to 3 months, when stored in a sealed bottle. In still other embodiments, pharmaceutical compositions of the invention are stable under the storage conditions of Stability Chamber 1 for a period of up to 2 months, when stored in a sealed bottle. In yet other embodiments, pharmaceutical compositions of the invention are stable under the storage conditions of Stability Chamber 1 for a period of up to 1 month, when stored in a sealed bottle.

In some embodiments, pharmaceutical compositions of the invention are stable under the storage conditions of Stability Chamber 2 for a period of up to two years, when stored in a vial. In other embodiments, pharmaceutical compositions of the invention are stable under the storage conditions of Stability Chamber 2 for a period of up to 12 months, when stored in a vial. In still other embodiments, pharmaceutical compositions of the invention are stable under the storage conditions of Stability Chamber 2 for a period of up to 6 months, when stored in a vial. In some embodiments, pharmaceutical compositions of the invention are stable under the storage conditions of Stability Chamber 2 for a period of up to 4 months, when stored in a vial. In other embodiments, pharmaceutical compositions of the invention are stable under the storage conditions of Stability Chamber 2 for a period of up to 3 months, when stored in a vial. In still other embodiments, pharmaceutical compositions of the invention are stable under the storage conditions of Stability Chamber 2 for a period of up to 2 months, when stored in a vial. In yet other embodiments, pharmaceutical compositions of the invention are stable under the storage conditions of Stability Chamber 2 for a period of up to 1 month, when stored in a vial.

In some embodiments, pharmaceutical compositions of the invention are stable under the storage conditions of Stability Chamber 2 for a period of up to two years, when stored in a sealed bottle. In other embodiments, pharmaceutical compositions of the invention are stable under the storage conditions of Stability Chamber 2 for a period of up to 12 months, when stored in a sealed bottle. In still other embodiments, pharmaceutical compositions of the invention are stable under the storage conditions of Stability Chamber 2 for a period of up to 6 months, when stored in a sealed bottle. In some embodiments, pharmaceutical compositions of the invention are stable under the storage conditions of Stability Chamber 2 for a period of up to 4 months, when stored in a sealed bottle. In other embodiments, pharmaceutical compositions of the invention are stable under the storage conditions of Stability Chamber 2 for a period of up to 3 months, when stored in a sealed bottle. In still other embodiments, pharmaceutical compositions of the invention are stable under the storage conditions of Stability Chamber 2 for a period of up to 2 months, when stored in a sealed bottle. In yet other embodiments, pharmaceutical compositions of the invention are stable under the storage conditions of Stability Chamber 2 for a period of up to 1 month, when stored in a sealed bottle.

Compound I, or pharmaceutically acceptable salts thereof, may be formulated into pharmaceutical dosage unit forms to provide an easily controllable dosage of the drug and to enable patient compliance with the prescribed regimen. Pharmaceutical dosage unit forms of Compound I, or pharmaceutically acceptable salts thereof, may be prepared for various routes and types of administration. Various dosage forms may exist for the same compound, since different medical conditions may warrant different routes of administration

In a third aspect, the invention relates to dosage unit forms comprising a solid dispersion of amorphous Compound I or a pharmaceutical composition described herein. In some embodiments the dosage unit forms are suitable for oral administration. In some embodiments, the dosage unit form is selected from a capsule, a suspension, a powder or a tablet. In other embodiments, it is a tablet.

In some embodiments, the dosage unit forms comprising a solid dispersion of amorphous Compound I further comprise at least one pharmaceutical excipient. In some embodiments it is a coating. As a non-limiting example, the coating is selected from a cellulose based material. In some embodiments, it is selected from a PVP based material.

In some embodiments, the dosage unit forms comprising a stable solid dispersion of amorphous Compound I are substantially free of crystalline Compound I when placed under the storage conditions of Stability Chamber 1 for a period of up to two years, when stored in a vial. In other embodiments, the dosage unit forms are stable under the storage conditions of Stability Chamber 1 for a period of up to 12 months, when stored in a vial. In still other embodiments, the dosage unit forms are stable under the storage conditions of Stability Chamber 1 for a period of up to 6 months, when stored in a vial. In some embodiments, the dosage unit forms are stable under the storage conditions of Stability Chamber 1 for a period of up to 4 months, when stored in a vial. In other embodiments, the dosage unit forms are stable under the storage conditions of Stability Chamber 1 for a period of up to 3 months, when stored in a vial. In still other embodiments, the dosage unit forms are stable under the storage conditions of Stability Chamber 1 for a period of up to 2 months, when stored in a vial. In yet other embodiments, the dosage unit forms are stable under the storage conditions of Stability Chamber 1 for a period of up to 1 month, when stored in a vial.

In some embodiments, the dosage unit forms are stable under the storage conditions of Stability Chamber 1 for a period of up to two years, when stored in a sealed bottle. In other embodiments, the dosage unit forms are stable under the storage conditions of Stability Chamber 1 for a period of up to 12 months, when stored in a sealed bottle. In still other embodiments, the dosage unit forms are stable under the storage conditions of Stability Chamber 1 for a period of up to 6 months, when stored in a sealed bottle. In some embodiments, the dosage unit forms are stable under the storage conditions of Stability Chamber 1 for a period of up to 4 months, when stored in a sealed bottle. In other embodiments, the dosage unit forms are stable under the storage conditions of Stability Chamber 1 for a period of up to 3 months, when stored in a sealed bottle. In still other embodiments, the dosage unit forms are stable under the storage conditions of Stability Chamber 1 for a period of up to 2 months, when stored in a sealed bottle. In yet other embodiments, the dosage unit forms are stable under the storage conditions of Stability Chamber 1 for a period of up to 1 month, when stored in a sealed bottle.

In some embodiments, the dosage unit forms are stable under the storage conditions of Stability Chamber 2 for a period of up to two years, when stored in a vial. In other embodiments, the dosage unit forms are stable under the storage conditions of Stability Chamber 2 for a period of up to 12 months, when stored in a vial. In still other embodiments, the dosage unit forms are stable under the storage conditions of Stability Chamber 2 for a period of up to 6 months, when stored in a vial. In some embodiments, the dosage unit forms are stable under the storage conditions of Stability Chamber 2 for a period of up to 4 months, when stored in a vial. In other embodiments, the dosage unit forms are stable under the storage conditions of Stability Chamber 2 for a period of up to 3 months, when stored in a vial. In still other embodiments, the dosage unit forms are stable under the storage conditions of Stability Chamber 2 for a period of up to 2 months, when stored in a vial. In yet other embodiments, the dosage unit forms are stable under the storage conditions of Stability Chamber 2 for a period of up to 1 month, when stored in a vial.

In some embodiments, the dosage unit forms are stable under the storage conditions of Stability Chamber 2 for a period of up to two years, when stored in a sealed bottle. In other embodiments, the dosage unit forms are stable under the storage conditions of Stability Chamber 2 for a period of up to 12 months, when stored in a sealed bottle. In still other embodiments, the dosage unit forms are stable under the storage conditions of Stability Chamber 2 for a period of up to 6 months, when stored in a sealed bottle. In some embodiments, the dosage unit forms are stable under the storage conditions of Stability Chamber 2 for a period of up to 4 months, when stored in a sealed bottle. In other embodiments, the dosage unit forms are stable under the storage conditions of Stability Chamber 2 for a period of up to 3 months, when stored in a sealed bottle. In still other embodiments, the dosage unit forms are stable under the storage conditions of Stability Chamber 2 for a period of up to 2 months, when stored in a sealed bottle. In yet other embodiments, the dosage unit forms are stable under the storage conditions of Stability Chamber 2 for a period of up to 1 month, when stored in a sealed bottle.

The amount of active ingredient that may be combined with the carrier material and other pharmaceutically acceptable excipients to produce a single dosage form will vary depending upon the subject treated and the particular mode of administration. The term "therapeutically effective amount" as used herein means that amount of active compound or pharmaceutical agent that elicits the biological or medicinal response in a tissue, system, animal or human that is being sought by a researcher, veterinarian, medical doctor or other clinician. The therapeutically or pharmaceutically effective amount of the compound to be administered will be governed by such considerations, and is the minimum amount necessary to ameliorate, cure or treat the disease or disorder or one or more of its symptoms.

"Treatment" can involve administering a compound described herein to a patient diagnosed with a disease, and may involve administering the compound to a patient who does not have active symptoms. Conversely, treatment may involve administering the compositions to a patient at risk of developing a particular disease, or to a patient reporting one or more of the physiological symptoms of a disease, even though a diagnosis of this disease may not have been made. The term "prophylactically effective amount" refers to an amount effective in reducing the severity of the disease or disorder before it is acquired or reducing the severity of one or more of its symptoms before the symptoms develop.

In some embodiments, the dosage unit forms comprising a stable solid dispersion of amorphous Compound I comprise a therapeutically effective amount of Compound I. In other embodiments, they comprise a prophylactically effective amount of Compound I. In some embodiments, they comprise about 5 mg of Compound I, about 10 mg of Compound I, about 15 mg of Compound I, about 20 mg of Compound I, about 25 mg of Compound I, about 30 mg of Compound I, about 35 mg of Compound I, about 40 mg of Compound I, about 45 mg of Compound I, about 50 mg of Compound I, about 55 mg of Compound I, about 60 mg of Compound I, about 65 mg of Compound I, about 70 mg of Compound I, about 75 mg of Compound I, about 80 mg of Compound I, about 85 mg of Compound I, about 90 mg of Compound I, about 95 mg of Compound I, about 100 mg of Compound I, about 105 mg of Compound I, about 110 mg of Compound I, about 115 mg of Compound I, about 120 mg of Compound I, about 125 mg of Compound I, about 130 mg of Compound I, about 135 mg of Compound I, about 140 mg of Compound I, about 140 mg of Compound I, about 145 mg of Compound I, or about 150 mg of Compound I. In some embodiments, the dosage unit forms comprising a solid dispersion of amorphous Compound I comprise between about 5 mg and 150 mg of Compound I. In other embodiments, the dosage unit forms comprising a solid dispersion of amorphous Compound I comprise between about 10 mg and 130 mg of Compound I, or between about 10 mg and 100 mg of Compound I, or between about 10 mg and 80 mg of Compound I. In some embodiments, the dosage unit forms comprising a solid dispersion of amorphous Compound I comprise between about 10 mg and 50 mg of Compound I, or between about 10 mg and 40 mg of Compound I, or between about 10 mg and 30 mg of Compound I. In some embodiments, the dosage unit forms comprising a solid dispersion of amorphous Compound I comprise between about 5 mg and 50 mg of Compound I, or between about 5 mg and 40 mg of Compound I, or between about 5 mg and 30 mg of Compound I. In some embodiments, the dosage unit forms comprising a solid dispersion of amorphous Compound I comprise between about 5 mg and 25 mg of Compound I, or between about 10 mg and 25 mg of Compound I. In some embodiments, the dosage unit forms comprising a solid dispersion of amorphous Compound I comprise between about 5 mg and 20 mg of Compound I, or between about 10 mg and 20 mg of Compound I, or between about 15 mg and 25 mg of Compound I.

"Dissolution testing of a unit dosage form comprising a solid dispersion of Compound I" in a use environment is carried out by introducing the unit dosage form comprising a solid dispersion of Compound I into a use environment (for instance an *in-vitro* solution such as those described above) at given conditions of temperature, atmospheric pressure, stirring speed etc., removing aliquots of these solutions at pre-determined times and analyzing said aliquots (for example using HPLC or some other analytical technique known in the art) to determine the concentration of Compound I that is present in solution at each time point, then further determining the percentage of Compound I that is present in solution relative to the total amount of Compound I known to be present in the amount of dosage unit forms used for the dissolution testing (e.g., the total amount of Compound I known to be present in a certain number of tablets comprising a given solid amorphous dispersion of Compound I of the invention).

When carrying out dissolution testing, the percentage of Compound I present in solution initially is zero and it increases as the dosage form slowly releases the dispersion of Compound I into the use environment to reach a maximum percentage of Compound I that the solution is able to maintain in a stable super saturated state after a certain period of time. After the maximum percentage of Compound I in solution is reached (ideally this percentage would be 100 % but in reality it is lower than 100 % in many instances), this can either be maintained for a long period of time or it may diminish as Compound I starts to precipitate out of solution, depending on the time of the test and on the stability of the given solid dispersion of Compound I in the use environment.

In some embodiments, dosage unit forms of the invention are characterized by achieving more than 60 % dissolution after 30 min of dissolution testing in FaSSIF. In other embodiments, dosage unit forms of the invention are characterized by achieving between 70 % and 80 % dissolution after 60 min of dissolution testing in FaSSIF. In other embodiments they are characterized by achieving between 75 % and 85 % dissolution after 60 min of dissolution testing in FaSSIF. In other embodiments, they are characterized by achieving between 75 % and 80 % dissolution after 60 min of dissolution testing in FaSSIF.

Unit dosage forms of the invention may also be tested *in vivo,* either in humans or a suitable animal model, for example, a dog.

In some embodiments, a pharmaceutical composition comprising a solid dispersion of amorphous Compound I or unit dosage form comprising a solid dispersion of amorphous Compound I is within the scope of the invention if, when tested *in vivo,* The Cmax/dose value achieved with said pharmaceutical composition or unit dosage form is higher by a factor of at least 1.5 (i.e. at least 50 % higher) relative to the Cmax/dose value achieved with a composition or unit dosage form comprising an equivalent quantity of Compound I in the absence of the dispersion polymer (for instance a pharmaceutical composition or unit dosage form comprising Compound I neat, either in crystalline or amorphous form, e.g., a wet granulation formulation of "native Compound I" or "native API") when both of them are tested under identical conditions (e.g., in the fed state, or in the fasted state and at the same temperature, atmospheric pressure and stirring speed). Cmax/dose is an abbreviation for the maximum drug concentration in ng/mL in serum or plasma of the test subject divided by the dose administered in mg/Kg. In other embodiments, the Cmax/dose achieved with said pharmaceutical composition or unit dosage form is higher by a factor of at least 2.0 (i.e. at least 100 % higher) relative to the Cmax/dose value achieved with a composition or unit dosage form comprising an equivalent quantity of Compound I in the absence of the dispersion polymer. In other embodiments, the Cmax/dose is higher by a factor of at least 2.5 (i.e. at least 150 % higher). In other embodiments, it is higher by a factor of at least 3.0 (i.e. 200 % higher). Still in other embodiments, it is higher by a factor of at least 1.25 (i.e., 25 % higher).

In some embodiments, a pharmaceutical composition comprising a solid dispersion of amorphous Compound I or unit dosage form comprising a solid dispersion of amorphous Compound I is within the scope of the invention if, when tested *in vivo,* the AUC/dose achieved with said pharmaceutical composition or unit dosage form is higher by a factor of at least 1.25 (i.e. at least 25 % higher) relative to the AUC/dose value achieved with a composition or unit dosage form comprising an equivalent quantity of Compound I in the absence of the dispersion polymer (for instance a pharmaceutical composition or unit dosage form comprising Compound I neat, either in crystalline or amorphous form, e.g., a wet granulation formulation of "native Compound I" or "native API") when both of them are tested under identical conditions (e.g., in the fed state, or in the fasted state, under identical conditions of temperature, atmospheric pressure and stirring speed). AUC/dose is an abbreviation for Area Under the Curve obtained when plotting the serum or plasma concentration for the test subject of Compound I in the y axis versus time in the x axis (in units of hours x ng/mL, divided by the dose administered (in units of mg/Kg). In other embodiments, the AUC/dose achieved with said pharmaceutical composition or unit dosage form is higher by a factor of at least 1.5 (i.e. at least 50 % higher) relative to the AUC/dose value achieved with a composition or unit dosage form comprising an equivalent quantity of Compound I in the absence of the dispersion polymer. In other embodiments, the AUC/dose is higher by a factor of at least 2.0 (i.e. at least 100 % higher). In other embodiments, it is higher by a factor of at least 2.5 (i.e. 150 % higher). In other embodiments, the AUC/dose is higher by a factor of at least 3.0 (i.e. at least 200 % higher).

In some embodiments, a pharmaceutical composition comprising a solid dispersion of amorphous Compound I or unit dosage form comprising a solid dispersion of amorphous Compound I is within the scope of the invention if, when tested *in vivo,* the estimated bioavailability with said pharmaceutical composition or unit dosage form is higher by a factor of at least 1.25 (i.e. at least 25 % higher) relative to the estimated bioavailability value achieved with a composition or unit dosage form comprising an equivalent quantity of Compound I in the absence of the dispersion polymer (for instance, a pharmaceutical composition or unit dosage form comprising Compound I neat, either in crystalline or amorphous form) when both of them are tested under identical conditions (e.g., in the fed state, or in the fasted state, and under identical conditions of temperature, atmospheric pressure and stirring speed). Bioavailability is well understood in the art. In other embodiments, the estimated bioavailability with said pharmaceutical composition or unit dosage form is higher by a factor of at least 1.5 (i.e., at least 50 % higher) relative to the estimated bioavailability value achieved with a composition or unit dosage form comprising an equivalent quantity of Compound I in the absence of the dispersion polymer. In other embodiments, the estimated bioavailability is higher by a factor of at least 2.0 (i.e., at least 100 % higher).

The pharmaceutical compositions or dosage unit forms of the invention may also include other types of excipients such as one or more buffers, stabilizing agents, antiadherents, surfactants, wetting agents, lubricating agents, emulsifiers, binders, suspending agents, disintegrants, fillers, sorbents, coatings (e.g., enteric or slow release) preservatives, antioxidants, opaquing agents, glidants, processing aids, colorants, sweeteners, perfuming agents, flavoring agents and other known additives to provide an elegant presentation of the drug or aid in the manufacturing of the pharmaceutical product (i.e., medicament).

The pharmaceutical compositions or dosage unit forms of the invention include those suitable for the administration routes detailed herein. The pharmaceutical compositions may conveniently be presented in unit dosage form and may be prepared by any of the methods well known in the art of pharmacy. Techniques and formulations generally are found in Remington's. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then, if necessary, shaping the product.

The terms "administer", "administering" or "administration" in reference to a solid dispersion of Compound I, pharmaceutical composition or dosage unit form of the invention means introducing said solid dispersion of Compound I, pharmaceutical composition or dosage unit form into the system of the animal in need of treatment. When Compound I is provided in combination with one or more other active agents, "administration" and its variants are each understood to include concurrent and/or sequential introduction said solid dispersion of Compound I, pharmaceutical composition or dosage unit form and the other active agents.

The pharmaceutical compositions described herein may be orally administered in any orally acceptable solid dosage form. Solid dosage forms for oral administration include capsules, tablets, pills, powders, and granules. In such solid dosage forms, the active compound may be mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. Tablets may be uncoated or may be coated by known techniques including microencapsulation to mask an unpleasant taste. A water soluble taste masking material such as hydroxypropyl-methylcellulose or hydroxypropyl-cellulose may be employed.

Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent.

The pharmaceutical composition or dosage unit form of the invention may be packaged in a variety of ways depending upon the method used for administering the drug. Generally, an article for distribution includes a container having deposited therein the pharmaceutical formulation in an appropriate form. Suitable containers are well-known to those skilled in the art and include materials such as bottles (plastic and glass), sachets, ampoules, plastic bags, metal cylinders, and the like. The container may also include a tamper-proof assemblage to prevent indiscreet access to the contents of the package. In addition, the container has deposited thereon a label that describes the contents of the container. The label may also include appropriate warnings.

The solid dispersions of Compound I, pharmaceutical compositions or dosage unit forms here disclosed may be packaged in unit-dose or multi-dose containers. Preferred unit dosage formulations are those containing a daily dose or unit daily sub-dose, as herein above recited, or an appropriate fraction thereof, of the active ingredient.

In another aspect, Compound I, or a pharmaceutically acceptable salt thereof, may be formulated in a veterinary composition comprising a veterinary carrier. Veterinary carriers are materials useful for the purpose of administering the composition and may be solid materials which are otherwise inert or acceptable in the veterinary art and are compatible with the active ingredient. These veterinary compositions may be administered orally.

### Therapeutic methods

In another aspect, the invention relates to the treatment of certain disorders by using a solid dispersion of amorphous Compound I of the invention, either alone or in combination, or its pharmaceutically acceptable salts, or pharmaceutical compositions or dosage unit forms comprising them, in a patient in need thereof.

The above solid amorphous dispersions, pharmaceutical compositions or dosage unit forms may be used alone or in combination with one or more additional agents, for treating and/or preventing various diseases, wherein an increase in the concentration of NO or an increase in the concentration of cGMP might be desirable. The diseases that can be treated include but are not limited to pulmonary hypertension, arterial hypertension, heart failure, atherosclerosis, inflammation, thrombosis, renal fibrosis and failure, liver cirrhosis, erectile dysfunction, female sexual disorders, disorders related to diabetes, ocular disorders and other related cardiovascular disorders.

Increased concentration of cGMP leads to vasodilation, inhibition of platelet aggregation and adhesion, anti-hypertensive effects, anti-remodeling effects, anti-apoptotic effects, anti-inflammatory effects and neuronal signal transmission effects. Thus, the above solid amorphous dispersions, pharmaceutical compositions and dosage unit forms may be used to treat and/or prevent a range of diseases and disorders, including but not limited to a peripheral, pulmonary, hepatic, liver, cardiac or cerebrovascular/endothelial disorders or conditions, a urogenital-gynecological or sexual disorder or condition, a thromboembolic disease, an ischemic disease, a fibrotic disorder, a topical or skin disorder, a pulmonary or respiratory disorder, a renal or hepatic disorder, a metabolic disorder, atherosclerosis, or a lipid related disorder.

In other embodiments, the above solid amorphous dispersions, pharmaceutical compositions and dosage unit forms may be useful in the prevention and/or treatment of diseases and disorders characterized by undesirable reduced bioavailability of and/or sensitivity to NO, such as those associated with conditions of oxidative stress or nitrosative stress.

Throughout this disclosure, the terms "hypertension", "arterial hypertension" or "high blood pressure (HBP)" are used interchangeable and refer to an extremely common and highly preventable chronic condition in which blood pressure (BP) in the arteries is higher than normal. If not properly controlled, it represents a significant risk factor for several serious cardiovascular and renal conditions. Hypertension may be a primary disease, called "essential hypertension" or "idiopathic hypertension", or it may be caused by other diseases, in which case it is classified as "secondary hypertension". Essential hypertension accounts for 90-95% of all cases.

As used herein, the term "resistant hypertension" refers to hypertension that remains above goal blood pressure (usually less than 140/90 mmHg, although a lower goal of less than 130/80 mmHg is recommended for patients with comorbid diabetes or kidney disease), in spite of concurrent use of *three* antihypertensive agents belonging to different antihypertensive drug classes. People who require four or more drugs to control their blood pressure are also considered to have resistant hypertension. Hypertension is an extremely common comorbid condition in diabetes, affecting ~20-60% of patients with diabetes, depending on obesity, ethnicity, and age. This type of hypertension is herein referred to as "diabetic hypertension". In type 2 diabetes, hypertension is often present as part of the metabolic syndrome of insulin resistance also including central obesity and dyslipidemia. In type 1 diabetes, hypertension may reflect the onset of diabetic nephropathy.

"Pulmonary hypertension (PH)", as used herein, is a disease characterized by sustained elevations of blood pressure in the pulmonary vasculature (pulmonary artery, pulmonary vein and pulmonary capillaries), which results in right heart hypertrophy, eventually leading to right heart failure and death. Common symptoms of PH include shortness of breath, dizziness and fainting, all of which are exacerbated by exertion. Without treatment, median life expectancy following diagnosis is 2.8 years. PH exists in many different forms, which are categorized according to their etiology. Categories include pulmonary arterial hypertension (PAH), PH with left heart disease, PH associated with lung diseases and /or hypoxaemia, PH due to chronic thrombotic and/or embolic disease and miscellaneous PH. PAH is rare in the general population, but the prevalence increases in association with certain common conditions such as HIV infection, scleroderma and sickle cell disease. Other forms of PH are generally more common than PAH, and, for instance, the association of PH with chronic obstructive pulmonary disease (COPD) is of particular concern. Current treatment for pulmonary hypertension depends on the stage and the mechanism of the disease.

As used herein "heart failure" is a progressive disorder of left ventricular (LV) myocardial remodeling that culminates in a complex clinical syndrome in which impaired cardiac function and circulatory congestion are the defining features, and results in insufficient delivery of blood and nutrients to body tissues. The condition occurs when the heart is damaged or overworked and unable to pump out all the blood that returns to it from the systemic circulation. As less blood is pumped out, blood returning to the heart backs up and fluid builds up in other parts of the body. Heart failure also impairs the kidneys' ability to dispose of sodium and water, complicating fluid retention further. Heart failure is characterized by autonomic dysfunction, neurohormonal activation and overproduction of cytokines, which contribute to progressive circulatory failure. Symptoms of heart failure include: dyspnea (shortness of breath) while exercising or resting and waking at night due to sudden breathlessness, both indicative of pulmonary edema; general fatigue or weakness, edema of the feet, ankles and legs, rapid weight gain, chronic cough, including that producing mucus or blood. Depending on its clinical presentation, heart failure is classified as *de novo,* transient or chronic. Acute heart failure, i.e. the rapid or gradual onset of symptoms requiring urgent therapy, may develop de novo or as a result of chronic heart failure becoming decompensated. Diabetes is a common comorbidity in patients with heart failure and is associated with poorer outcomes as well as potentially compromising the efficacy of treatments. Other important comorbidities include systemic hypertension, chronic airflow obstruction, sleep apnea, cognitive dysfunction, anemia, chronic kidney disease and arthritis. Chronic left heart failure is frequently associated with the development of pulmonary hypertension. The frequency of certain comorbidities varies by gender: among women, hypertension and thyroid disease are more common, while men more commonly suffer from chronic obstructive pulmonary disease (COPD), peripheral vascular disease, coronary artery disease and renal insufficiency. Depression is a frequent comorbidity of heart failure and the two conditions can and often do complicate one another. Cachexia has long been recognized as a serious and frequent complication of heart failure, affecting up to 15% of all heart failure patients and being associated with poor prognosis. Cardiac cachexia is defined as the non-edematous, nonvoluntary loss of at least 6% of body weight over a period of six months.

The term "sleep apnea" refers to the most common of the sleep-disordered breathing disorders. It is a condition characterized by intermittent, cyclical reductions or total cessations of airflow, which may or may not involve obstruction of the upper airway. There are three types of sleep apnea: obstructive sleep apnea, the most common form, central sleep apnea and mixed sleep apnea.

"Central sleep apnea (CSA)", is caused by a malfunction in the brain's normal signal to breathe, rather than physical blockage of the airway. The lack of respiratory effort leads to an increase in carbon dioxide in the blood, which may rouse the patient. CSA is rare in the general population, but is a relatively common occurrence in patients with systolic heart failure.

As used herein, the term "metabolic syndrome", "insulin resistance syndrome" or "syndrome X", refers to a group or clustering of metabolic conditions (abdominal obesity, elevated fasting glucose, "dyslipidemia" (i.e., elevated lipid levels) and elevated blood pressure (HBP)) which occur together more often than by chance alone and that together promote the development of type 2 diabetes and cardiovascular disease. Metabolic syndrome is characterized by a specific lipid profile of increased triglycerides, decreased high-density lipoprotein cholesterol (HDL-cholesterol) and in some cases moderately elevated low-density lipoprotein cholesterol (LDL-cholesterol) levels, as well as accelerated progression of "atherosclerotic disease" due to the pressure of the component risk factors. There are several types of dyslipidemias: "hypercholesterolemia" refers to elevated levels of cholesterol. Familial hypercholesterolemia is a specific form of hypercholesterolemia due to a defect on chromosome 19 (19p13.1-13.3). "Hyperglyceridemia" refers to elevated levels of glycerides (e.g., "hypertrigliceridemia" involves elevated levels of triglycerides). "Hyperlipoproteinemia" refers to elevated levels of lipoproteins (usually LDL unless otherwise specified).

As used herein, the term "peripheral vascular disease (PVD)", also commonly referred to as "peripheral arterial disease (PAD)" or "peripheral artery occlusive disease (PAOD)", refers to the obstruction of large arteries *not* within the coronary, aortic arch vasculature, or brain. PVD can result from atherosclerosis, inflammatory processes leading to stenosis, an embolism, or thrombus formation. It causes either acute or chronic "ischemia (lack of blood supply)". Often PVD is a term used to refer to atherosclerotic blockages found in the lower extremity. PVD also includes a subset of diseases classified as microvascular diseases resulting from episodal narrowing of the arteries (e.g., "Raynaud's phenomenon"), or widening thereof (erythromelalgia), i.e. vascular spasms.

The term "thrombosis" refers to the formation of a blood clot ("thrombus") inside a blood vessel, obstructing the flow of blood through the circulatory system. When a blood vessel is injured, the body uses platelets (thrombocytes) and fibrin to form a blood clot to prevent blood loss. Alternatively, even when a blood vessel is not injured, blood clots may form in the body if the proper conditions present themselves. If the clotting is too severe and the clot breaks free, the traveling clot is now known as an "embolus". The term "thromboembolism" refers to the combination of thrombosis and its main complication, "embolism". When a thrombus occupies more than 75% of surface area of the lumen of an artery, blood flow to the tissue supplied is reduced enough to cause symptoms because of decreased oxygen (hypoxia) and accumulation of metabolic products like lactic acid ("gout"). More than 90% obstruction can result in anoxia, the complete deprivation of oxygen, and "infarction", a mode of cell death.

An "embolism" (plural embolisms) is the event of lodging of an embolus (a detached intravascular mass capable of clogging arterial capillary beds at a site far from its origin) into a narrow capillary vessel of an arterial bed which causes a blockage (vascular occlusion) in a distant part of the body. This is not to be confused with a thrombus which blocks at the site of origin.

A "stroke", or cerebrovascular accident (CVA), is the rapid loss of brain function(s) due to disturbance in the blood supply to the brain. This can be due to "ischemia" (lack of blood flow) caused by blockage (thrombosis, arterial embolism), or a hemorrhage (leakage of blood). As a result, the affected area of the brain cannot function, which might result in an inability to move one or more limbs on one side of the body, inability to understand or formulate speech, or an inability to see one side of the visual field. Risk factors for stroke include old age, hypertension, previous stroke or transient ischemic attack (TIA), diabetes, high cholesterol, cigarette smoking and atrial fibrillation. High blood pressure is the most important modifiable risk factor of stroke. An "ischemic stroke" is occasionally treated in a hospital with thrombolysis (also known as a "clot buster"), and some hemorrhagic strokes benefit from neurosurgery. Prevention of recurrence may involve the administration of antiplatelet drugs such as aspirin and dipyridamole, control and reduction of hypertension, and the use of statins. Selected patients may benefit from carotid endarterectomy and the use of anticoagulants.

"Ischemia" is a restriction in blood supply to tissues, causing a shortage of oxygen and glucose needed for cellular metabolism (to keep tissue alive). Ischemia is generally caused by problems with blood vessels, with resultant damage to or dysfunction of tissue. It also means local anemia in a given part of a body sometimes resulting from congestion (such as vasoconstriction, thrombosis or embolism).

According to the American Psychiatric Association's Diagnostic and Statistical Manual of Mental Disorders, Fourth Edition (DSM-IV), the term "sexual dysfunction" encompasses a series of conditions "characterized by disturbances in sexual desire and in the psychophysiological changes associated with the sexual response cycle"; while problems of this type are common, sexual dysfunction is only considered to exist when the problems cause distress for the patient. Sexual dysfunction can be either physical or psychological in origin. It can exist as a primary condition, generally hormonal in nature, although most often it is secondary to other medical conditions or to drug therapy for said conditions. All types of sexual dysfunction can be further classified as life-long, acquired, situational or generalized (or combinations thereof).

The DSM-IV-TR specifies five major categories of "female sexual dysfunction": sexual desire/interest disorders; "sexual arousal disorders (including genital, subjective and combined)"; orgasmic disorder; dyspareunia and vaginismus; and persistent sexual arousal disorder.

"Female sexual arousal disorder (FSAD)" is defined as a persistent or recurring inability to attain or maintain sufficient levels of sexual excitement, causing personal distress. FSAD encompasses both the lack of subjective feelings of excitement (i.e., subjective sexual arousal disorder) and the lack of somatic responses such as lubrication and swelling (i.e., genital/physical sexual arousal disorder). FSAD may be strictly psychological in origin, although it generally is caused or complicated by medical or physiological factors. Hypoestrogenism is the most common physiologic condition associated with FSAD, which leads to urogenital atrophy and a decrease in vaginal lubrication.

As used herein, "erectile dysfunction (ED)" is a male sexual dysfunction characterized by the inability to develop or maintain an erection of the penis during sexual performance. A penile erection is the hydraulic effect of blood entering and being retained in sponge-like bodies within the penis. The process is often initiated as a result of sexual arousal, when signals are transmitted from the brain to nerves in the penis. Erectile dysfunction is indicated when an erection is difficult to produce. The most important organic causes are cardiovascular disease and diabetes, neurological problems (for example, trauma from prostatectomy surgery), hormonal insufficiencies (hypogonadism) and drug side effects.

As used herein, the term "bronchoconstriction" is used to define the constriction of the airways in the lungs due to the tightening of surrounding smooth muscle, with consequent coughing, wheezing, and shortness of breath. The condition has a number of causes, the most common being as well as asthma. Exercise and allergies can bring on the symptoms in an otherwise asymptomatic individual. Other conditions such as chronic obstructive pulmonary disease (COPD) can also present with bronchoconstriction.

Specific diseases of disorders which may be treated and/or prevented by administering an sGC stimulator of the invention include, but are not limited to: hypertension (e.g., diabetic hypertension, arterial hypertension, pulmonary hypertension, resistant hypertension, peripheral artery disease, etc.), heart failure (e.g., left ventricular diastolic dysfunction (LVDD) and left ventricular systolic dysfunction (LVSD)), heart failure with preserved ejection fraction (HFpEF; also referred to as diastolic heart failure), heart failure with reduced ejection fraction (HFrEF; also referred to as systolic heart failure), sleep apnea associated with heart failure), arteriosclerotic disease (e.g., atherosclerosis), thromboembolic disorders (e.g., chronic thromboembolic pulmonary hypertension, thrombosis, stroke, embolism, pulmonary embolism), Alzheimer's disease, renal diseases (e.g., renal fibrosis, ischemic renal disease, renal failure, renal insufficiency, chronic kidney disease), hepatic disease (e.g., liver fibrosis or cirrhosis), respiratory disease (e.g., pulmonary fibrosis, asthma, chronic obstructive pulmonary disease, interstitial lung disease), sexual disorders (e.g., erectile dysfunction, male and female sexual dysfunction, vaginal atrophy), sickle cell anemia, neuro inflammatory diseases or disorders and metabolic disorders (e.g., lipid related disorders).

The solid dispersions, pharmaceutical formulations and dosage forms of the invention are useful in the prevention and/or treatment of the following types of diseases, conditions and disorders which can benefit from sGC stimulation or an upregulation of the NO pathway:
(1) Peripheral, pulmonary, hepatic, kidney, cardiac or cerebral vascular/endothelial disorders/conditions or diseases otherwise related to circulation:
   - disorders related to high blood pressure and decreased coronary blood flow such as increased acute and chronic coronary blood pressure, arterial hypertension and vascular disorder resulting from cardiac and renal complications (e.g., heart disease, stroke, cerebral ischemia, renal failure); heart failure with preserved ejection fraction (HFpEF; also referred to as diastolic heart failure), heart failure with reduced ejection fraction (HFrEF; also referred to as systolic heart failure), resistant hypertension, diabetic hypertension, congestive heart failure; diastolic or systolic dysfunction; coronary insufficiency; arrhythmias; reduction of ventricular preload; cardiac hypertrophy; heart failure/cardiorenal syndrome; portal hypertension; endothelial dysfunction or injury;
   - thromboembolic disorders and ischemias such as myocardial infarction, stroke, transient ischemic attacks (TIAs); obstructive thromboanginitis; stable or unstable angina pectoris; coronary spasms, variant angina, Prinzmetal's angina; prevention of restenosis after thrombolysis therapies; thrombogenic disorders;
   - peripheral arterial disease, peripheral occlusive arterial disease; peripheral vascular disease; hypertonia; Raynaud's syndrome or phenomenon, critical limb ischemia, vasculitis; peripheral embolism; intermittent claudication; vaso-occlusive crisis; Duchenne muscular dystrophy; Becker muscular dystrophy; microcirculation abnormalities; control of vascular leakage or permeability;
   - shock; sepsis; cardiogenic shock; control of leukocyte activation; inhibition or modulation of platelet aggregation;
   - pulmonary/respiratory conditions such as pulmonary hypertension, pulmonary arterial hypertension, and associated pulmonary vascular remodeling (e.g., localized thrombosis and right heart hypertrophy); pulmonary hypertonia; primary pulmonary hypertension, secondary pulmonary hypertension, familial pulmonary hypertension, sporadic pulmonary hypertension, pre-capillary pulmonary hypertension, idiopathic pulmonary hypertension, thrombotic pulmonary arteriopathy, plexogenic pulmonary arteriopathy; cystic fibrosis; bronchoconstriction or pulmonary bronchoconstriction; acute respiratory distress syndrome; lung fibrosis, lung transplant;
   - pulmonary hypertension associated with or related to: left ventricular dysfunction, hypoxemia, WHO groups I, II, III, IV and V hypertensions, mitral valve disease, constrictive pericarditis, aortic stenosis, cardiomyopathy, mediastinal fibrosis, pulmonary fibrosis, anomalous pulmonary venous drainage, pulmonary venooclusive disease, pulmonary vasculitis, collagen vascular disease, congenital heart disease, pulmonary venous hypertension, interstitial lung disease, sleep-disordered breathing, sleep apnea, alveolar hypoventilation disorders, chronic exposure to high altitude, neonatal lung disease, alveolar-capillary dysplasia, sickle cell disease, other coagulation disorders, chronic thromboembolism, pulmonary embolism (due to tumor, parasites or foreign material), connective tissue disease, lupus, schistosomiasis, sarcoidosis, chronic obstructive pulmonary disease, asthma, emphysema, chronic bronchitis, pulmonary capillary hemangiomatosis; histiocytosis X, lymphangiomatosis and compressed pulmonary vessels (such as due to adenopathy, tumor or fibrosing mediastinitis);
   - arterosclerotic diseases or conditions such as atherosclerosis (e.g., associated with endothelial injury, platelet and monocyte adhesion and aggregation, smooth muscle proliferation and migration); restenosis (e.g., developed after thrombolysis therapies, percutaneous transluminal angioplasties (PTAs), percutaneous transluminal coronary angioplasties (PTCAs) and bypass); inflammation;
   - cardiovascular disease associated with metabolic syndrome (e.g., obesity, dyslipidemia, diabetes, high blood pressure); lipid related disorders such as dyslipidemia, hypercholesterolemia, hypertriglyceridemia, sitosterolemia, fatty liver disease, and hepatitis; preeclampsia; polycystic kidney disease progression; subcutaneous fat; obesity;
   - liver cirrhosis, associated with chronic liver disease, hepatic fibrosis, hepatic stellate cell activation, hepatic fibrous collagen and total collagen accumulation; liver disease of necro-inflammatory and/or of immunological origin; and urogenital system disorders, such as renal fibrosis and renal failure resulting from chronic kidney diseases or insufficiency (e.g., due to accumulation/ deposition and tissue injury, progressive sclerosis, glomerulonephritis); prostate hypertrophy systemic sclerosis; cardiac interstitial fibrosis; cardiac remodeling and fibrosis; cardiac hypertrophy; non-alcoholic steatohepatitis or NASH;
(2) ischemia, reperfusion damage; ischemia/reperfusion associated with organ transplant, lung transplant, pulmonary transplant, cardiac transplant; conserving blood substituents in trauma patients;
(3) sexual, gynecological and urological disorders of conditions: erectile dysfunction; impotence; premature ejaculation; female sexual dysfunction (e.g., female sexual arousal dysfunction, hypoactive sexual arousal disorder), vaginal atrophy, dyspaneuria, atrophic vaginitis; benign prostatic hyperplasia (BPH) or hypertrophy or enlargement, bladder outlet obstruction; bladder pain syndrome (BPS), interstitial cystitis (IC), overactive bladder, neurogenic bladder and incontinence; diabetic nephropathy;
(4) ocular diseases or disorders: glaucoma, retinopathy, diabetic retinopathy, blepharitis, dry eye syndrome, Sjögren's Syndrome;
(5) hearing diseases or disorders: hearing impairment, partial or total hearing loss; partial or total deafness; tinnitus; noise-induced hearing loss;
(6) topical or skin disorders or conditions: dermal fibrosis, scleroderma, skin fibrosis;
(7) wound healing: for instance in diabetics; microvascular perfusion improvement (e.g., following injury, to counteract the inflammatory response in perioperative care), anal fissures, diabetic ulcers;
(8) other diseases or conditions: cancer metastasis, osteoporosis, gastroparesis; functional dyspepsia; diabetic complications, diseases associated with endothelial dysfunction, and neurologic disorders associated with decreased nitric oxide production; achalasia or esophageal achalasia.
(9) a CNS disease, health condition or disorder selected from Alzheimer's disease, amyotrophic lateral sclerosis (ALS or Lou Gehrig's disease), Down syndrome, dementia, vascular dementia, vascular cognitive impairment, Binswanger's dementia (subcortical arteriosclerotic encephalopathy), Cerebral Autosomal-Dominant Arteriopathy with Subcortical Infarcts and Leukoencephalopathy (CADASII, or CADASIL syndrome), frontotemporal lobar degeneration or dementia, HIV-associated dementia, Lewy body dementia, pre-senile dementia (mild cognitive impairment, MCI), glaucoma, Huntington's diseases (or chorea, HD), multiple sclerosis (MS), multiple system atrophy (MSA), Parkinson's disease, Parkinsonism Plus, spinocerebellar ataxias, Steel-Richardson-Olszewski disease (progressive supranuclear palsy), attention deficit disorder (ADD) or attention deficit hyperactivity disorder (ADHD);
(10) a CNS disorder or condition selected from Alzheimer's disease or pre-Alzheimer's disease, mild to moderate Alzheimer's disease or moderate to severe Alzheimer's disease;
(11) a CNS disorder is selected from either traumatic (closed or open, penetrating head injuries), traumatic brain injury (TBI), or nontraumatic (stroke, aneurism, hypoxia) injury to the brain or cognitive impairment or dysfunction resulting from brain injuries or neurodegenerative disorders;
(12) a CNS disease or disorder is selected from dystonias, including for example, generalized, focal, segmental, sexual, intermediate, acute dystonic reaction, and genetic/primary dystonia; and dyskinesias, including for example, acute, chronic/tardive, or non-motor or levo-dopa induced dyskinesia (LID);
(13) a CNS disease or disorder is selected from disorders characterized by a relative reduction in synaptic plasticity and synaptic processes including, for example, Fragile X, Rhett's disorder, Williams syndrome, Renpenning's syndrome, autism spectrum disorders, including autism, Asperger's syndrome, pervasive development disorder or childhood disintegrative disorder;
(14) a CNS disorder is neuropathic pain;
(15) a CNS disorder is a psychiatric, mental, mood or affective disorder selected from a bipolar disorder, schizophrenia, general psychosis, drug-induced psychosis, a delusional disorder, schizoaffective disorder, obsessive compulsive disorder (OCD), a depressive disorder, an anxiety disorder, a panic disorder, or post-traumatic stress disorder (PTSD); or
(16) a CNS disorder is selected from chemo brain, levo-dopa induced addictive behavior, alcoholism, narcotic dependence (including but not limited to amphetamine, opiates or other substances) or substance abuse.

In other embodiments of the invention, the solid dispersion, pharmaceutical formulations and solid dosage forms of the invention are useful in the prevention and/or treatment of the following types of diseases, conditions and disorders which can benefit from sGC stimulation or an upregulation of the NO pathway:

hypertension, resistant hypertension, diabetic hypertension, pulmonary hypertension (PH), pulmonary arterial hypertension, PH associated with COPD, chronic airflow obstruction, asthma or pulmonary fibrosis, thrombosis, embolism, thromboembolic disorders, Alzheimer's disease, atherosclerosis, right heart hypertrophy, heart failure, diastolic dysfunction, systolic dysfunction, sleep apnea associated with heart failure, liver cirrhosis, renal fibrosis, renal failure resulting from chronic kidney diseases or insufficiency, metabolic disorder, dyslipidemia, hypercholesterolemia, hypertriglyceridemia, sitosterolemia, fatty liver disease, hepatitis, erectile dysfunction, female sexual dysfunction, female sexual arousal dysfunction or vaginal atrophy.

In some embodiments, the invention relates to a method of treating a disease, health condition or disorder in a subject, comprising administering a therapeutically effective amount of a compound of any of the above depicted Formulae, or a pharmaceutically acceptable salt thereof, to the subject in need of treatment, wherein the disease, health condition or disorder is selected from one of the diseases listed above.

In other embodiments the disease, health condition or disorder is selected from a peripheral, pulmonary, hepatic, kidney, cardiac or cerebral vascular/endothelial disorder or condition, or a disease otherwise related to circulation selected from: increased acute and chronic coronary blood pressure, arterial hypertension and vascular disorder resulting from cardiac and renal complications, heart disease, stroke, cerebral ischemia, renal failure; resistant hypertension, diabetic hypertension, congestive heart failure; diastolic or systolic dysfunction; coronary insufficiency; arrhythmias; reduction of ventricular preload; cardiac hypertrophy; heart failure/cardiorenal syndrome; portal hypertension; endothelial dysfunction or injury; myocardial infarction; stroke or transient ischemic attacks (TIAs); obstructive thromboanginitis; stable or unstable angina pectoris; coronary spasms, variant angina, Prinzmetal's angina; restenosis as a result of thrombolysis therapies; or thrombogenic disorders.

In still other embodiments, the disease, health condition or disorder is selected from a peripheral vascular/endothelial disorder or condition or a disease otherwise related to circulation selected from: peripheral arterial disease, peripheral occlusive arterial disease; peripheral vascular disease; hypertonias; Raynaud's syndrome or phenomenon; critical limb ischemia; vasculitis; peripheral embolism; intermittent claudication; vaso-occlusive crisis; Duchenne and Becker muscular dystrophies; microcirculation abnormalities; or vascular leakage or permeability issues.

In further embodiments, the disease, health condition or disorder is a pulmonary disorder or condition or a disease otherwise related to circulation selected from: pulmonary hypertension; pulmonary arterial hypertension and associated pulmonary vascular remodeling; localized thrombosis; right heart hypertrophy; pulmonary hypertonia; primary pulmonary hypertension, secondary pulmonary hypertension, familial pulmonary hypertension, sporadic pulmonary hypertension, pre-capillary pulmonary hypertension, idiopathic pulmonary hypertension, thrombotic pulmonary arteriopathy, plexogenic pulmonary arteriopathy; cystic fibrosis; bronchoconstriction or pulmonary bronchoconstriction; acute respiratory distress syndrome; lung fibrosis and lung transplant. In some of these embodiments, the pulmonary hypertension is pulmonary hypertension associated with or related to: left ventricular dysfunction, hypoxemia, WHO groups I, II, III, IV and V hypertensions, mitral valve disease, constrictive pericarditis, aortic stenosis, cardiomyopathy, mediastinal fibrosis, pulmonary fibrosis, anomalous pulmonary venous drainage, pulmonary venooclusive disease, pulmonary vasculitis, collagen vascular disease, congenital heart disease, pulmonary venous hypertension, interstitial lung disease, sleep-disordered breathing, sleep apnea, alveolar hypoventilation disorders, chronic exposure to high altitude, neonatal lung disease, alveolar-capillary dysplasia, sickle cell disease, coagulation disorders, chronic thromboembolism; pulmonary embolism, due to tumor, parasites or foreign material; connective tissue disease, lupus, schistosomiasis, sarcoidosis, chronic obstructive pulmonary disease, asthma, emphysema, chronic bronchitis, pulmonary capillary hemangiomatosis; histiocytosis X; lymphangiomatosis; or compressed pulmonary vessels due to adenopathy, tumor or fibrosing mediastinitis.

In still other embodiments, the health condition or disorder is a vascular or endothelial disorder or condition or a disease otherwise related to circulation selected from: arterosclerotic diseases; atherosclerosis, atherosclerosis associated with endothelial injury, atherosclerosis associated with platelet and monocyte adhesion and aggregation, atherosclerosis associated with smooth muscle proliferation and migration; restenosis, restenosis developed after thrombolysis therapies; restenosis developed after percutaneous transluminal angioplasties; restenosis developed after percutaneous transluminal coronary angioplasties and bypass; inflammation; cardiovascular disease associated with metabolic syndrome, obesity, dyslipidemia, diabetes or high blood pressure; lipid related disorders, dyslipidemia, hypercholesterolemia, hypertriglyceridemia, sitosterolemia, fatty liver disease, and hepatitis; preeclampsia; polycystic kidney disease progression; or subcutaneous fat.

In yet other embodiments, the disease, health condition or disorder selected from liver cirrhosis, liver cirrhosis associated with chronic liver disease, hepatic fibrosis, hepatic stellate cell activation, hepatic fibrous collagen and total collagen accumulation; or liver disease of necro-inflammatory or of immunological origin.

In further embodiments, the disease, health condition or disorder is a urogenital system disorder selected from renal fibrosis; renal failure resulting from chronic kidney diseases or insufficiency; renal failure due to accumulation or deposition and tissue injury, progressive sclerosis or glomerulonephritis; or prostatic hypertrophy.

In further embodiments, the disease, health condition or disorder is systemic sclerosis.

In further embodiments, the disease, health condition or disorder is a cardiac disorder selected from cardiac interstitial fibrosis; cardiac remodeling and fibrosis or cardiac hypertrophy.

In some embodiments, the disorder is a CNS disease, health condition or disorder selected from Alzheimer's disease, amyotrophic lateral sclerosis (ALS or Lou Gehrig's disease), Down syndrome, dementia, vascular dementia, vascular cognitive impairment, Binswanger's dementia (subcortical arteriosclerotic encephalopathy), Cerebral Autosomal-Dominant Arteriopathy with Subcortical Infarcts and Leukoencephalopathy (CADASII, or CADASIL syndrome), frontotemporal lobar degeneration or dementia, HIV-associated dementia, Lewy body dementia, pre-senile dementia (mild cognitive impairment, MCI), glaucoma, Huntington's diseases (or chorea, HD), multiple sclerosis (MS), multiple system atrophy (MSA), Parkinson's disease, Parkinsonism Plus, spinocerebellar ataxias, Steel-Richardson-Olszewski disease (progressive supranuclear palsy), attention deficit disorder (ADD) or attention deficit hyperactivity disorder (ADHD).

In further embodiments, the disease, health condition or disorder is a CNS disorder or condition selected from Alzheimer's disease or pre-Alzheimer's disease, mild to moderate Alzheimer's disease or moderate to severe Alzheimer's disease.

In other embodiments, the CNS disorder is selected from either traumatic (closed or open, penetrating head injuries), traumatic brain injury (TBI), or nontraumatic (stroke, aneurism, hypoxia) injury to the brain or cognitive impairment or dysfunction resulting from brain injuries or neurodegenerative disorders.

In other embodiments, the CNS disease or disorder is selected from dystonias, including for example, generalized, focal, segmental, sexual, intermediate, acute dystonic reaction, and genetic/primary dystonia; and dyskinesias, including for example, acute, chronic/tardive, or non-motor or levo-dopa induced dyskinesia (LID).

In other embodiments, the CNS disease or disorder is selected from disorders characterized by a relative reduction in synaptic plasticity and synaptic processes including, for example, Fragile X, Rhett's disorder, Williams syndrome, Renpenning's syndrome, autism spectrum disorders, including autism, Asperger's syndrome, pervasive development disorder or childhood disintegrative disorder.

In other embodiments, the CNS disorder is neuropathic pain.

In other embodiments, the CNS disorder is a psychiatric, mental, mood or affective disorder selected from a bipolar disorder, schizophrenia, general psychosis, drug-induced psychosis, a delusional disorder, schizoaffective disorder, obsessive compulsive disorder (OCD), a depressive disorder, an anxiety disorder, a panic disorder, or post-traumatic stress disorder (PTSD).

In other embodiments, the CNS disorder is selected from chemo brain, levo-dopa induced addictive behavior, alcoholism, narcotic dependence (including but not limited to amphetamine, opiates or other substances) or substance abuse.

In some embodiments, the disease or disorder is achalasia or esophageal achalasia.

In other embodiments, the disease or disorder is non-alcoholic steatohepatitis or NASH.

In further embodiments, the disease, health condition or disorder is selected from ischemia, reperfusion damage; ischemia/reperfusion associated with organ transplant, lung transplant, pulmonary transplant or cardiac transplant; or conserving blood substituents in trauma patients.

In further embodiments, the disease, health condition or disorder is a sexual, gynecological or urological disorder of condition selected from erectile dysfunction; impotence; premature ejaculation; female sexual dysfunction; female sexual arousal dysfunction; hypoactive sexual arousal disorder; vaginal atrophy, dyspaneuria, atrophic vaginitis; benign prostatic hyperplasia (BPH) or hypertrophy or enlargement; bladder outlet obstruction; bladder pain syndrome (BPS); interstitial cystitis (IC); overactive bladder, neurogenic bladder; incontinence; or diabetic nephropathy.

In further embodiments, the disease, health condition or disorder is selected from vaginal atrophy, dyspaneuria or atrophic vaginitis.

In further embodiments, the disease, health condition or disorder is selected from benign prostatic hyperplasia (BPH) or hypertrophy or enlargement; bladder outlet obstruction; bladder pain syndrome (BPS); interstitial cystitis (IC); overactive bladder, neurogenic bladder or incontinence.

In further embodiments, the disease, health condition or disorder is a sexual, condition selected from erectile dysfunction; impotence; premature ejaculation; female sexual dysfunction; female sexual arousal dysfunction or hypoactive sexual arousal disorder.

In further embodiments, the disease or disorder is diabetic nephropathy.

In further embodiments, the disease or disorder is diabetic retinopathy.

In further embodiments, the disease or disorder is heart failure. In some embodiments, the heart failure is heart failure with preserved ejection fraction (HFpEF; also referred to as diastolic heart failure). In other embodiments, the heart failure is heart failure with reduced ejection fraction (HFrEF; also referred to as systolic heart failure).

In further embodiments, the disease, health condition or disorder is Duchenne dystrophy or Becker muscular dystrophy.

In further embodiments, the disease is an ocular disease or disorder selected from glaucoma, retinopathy, diabetic retinopathy, blepharitis, dry eye syndrome, or Sjögren's Syndrome.

In further embodiments, the disease is a hearing disease or disorder selected from hearing impairment, partial or total hearing loss; partial or total deafness; tinnitus; or noise-induced hearing loss.

In further embodiments, the disease is a topical or skin disorder or condition selected from dermal fibrosis, scleroderma, or skin fibrosis.

In further embodiments, the treatment involves wound healing; wound healing in diabetics; improvement of microvascular perfusion; improvement of microvascular perfusion issues following injury; treatment of anal fissures; or treatment of diabetic ulcers.

In further embodiments, the disease or condition is selected from cancer metastasis; osteoporosis; gastroparesis; functional dyspepsia; diabetic complications; diseases associated with endothelial dysfunction or neurologic disorders associated with decreased nitric oxide production.

The terms "disease", "disorder" and "condition" may be used interchangeably here to refer to an sGC, cGMP and/or NO mediated medical or pathological condition.

As used herein, the terms "subject" and "patient" are used interchangeably. The terms "subject" and "patient" refer to an animal (e.g., a bird such as a chicken, quail or turkey, or a mammal), specifically a "mammal" including a non-primate (e.g., a cow, pig, horse, sheep, rabbit, guinea pig, rat, cat, dog, and mouse) and a primate (e.g., a monkey, chimpanzee and a human), and more specifically a human. In some embodiments, the subject is a non-human animal such as a farm animal (e.g., a horse, cow, pig or sheep), or a pet (e.g., a dog, cat, guinea pig or rabbit). In some embodiments, the subject is a human.

The invention also provides a method for treating one of the above diseases, conditions and disorders in a subject, comprising administering solid amorphous dispersions, pharmaceutical compositions and dosage unit forms of the inventionto the subject in need of the treatment. Alternatively, the invention provides the use of solid amorphous dispersions, pharmaceutical compositions and dosage unit forms of the invention in the treatment of one of these diseases, conditions and disorders in a subject in need of the treatment. The invention further provides a method of making or manufacturing a medicament useful for treating one of these diseases, conditions and disorders comprising using a solid amorphous dispersion, pharmaceutical composition or dosage unit form of the invention. The term "biological sample", as used herein, refers to an *in vitro* or *ex vivo* sample, and includes, without limitation, cell cultures or extracts thereof; biopsied material obtained from a mammal or extracts thereof; blood, saliva, urine, faeces, semen, tears, lymphatic fluid, ocular fluid, vitreous humour, or other body fluids or extracts thereof.

The compounds and pharmaceutical compositions described herein can be used alone or in combination therapy for the treatment or prevention of a disease or disorder mediated, regulated or influenced by sGC, cGMP and/or NO.

Solid amorphous dispersions, pharmaceutical compositions and dosage unit forms here disclosed are also useful for veterinary treatment of companion animals, exotic animals and farm animals, including, without limitation, dogs, cats, mice, rats, hamsters, gerbils, guinea pigs, rabbits, horses, pigs and cattle.

In other embodiments, the invention provides a method of stimulating sGC activity in a biological sample, comprising contacting said biological sample with a solid dispersion, pharmaceutical composition or dosage unit form of the invention. Use of a solid dispersion, pharmaceutical composition or dosage unit form of the invention in a biological sample is useful for a variety of purposes known to one of skill in the art. Examples of such purposes include, without limitation, biological assays and biological specimen storage.

### EXAMPLES

### Equipment

X-ray powder diffractometer (XRPD)
Hydraulic manual tablet press
Turbula Shaker, Glen Mills
Analytical balance
Top loading balance
UPLC1: Waters Acquity UPLC
Incubator Shaker
Sonicator
Eppendorf Centrifuge
Tablet Hardness Tester
Differential Scanning Calorimeter (DSC)
Benchtop Spray Dryer: Buchi B290
Microscope/Digital Camera
Thermogravimetric Analyzer (TGA)
Vacuum Oven
Moisture Balance
8-Station Automated Tablet Press
V-Blender,
Thickness Tester
Benchtop Roller Compactor
Quadro Comill
Induction Sealer
Disintegration Tester
pH meter

### Example 1A: Preparation of Compound I in small scale

Compound I and its preparation were previously described in published patent application publication WO 2014/144100 (September 18, 2014). The description of Compound I and its preparation is included herein by reference.

### Example 1B: Large scale preparation of Compound I

### i): coupling of Compound (1') and N,O-Dimethylhydroxylamine to provide N-methoxy-N-methylisoxazole-3-carboxamide (2')

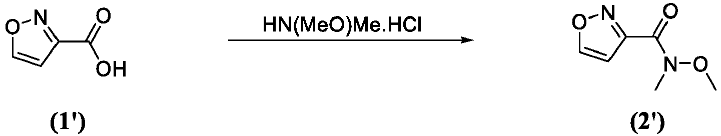

Isooxazole-3-carboxylic acid **((1'),** 241.6 g, 2137 mmols, 1.0 equiv.), toluene (1450 mL) and DMF (7.8 g, 107 mmols, 0.05 equiv.) were charged to a suitable reaction vessel equipped with a mechanical stirrer and a digital thermometer. The resulting slurry was heated to 45-50 °C. Oxalyl chloride (325 g, 2559 mmols, 1.2 equiv.) was then charged *via* an addition funnel over the course of 2 h while maintaining the reaction temperature between 45 to 50 °C and a vigorous gas evolution was observed. A brown mixture was obtained after addition. The brown mixture was heated to 87 to 92 °C over 1 h and stirred at 87 to 92 °C for 1 h. The reaction was complete by HPLC. During the heating the brown mixture turned into a dark solution. The reaction was monitored by quenching a portion of the reaction mixture into piperidine and monitoring the piperidine amide by HPLC. The dark mixture was cooled to 20-25 °C and then filtered through a sintered glass funnel to remove any insolubles. The dark filtrate was concentrated under reduced pressure to a volume of 400 mL dark oil.

Potassium carbonate (413 g, 2988 mmols, 1.4 equiv.) and water (1000 mL) were charged to a suitable reaction vessel equipped with a mechanical stirrer and a digital thermometer. The reaction solution was cooled to -10 to -5 °C. N,O-dimethylhydroxyamine hydrochloride (229 g, 2348 mmols, 1.1 equiv.) was charged to a suitable reaction vessel and dissolved in water (1000 mL). The N,O-dimethylhydroxyamine solution and dichloromethane (2500 mL) were then charged to the potassium carbonate solution.

The above dark oil (400 mL) was then charged slowly *via* an addition funnel while maintaining the reaction temperature -10 to 0 °C. The addition was a little exothermic and a brown mixture was obtained after addition. The mixture was stirred at 0 to 5 °C, over 20 min. and then warmed to 20 to 25 °C. The bottom organic layer was collected and the top aq. layer was extracted with dichloromethane (400 mL). The combined organic layers were washed with 15% sodium chloride solution (1200 mL). The organic layer was dried over magnesium sulfate and then filtered. The filtrate was concentrated under reduced pressure to give intermediate **(2')** as a dark oil (261.9 g, 97 wt.%, 76% yield, 3 wt.% toluene by ¹H-NMR, 0.04 wt.% water content by KF). ¹H-NMR (500 MHz, CDCl₃) δ ppm 8.48 (s, 1 H); 6.71(s, 1 H); 3.78 (s, 3 H); 3.38 (s, 3 H).

### ii): alkylation of Compound (2') and ethyl propiolate to provide (E)-ethyl 4-(isoxazol-3-yl)-2-(methoxy(methyl)amino)-4-oxobut-2-enoate (3')

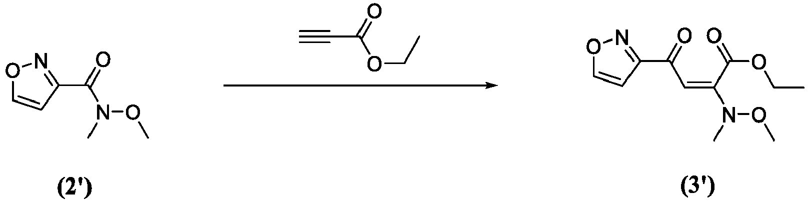

Intermediate **(2')** (72.2 g, 96 wt.%, 444 mmols, 1.0 equiv.), ethyl propiolate (65.7 g, 670 mmols, 1.5 equiv.) and anhydrous THF (650 mL) were charged to a suitable reaction vessel equipped with a mechanical stirrer and a digital thermometer. The solution was cooled to -65 to -55 °C. Sodium bis(trimethylsilyl)amide in THF (1 M, 650 mL, 650 mmols, 1.46 equiv.) was then charged slowly *via* an addition funnel while maintaining the reaction temperature -65 to -55 °C. The mixture was stirred below -55 °C over 10 min. after addition was complete. Then 1 N HCl (650 mL, 650 mmols, 1.46 equiv.) was charged to quench the reaction while maintaining the reaction temperature below -20 °C followed immediately with the addition of ethyl acetate (1500 mL) and water (650 mL). The top ethyl acetate layer was collected and the bottom aqueous layer was extracted with ethyl acetate (800 mL). The combined organic layers were washed with 10% citric acid (1000 mL) and saturated sodium chloride solution (650 mL). The organic layer was concentrated under reduced pressure to give a dark oil.

The dark oil was dissolved in a solution of dichloromethane/ethyl acetate/heptane (150mL/100mL/100mL). The solution was loaded on a silica pad (410 g) and the silica pad was eluted with ethyl acetate/heptane (1/1 v/v). The filtrate (~ 3000 mL) was collected and then concentrated under reduced pressure to a volume of 150 mL to give a slurry upon standing. Heptane (200 mL) was then added to the slurry and the slurry was concentrated under reduced pressure to a volume of 150 mL. The resulting slurry was filtered, and the filter cake was washed with heptane (150 mL). The filter cake was then air dried overnight to furnish intermediate **(3')** as a brown solid (63.4 g, 56% yield, >99% pure by HPLC). ¹H-NMR (500 MHz, CDCl₃) δ ppm 8.42 (d, *J*=1.53 Hz, 1 H); 6.76 (d, *J*=1.53 Hz, 1 H); 6.18 (s, 1 H); 4.47 (q, *J*=7.07 Hz, 2H); 3.75 (s, 3 H); 3.21 (s, 3 H); 1.41 (t, *J*=7.17 Hz, 3 H).

### iii): cyclization of Compound 3' and 2-fluorobenzylhydrazine to provide ethyl 1-(2-fluorobenzyl)-5-(isoxazol-3-yl)-1H-pyrazole-3-carboxylate (4')

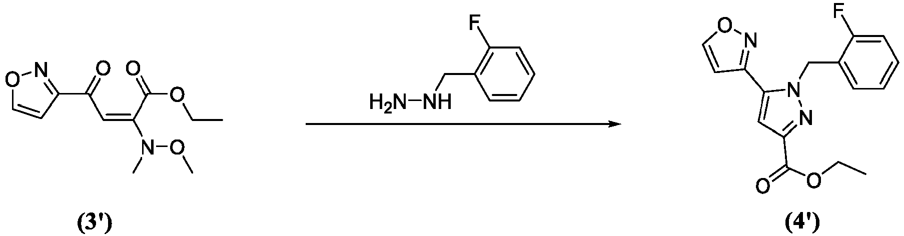

Intermediate **(3')** (72.9 g, 287 mmols, 1.0 equiv.) and absolute ethanol (730 mL) were charged to a suitable reaction vessel equipped with a mechanical stirrer and a digital thermometer. The mixture was cooled to 0 to 5 °C. 2-Fluorobenzylhydrazine (48.2 g, 344 mmols, 1.2 equiv.) was then charged to the mixture. The mixture was stirred at 0 to 10 °C over 1 h and then warmed to 20 to 25 °C and stirred at 20 to 25 °C over 16 h. The reaction was complete by HPLC. Concentrated HCl (33.9 g, 37 wt.%, 344 mmols, 1.2 equiv.) was charged to the reaction mixture over 1 min and the batch temperature exothermed from 20 °C to 38 °C. A slurry was obtained. The mixture was cooled to 0 to 10 °C over 1 h and stirred at 0-10 °C for 1 h. The resulting slurry was filtered, and the filter cake was washed with ethanol (200 mL). The filter cake was dried under vacuum at 30 to 40 °C over 16 h to furnish intermediate **(4')** as an off-white solid (81.3 g, 90% yield, >99% pure by HPLC). ¹H-NMR (500 MHz, CDCl₃) δ ppm 8.47 (d, *J*=1.68 Hz, 1 H); 7.15 - 7.26 (m, 2 H); 6.94 - 7.08 (m, 2H); 6.77 - 6.87 (m, 1 H); 6.55 (d, *J*=1.68 Hz, 1 H); 5.95 (s, 2 H); 4.43 (q, *J*=7.02 Hz, 2 H); 1.41 (t, *J*=7.17 Hz, 3 H).

### iv): amination of Compound (4') to provide 1-(2-fluorobenzyl)-5-(isoxazol-3-yl)-1H-pyrazole-3-carboximidamide hydrochloride (5'B)

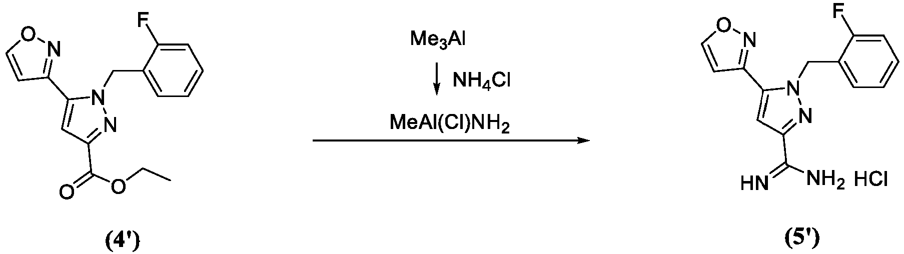

Anhydrous ammonium chloride (267 g, 4991 mmols, 5.0 equiv.) and toluene (5400 mL) were charged to a suitable reaction vessel equipped with a mechanical stirrer and a digital thermometer. Trimethylaluminum in toluene (2 M, 2400 mL, 4800 mmols, 4.8 equiv.) was charged slowly *via* an addition funnel while maintaining the reaction temperature at 20 to 40 °C (Note: Methane gas evolution was observed during addition). Then the mixture was heated to 75 to 80 °C over 30 min. and a clear white solution was obtained. Intermediate **(4')** (315 g, 999 mmols, 1.0 equiv.) was charged to reaction mixture in four equal portions over 1 h at 75 to 90 °C. The reaction was stirred at 80 to 90 °C over 30 min. and then heated to 100 to 110 °C and stirred at 100 to 110 °C over 3 h. The reaction was complete by HPLC. The reaction mixture was cooled to 10 to 20 °C and methanol (461 g, 14.4 mols, 14.4 equiv.) was charged slowly *via* an addition funnel while maintaining the reaction temperature 10-40 °C. (Note: very exothermic quench and a lot gas evolution was observed). A thick slurry was obtained. A 3N HCl (6400 mL, 3 N, 19.2 mols, 19.2 equiv.) was then charged slowly *via* an addition funnel while maintaining the reaction temperature at 20 to 45 °C. The mixture was heated to 80 to 85 °C and stirred at 80 to 85 °C over 10 min. to obtain a clear biphasic mixture. The mixture was cooled to 0 to 5 °C over 3 h and stirred at 0 to 5 °C over 1 h. The resulting slurry was filtered, and the filter cake was washed with water (3000 mL). The filter cake was dried under vacuum at 40 to 50 °C over 24 h to furnish intermediate **(5'B)** as an off-white solid (292 g, 91% yield, >99% pure by HPLC). ¹H-NMR (500 MHz, DMSO-*d*₆) δ ppm 9.52 (s, 2 H); 9.33 (s, 2 H); 9.18 (d, *J*=1.53 Hz, 1 H); 7.88 (s, 1 H); 7.29 - 7.38 (m, 1 H); 7.19 - 7.25 (m, 1 H); 7.10 - 7.16 (m, 1 H); 7.03 (d, *J*=1.53 Hz, 1 H); 6.92 - 6.98 (m, 1 H); 5.91 (s, 2 H). M.P. 180-185 °C.

### v): cyclization of Compound (5'B) and diethyl fluoromalonate to provide 5-fluoro-2-(1-(2-fluorobenzyl)-5-(isoxazol-3-yl)-1H-pyrazol-3-yl)pyrimidine-4,6-diol (6')

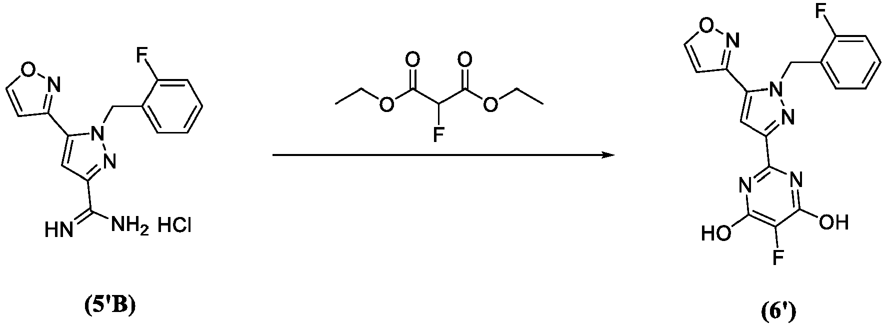

Intermediate **(5'B)** (224.6 g, 698 mmols, 1.0 equiv.), methanol (2250 mL) and diethyl fluoromalonate (187 g, 1050 mmols, 1.5 equiv.) were charged to a suitable reaction vessel equipped with a mechanical stirrer and a digital thermometer. Then sodium methoxide in methanol solution (567 g, 30 wt. %, 3149 mmols, 4.5 equiv.) was charged *via* an addition funnel while maintaining the reaction temperature 20 to 35 °C. The mixture was stirred at 20 to 35 °C over 30 min. and a light suspension was obtained. The reaction was complete by HPLC. A solution of 1.5 N HCl (2300 mL, 3450 mmols, 4.9 equiv.) was charged *via* an addition funnel over 1 h while maintaining the reaction temperature 20 to 30 °C. A white suspension was obtained. The pH of the reaction mixture was to be ~1 by pH paper. The slurry was stirred at 20 to 30 °C over 30 min. The resulting slurry was filtered, and the filter cake was washed with a pre-mixed solution of methanol and water (500 mL/500 mL), and then with water (1000 mL). The filter cake was dried under vacuum at 50 to 60 °C over 16 h to furnish intermediate **(6')** as an off-white solid (264 g, 97% yield, >99% pure by HPLC). ¹H-NMR (500 MHz, DMSO-*d₆*) δ ppm 12.82 (br. s., 1 H); 12.31 (br. s., 1 H); 9.14 (d, *J*=1.53 Hz, 1 H); 7.55 (s, 1 H); 7.31 - 7.37 (m, 1 H); 7.18 - 7.25 (m, 1 H); 7.10 - 7.15 (m, 2 H); 6.97 - 7.02 (t, *J*=7.55 Hz, 1 H); 5.88 (s, 2 H).

### vi): chlorination of Compound (6') to provide 3-(3-(4,6-dichloro-5-fluoropyrimidin-2-yl)-1-(2-fluorobenzyl)-1H-pyrazol-5-yl)isoxazole (7')

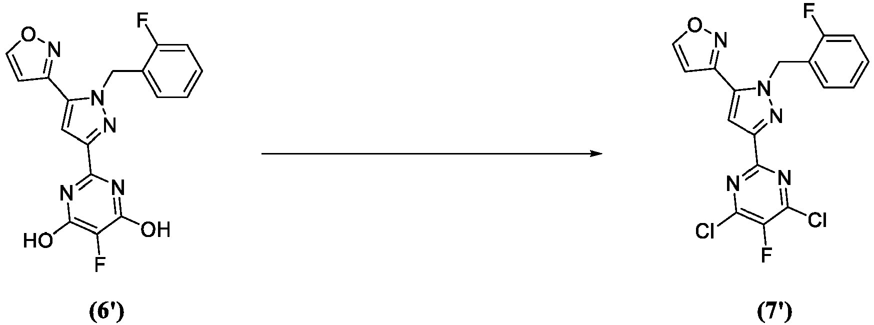

Intermediate **(6')** (264 g, 711 mmols, 1.0 equiv.), acetonitrile (4000 mL) and N,N-dimethylaniline (138 g, 1137 mmols, 1.6 equiv.) were charged to a suitable reaction vessel equipped with a mechanical stirrer and a digital thermometer. The slurry mixture was heated to 70-80 °C. Then phosphorous oxychloride (655 g, 4270 mmols, 6.0 equiv.) was charged *via* an addition funnel over 1 h while maintaining the reaction temperature 70 to 80 °C. The mixture was stirred at 75 to 80 °C over 22 h and a brown solution was obtained. The reaction was complete by HPLC. Then the mixture was cooled to 0 to 5 °C and cotton like solids precipitated out at 25°C. Water (3000 mL) was charged slowly *via* an addition funnel while maintaining the reaction temperature at 0 to 10 °C. The slurry was stirred at 0 to 10 °C over 30 min. The resulting slurry was filtered, and the filter cake was washed with a pre-mixed solution of acetonitrile and water (500 mL/500 mL). The filter cake was dried under vacuum at 35 to 45 °C over 16 h to furnish intermediate **(7')** as an off-white solid (283 g, 98% yield, >99% pure by HPLC). ¹H-NMR (500 MHz, CDCl₃) δ ppm 8.48 (d, *J*=1.68 Hz, 1 H); 7.44 (s, 1 H); 7.19 - 7.25 (m, 1 H); 6.96 - 7.08 (m, 2 H); 6.81 - 6.88 (m, 1 H); 6.60 (d, *J*=1.68 Hz, 1 H); 6.03 (s, 2 H).

### vii): substitution of Compound (7') with methoxide to provide 3-(3-(4-chloro-5-fluoro-6-methoxypyrimidin-2-yl)-1-(2-fluorobenzyl)-1H-pyrazol-5-yl)isoxazole (8')

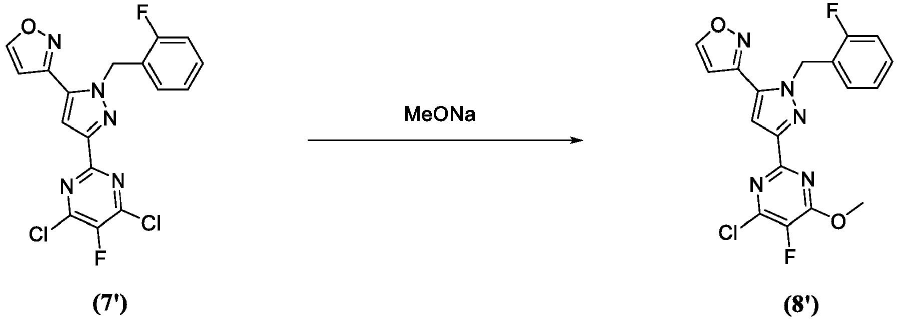

Methanol (3400 mL) and sodium methoxide in methanol (154 mL, 5.4 M, 832 mmols, 1.2 equiv.) were charged to a suitable reaction vessel equipped with a mechanical stirrer and a digital thermometer. The reaction mixture was heated to 23 to 27 °C. Intermediate (7') (283 g, 693 mmols, 1.0 equiv.) was charged to the mixture in small portions (5-10 g each portion) over 40 min while maintaining the reaction temperature 23 to 27 °C. The slurry was stirred at 23 to 27 °C over 30 min. The reaction was complete by HPLC. The resulting slurry was filtered, and the filter cake was washed with methanol (850 mL) and then water (850 mL). The filter cake was dried under vacuum at 35 to 45 °C over 16 h to furnish intermediate **(8')** as an off-white solid (277 g, 99% yield, 97% pure by HPLC). ¹H-NMR (500 MHz, CDCl₃) δ ppm 8.47 (d, *J*=1.83 Hz, 1 H); 7.38 (s, 1 H); 7.18 - 7.25 (m, 1 H); 7.01 - 7.08 (m, 1 H); 6.94 - 7.00 (m, 1 H); 6.81 - 6.88 (m, 1 H); 6.60 (d, *J*=1.68 Hz, 1 H); 6.00 (s, 2 H); 4.21 (s, 3 H).

### viii): hydrogenation of Compound (8') to provide 3-(3-(5-fluoro-4-methoxypyrimidin-2-yl)-1-(2-fluorobenzyl)-1H-pyrazol-5-yl)isoxazole (9')

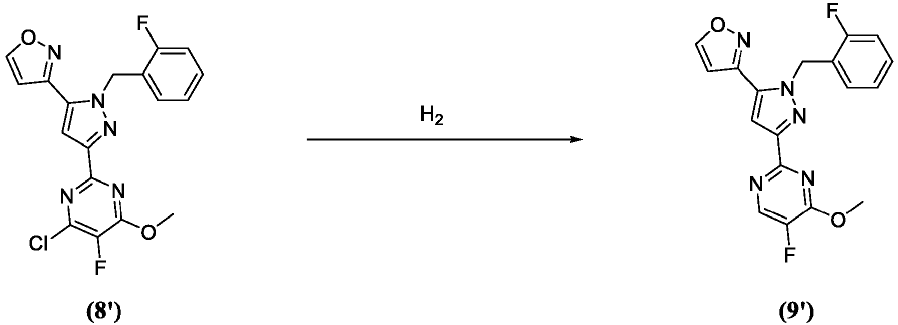

Intermediate **(8')** (226 g, 560 mmols, 1.0 equiv.), palladium (10% on activated carbon, nominally 50% water wet, 22.6 g), tetrahydrofuran (3400 mL) and triethylamine (91 g, 897 mmols, 1.6 equiv.) were charged to a suitable reaction vessel equipped with a mechanical stirrer and a digital thermometer. Nitrogen was bubbled into the reaction mixture via Teflon tubing over 10 min. at 20 to 30 °C. Then the mixture was heated to 40 to 50 °C and hydrogen gas was bubbled into the reaction mixture via Teflon tubing over 6 h while maintaining the reaction temperature 40 to 50 °C. The reaction was complete by HPLC. Nitrogen was then bubbled into the reaction mixture via Teflon tubing over 10 min. at 40 to 50 °C. The reaction mixture was hot filtered through Hypo Supercel^{™} and the filter cake was washed with tetrahydrofuran (2000 mL). The filtrate was concentrated under reduced pressure to a volume of ~1300 mL to give a slurry. Tetrahydrofuran was then solvent exchanged to methanol under reduced pressure via continuously feeding methanol (3000 mL). The final volume after solvent exchange was 1300 mL. The resulting slurry was filtered, and the filter cake was washed with methanol (500 mL). The filter cake was dried under vacuum at 20 to 25 °C over 16 h to furnish intermediate **(9')** as a white solid (192 g, 93% yield, 98% pure by HPLC). ¹H-NMR (500 MHz, CDCl₃) δ ppm 8.47 (d, *J*=1.68 Hz, 1 H); 8.41 (d, *J*=2.59 Hz, 1 H); 7.36 (s, 1 H); 7.17 - 7.24 (m, 1 H); 6.95 - 7.07 (m, 2 H); 6.83 - 6.90 (m, 1 H); 6.60 (d, *J*=1.68 Hz, 1 H); 5.99 (s, 2 H); 4.19 (s, 3 H).

### ix: demethylation of Compound (9') to provide 5-fluoro-2-(1-(2-fluorobenzyl)-5-(isoxazol-3-yl)-1H-pyrazol-3-yl)pyrimidin-4-ol (10')

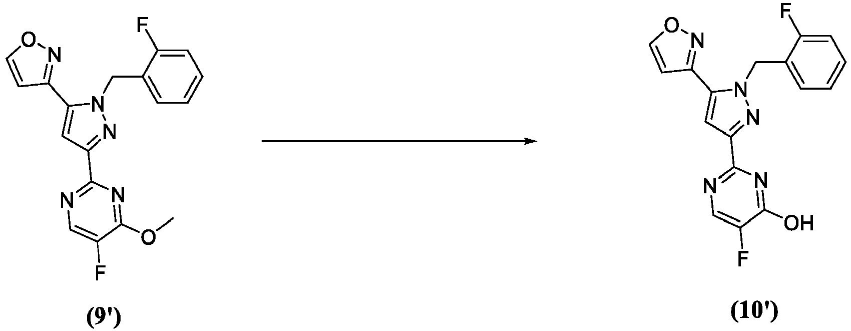

Intermediate **(9')** (230 g, 623 mmols, 1.0 equiv.), MeOH (3450 mL) and conc. HCl (307 g, 37 wt%, 3117 mmols, 5.0 equiv.) were charged to a suitable reaction vessel equipped with a mechanical stirrer and a digital thermometer. The mixture was heated to 60 to 65 °C and a solution was obtained. The mixture was then stirred at 60 to 65 °C over 17 h and a slurry was obtained. The reaction was complete by HPLC. The slurry was cooled to 20 to 25 °C over 2 h and stirred at 20 to 25 °C over 30 min. The resulting slurry was filtered, and the filter cake was washed with methanol (1000 mL). The filter cake was dried under vacuum at 35 to 45 °C over 16 h to furnish intermediate **(10')** as a white solid (214 g, 97% yield, >99% pure by HPLC). ¹H-NMR (500 MHz, DMSO-*d₆*) δ ppm 12.90 - 13.61 (br. s., 1 H); 9.11 (d, *J*=1.68 Hz, 1 H); 8.16 (s, 1 H); 7.64 (s, 1 H); 7.29 - 7.42 (m, 1 H); 7.17 - 7.28 (m, 2 H); 7.08 - 7.15 (m, 1 H); 6.97 (s, 1 H); 5.91 (s, 3 H).

### x): chlorination of Compound (10') to provide 3-(3-(4-chloro-5-fluoropyrimidin-2-yl)-1-(2-fluorobenzyl)-1H-pyrazol-5-yl)isoxazole (Formula IV)

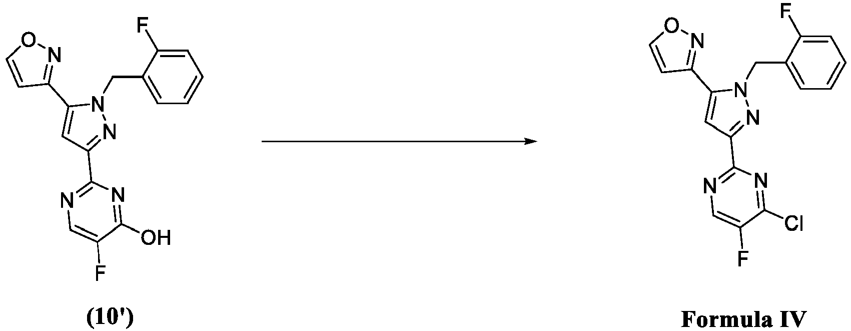

Intermediate **(10')** (214 g, 602 mmols, 1.0 equiv.), acetonitrile (3000 mL) and *N,N-*dimethylaniline (109 g, 899 mmols, 1.5 equiv.) were charged to a suitable reaction vessel equipped with a mechanical stirrer and a digital thermometer. The slurry mixture was heated to 70 to 80 °C. Then phosphorous oxychloride (276 g, 1802 mmols, 3.0 equiv.) was charged *via* an addition funnel over 30 min. while maintaining the reaction temperature 70-80 °C. The mixture was stirred at 75 to 80 °C over 2 h and a green solution was obtained. The reaction was complete by HPLC. Then the mixture was cooled to 0 to 5 °C. Water (1500 mL) was charged slowly *via* an addition funnel while maintaining the reaction temperature 0 to 10 °C. The slurry was stirred at 0 to 10 °C over 30 min. The resulting slurry was filtered, and the filter cake was washed with a pre-mixed solution of acetonitrile and water (500 mL/500 mL) and water (500 mL). The filter cake was dried under vacuum at 30 to 40 °C over 16 h to furnish intermediate of **Formula IV** as an off-white to pink solid (214 g, 95% yield, >99% pure by HPLC). ¹H NMR (500 MHz, CDCl₃) δ ppm 8.65 (s, 1 H); 8.48 (d, *J*=1.68 Hz, 1 H); 7.44 (s, 1 H); 7.21 - 7.25 (m, 1 H); 6.97 - 7.06 (m, 2 H); 6.83 - 6.87 (m, 1 H); 6.61 (d, *J*=1.68 Hz, 1 H); 6.03 (s, 2 H).

### a): amination of Compound 12 to provide 2-(aminomethyl)-1,1,1,3,3,3-hexafluoropropan-2-ol (14)

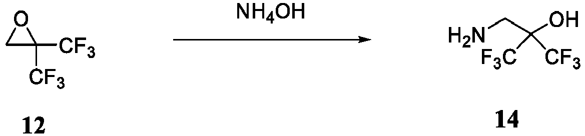

Ammonium hydroxide (29% (as NH₃) solution in water, 354 mL, 5435 mmols, 9.7 equiv.) and methyl t-butyl ether (354 mL) were charged to a suitable reaction vessel fitted with a mechanical stirrer and a digital thermometer. (Note: The condenser temperature was set to be - 20 °C and to minimize the evaporation of ammonium hydroxide). 2,2-Bis(trifluoromethyl)oxirane **((12),** 101 g, 561 mmols, 1.0 equiv.) was charged *via* an addition funnel over 40 min. while maintaining the reaction temperature at 20 to 26 °C. The mixture was stirred at 20 to 26 °C over 3 h after addition. The mixture was allowed to separate and the bottom aqueous layer was extracted with methyl t-butyl ether (2 x 354 mL). The combined organic layers were concentrated under reduced pressure to bring the volume to 303 mL. Methyl t-butyl ether (354 mL) was added and the mixture was concentrated under reduced pressure to bring the volume to 303 mL. Heptane (303 mL) was added and the mixture was concentrated under reduced pressure to bring the volume to 303 mL. The slurry was filtered and the filter cake was washed with heptane (100 mL). The solid was dried in the hood at 20 to 25 °C over 2 h until at constant weight to provide intermediate **(14)** as a white solid. (79.5 g, 71% yield,). ¹H NMR (500 MHz, MeOD) δ ppm 3.09 (s, 2 H).

### b): coupling of compound of Formula IV and Compound 14 to provide 1,1,1,3,3,3-hexafluoro-2-(((5-fluoro-2-(1-(2-fluorobenzyl)-5-(isoxazol-3-yl)-1H-pyrazol-3-yl)pyrimidin-4-yl)amino)methyl)propan-2-ol (Compound I)

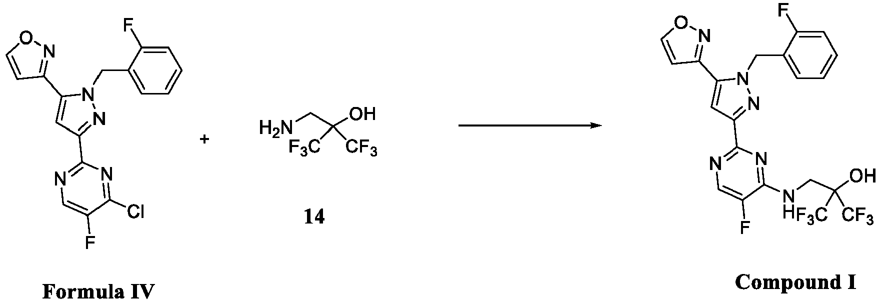

Intermediate of **Formula IV** (133 g, 356 mmols, 1.0 equiv.), intermediate **(14)** in a dimethyl sulfoxide solution (352 g, 60 wt%, 1071 mmols, 3.0 equiv.) and dimethyl sulfoxide (1200 mL) were charged to a suitable reaction vessel fitted with a mechanical stirrer and a digital thermometer. The reaction mixture was heated to 125 to 130 °C and stirred at 125 to 130 °C over 4 h. The reaction was complete by HPLC. Then the mixture was cooled to 20 to 25 °C. Methyl t-butyl ether (3800 mL) and water (2600 mL) were then charged to reaction mixture. The organic layer was washed with a saturated sodium bicarbonate solution (1000 mL) and with 1 N HCl solution (1000 mL) and then concentrated under reduced pressure to a volume of 1500 mL. The organic solution was loaded on a silica pad (800 g) and the silica pad was eluted with methyl t-butyl ether. The clean fractions were collected and concentrated under reduced pressure to a volume of 2000 mL. The MTBE solution was heated to 45 to 55 °C and heptane (2000 mL) was charged *via* an addition funnel over 30 min while maintaining the reaction temperature 45 to 55 °C to obtain a slurry. The slurry was cooled to 20 to 25 °C and stirred at 20 to 25 °C over 30 min. The resulting slurry was filtered, and the filter cake was washed with a pre-mixed solution of MTBE and heptane (400 mL/600 mL). The filter cake was then dried under vacuum at 45 to 55 °C over 5 hr. to furnish Compound I as an off-white solid (130 g, 68% yield, >99% pure by HPLC). ¹H NMR (500 MHz, DMSO-*d₆*) δ ppm 9.11 (d, *J*=1.96 Hz, 1 H); 8.66 (s, 1 H); 8.37 (d, *J*=3.13 Hz, 1 H); 8.11 (t, *J*=5.87 Hz, 1 H); 7.48 (s, 1 H); 7.30 - 7.37 (m, 1 H); 7.17 - 7.24 (m, 1 H); 7.21 (d, *J*=1.7 Hz, 1 H); 7.06 - 7.13 (m, 1 H); 7.00 - 7.06 (m, 1 H); 5.87 (s, 2 H); 4.11 (d, *J*=5.87 Hz, 2 H).

### Example 2: spray drying of Compound I alone and with polymers as spray dried dispersions and physical stability of these powders

### Materials used:

Cellulose acetate phthalate (CAP), from Fluka; HPMCAS-MG (HPMCAS-M), from Shin Etsu; Compound I.

### Procedure:

10 g of Compound I were weighed into a 250 mL Pyrex bottle. 100 mL of tetrahydrofuran (THF) were then added and the mixture stirred until Compound I was fully dissolved. This solution was used to prepare a spray dried dispersion of neat Compound I.

5 g of Compound I were placed into each of three 250 mL Pyrex bottles. 5 g of each of the polymers were then added, each polymer to a different Pyrex bottle. 100 mL of THF was then added to each bottle and the contents were stirred until dissolved.

Each of the solutions prepared above was spray dried with the following conditions: Inlet temp: 100°C; Outlet temp: ~ 35-40°C based on the inlet temp; Aspirator: 100%; Pump: 35%; a high efficiency cyclone was used.

The spray dried product obtained for each batch was then collected and evaluated by XRPD. Three spray-dried solid dispersions of Compound I were evaluated:
**Dispersion 1:** Spray dried neat Compound I
**Dispersion 2:** Spray dried dispersion Compound I:CAP (50:50)
**Dispersion 4:** Spray dried dispersion Compound I:HPMCAS-M (50:50)

The following XRPD method was used to analyze the dispersions: scan 5-45° 2-theta, 0.02° step size, 1 second per step on low background holder.

After the initial spray-dried solid dispersions were evaluated by XRPD, they were placed in vials in the stability chambers and evaluated again by XRPD after 1 month. Two different stability chambers at 25°C/60 % relative humidity (RH) (Stability Chamber 1) and 40°C/75% RH (Stability Chamber 2) were used. The containers were not sealed and no desiccant was used.

The XRPD for the spray dried neat Compound I alone was performed shortly after production (2 hours) and showed some crystalline peaks, indicating that the neat amorphous form of Compound I was not stable.

The XRPD for the dispersions with polymers was measured after 1 month at the stability conditions and did not show any crystalline peaks, indicating that these were stable for at least 1 month under the given conditions.

Spray dried dispersions of Compound I with cellulose acetate phthalate (CAP) and hydroxypropylmethyl cellulose acetate succinate (HPMCAS-M) were stable after 1 month as measured by XRPD at 25°C/60% RH (Stability Chamber 1) and 40°C/75% RH (Stability Chamber 2).

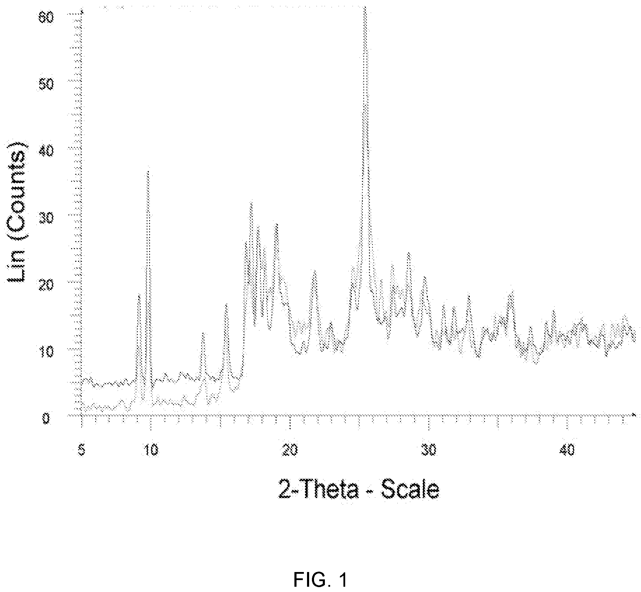

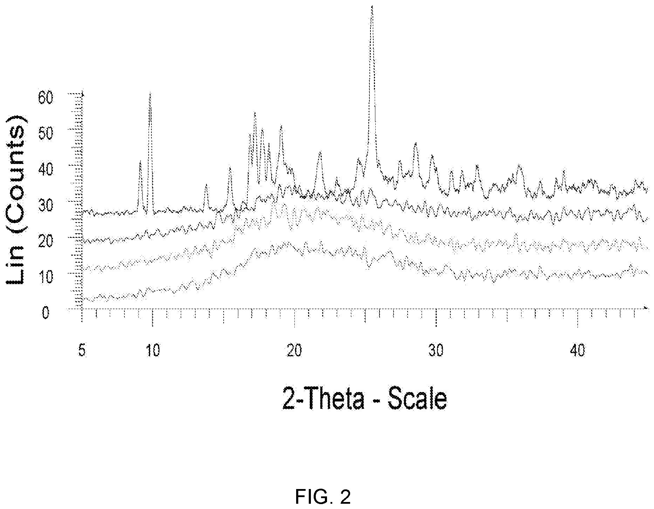

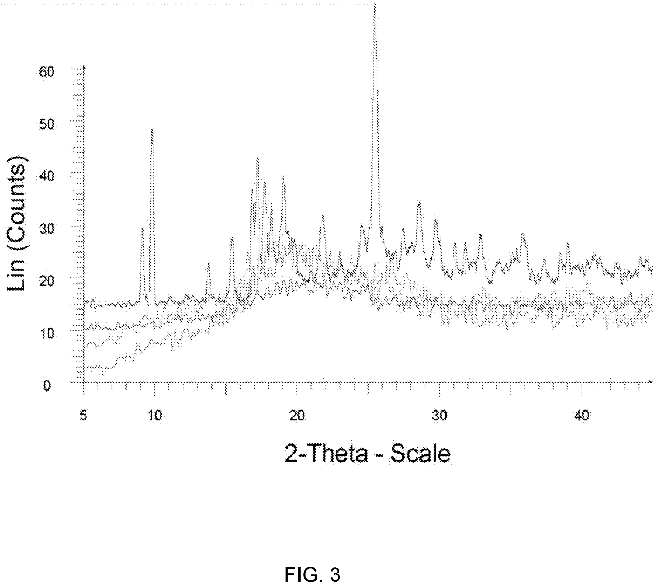

XRPD graphs for each of the Dispersions are depicted in FIG. 1, FIG. 2 and FIG. 3. FIG. 1 shows the XRPD results for crystalline Compound I (darker line) and spray dried neat Compound I (lighter line) after two hours of production. FIG. 2 shows the XRPD results for a solid dispersion of amorphous Compound I with CAP (50:50) at time 0 versus after 1 month in Stability Chamber 1 or Stability Chamber 2, unsealed, with no desiccant. The XRPD lines in order, from top to bottom at the left of the graph, are as follows: Compound I; Compound I: CAP (50:50) at time 0; Compound I: CAP (50:50) after 1 month in Stability Chamber 1; and Compound I: CAP (50:50) after 1 month in Stability Chamber 2. FIG. 3 shows the XRPD results for a solid dispersion of amorphous Compound I with HPMCAS-M (50:50) at time 0 versus after 1 month in Stability Chamber 1 or Stability Chamber 2, unsealed, with no desiccant. The XRPD lines in order, from top to bottom at the left of the graph, are as follows: Compound I; Compound I: HPMCAS-M (50:50) at time 0; Compound I: HPMCAS-M (50:50) after 1 month in Stability Chamber 1; and Compound I: HPMCAS-M (50:50) after 1 month in Stability Chamber 2.

### Example 3: Stability of spray dried dispersion generated in Example 2

In Example 2, dispersions were produced with cellulose acetate phthalate (CAP) and hydroxypropylmethylcellulose acetate succinate (HPMCAS-M) (Dispersions 2 and 4, respectively), placed on stability and evaluated for physical stability after 1 month. In this study, the evaluation of stability was continued.

Of the two dispersions that were placed on stability, when these were stored in containers not protected from moisture (i.e., not induction sealed and not stored with desiccant), all showed at least a small amount of recrystallization after 4 months at 40°C/75%RH (Stability Chamber 2). The dispersion with CAP appeared to show the least amount of recrystallization as indicated by microscopy.

### Materials

Dispersion 2 and Dispersion 4 generated in Example 2 were analyzed using three different techniques: XRPD, DSC and microscopy.

### Procedure

Conditions previously described in Example 2 were used for XRPD analysis.

DSC method: Modulated DSC (mDSC); Pan: Hermetic sealed pan with a pinhole added; Equilibrate at 0.00 °C; Modulate ± 1.00 °C every 60 s; Isothermal for 5.00 min; Ramp 1.50 °C/min to 200.00 °C.

Microscopy method: Performed with 20X objective lens under non-polarized light. Images were obtained for each dispersion from each stability condition (Stability Chamber 1 and Stability Chamber 2).

### Results

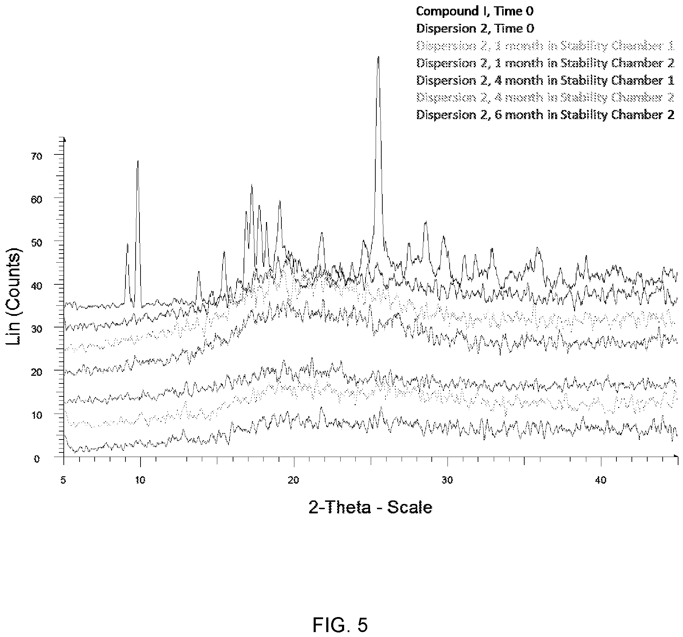

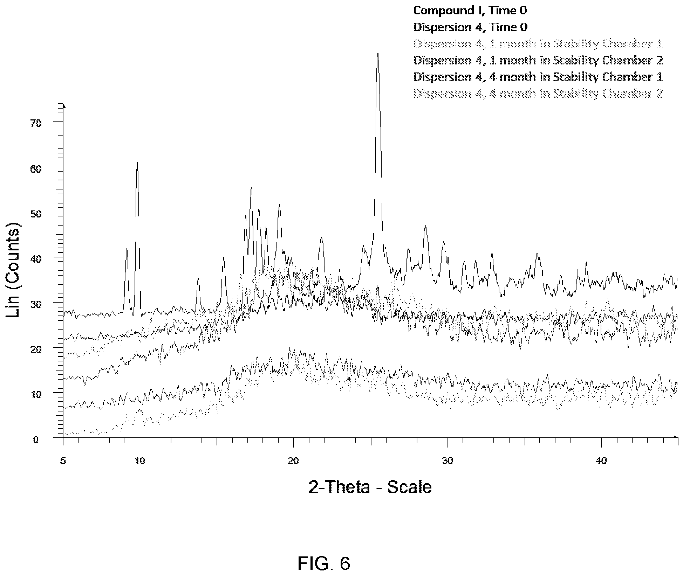

Clear crystallization peaks could not be identified by XRPD for the dispersions with CAP (Dispersion 2) or HPMCAS-M (Dispersion 4) after 4 months. Results are shown in Figure 5 (solid dispersion 2, prepared in Example 2) and Figure 6 (solid dispersion 4, prepared in Example 2).

mDSC indicated the following:
Dispersion 2: after 4 months in Stability Chamber 1, the Tg was 91°C; after 4 months in Stability Chamber 2, an exothermic peak was present at about 100°C, indicating possible recrystallization of Compound I under the conditions of Stability Chamber 2.
Dispersion 4: after 4 months at 25°C/60%RH, the Tg was 72°C; after 4 months at 40°C/75%RH, an exothermic peak was present at about 100°C indicating possible recrystallization of Compound I under the conditions of Stability Chamber 2.

Microscopy indicated the following:
Dispersion 2: no morphology change is observed at either stability condition after 4 months.
Dispersion 4: no morphology change is observed after storage at 25°C/60% RH; after storage at 40°C/75% RH, the formation of several small needles/rods (the crystalline morphology of Compound I) is visible, indicating that recrystallization has occurred.

### Example 4: spray drying of Compound I with additional polymers

Spray dried dispersions of amorphous Compound I were produced at a ratio of 50:50 Compound I:polymer in Example 2 and their stability assessed after 1 month in Example 2 and after 4 months in Example 3. In this example, dispersions with the same polymers were produced at a ratio of 25:75 Compound I:polymer. In addition, spray dried solid dispersions of amorphous Compound I were produced at a ratio of 25:75 Compound I:polymer with three additional polymers: Hydroxypropylmethylcellulose phthalate (HPMCP), PVP K29/32 (a polyvinylpyrrolidone polymer), and Plasdone S630 (a copolymer of polyvinylpyrrolidone and polyvinylacetate).

The resulting solid dispersions were evaluated by XRPD for stability at varying time points after being placed under the conditions of Stability Chamber 1 and Stability Chamber 2.

### Materials:

Compound I; Cellulose acetate phthalate (CAP), from Fluka; Hydroxypropylmethylcellulose acetate succinate (HPMCAS-M), MG Grade, from Shin Etsu; Hydroxypropylmethylcellulose phthalate (HPMCP), HP-50 Grade, from Shin Etsu ; Hydroxypropylmethylcellulose (HPMC), Methocel E5 Premium LV, from Dow; Hydroxypropylcellulose (HPC), LH-21 Grade, from Shin Etsu; Eudragit L100-55, from Evonik; Polyvinylpyrrolidone (PVP), Plasdone K29/32, from Ashland; Copolymer of polyvinylpyrrolidone and polyvinylacetate (Plasdone), Plasdone S630, from Ashland; Desiccant, Sorb-IT.

### Procedure:

As CAP and HPMCAS-M were previously used in spray drying of Compound I from tetrahydrofuran (THF), the solubility of these polymers in the solvent was already known. HPMCP, HPMC, HPC, Eudragit, PVP, and Plasdone were added at approximately 1 g into 20 mL scintillation vials. 10 mL of THF was added to each vial and the vials were vortexed and sonicated for 1 hour to determine if these polymers were soluble. HPMCP, PVP, and Plasdone were soluble at this concentration and were selected for inclusion to be spray dried with Compound I.

For preparation of the 50:50 dispersions, 5 g Compound I and 5 g of the polymer were weighed into 250 mL Pyrex bottles. 100 mL of THF were then added and the mixtures stirred until fully dissolved.

For preparation of the 25:75 dispersions, 5 g of Compound I and 15 g of the polymer were weighed into 250 mL Pyrex bottles. 100 mL of THF were then added and stirred until fully dissolved.

Each of the solutions was spray dried with the conditions described in Example 2.

The following spray dried solid dispersions of amorphous Compound I were produced:
**Dispersion 5:** 50:50 Compound I:CAP
**Dispersion 6:** 25:75 Compound I:CAP
**Dispersion** 9: 50:50 Compound I:HPMCAS-M
**Dispersion 10:** 25:75 Compound I:HPMCAS-M
**Dispersion 11:** 25:75 Compound I:HPMCP
**Dispersion 12:** 25:75 Compound I:PVP
**Dispersion 13:** 25:75 Compound I:Plasdone

The spray dried dispersion obtained for each batch was collected and evaluated by XRPD within a maximum of 5 days of production, using the same method as described in Example 2 and Example 3. The dispersions were placed into the stability chambers 4 days after the initial XRPD evaluation.

Nineteen days after initial placing in the stability chambers, the dispersions were added to 30 cc HDPE bottles and induction sealed. These samples were placed under both stability conditions of Stability Chamber 1 and Stability Chamber 2.

Thirty one days after initial placing in the stability chambers the dispersions were added to 30cc HDPE bottles containing 1 g of desiccant (SorbIT) and induction sealed. These samples were placed under both stability conditions of Stability Chamber 1 and Stability Chamber 2 for later analysis.

The XRPD for the above dispersions at the time of initial evaluation was compared to previous analysis of neat Compound I and did not show any crystalline peaks indicating that all the dispersions were amorphous at time zero (Figure 4).

### Example 5: Larger scale spray dried dispersions of amorphous Compound I with several polymers at a 25:75 ratio

### Materials:

Compound I, Cellulose acetate phthalate (CAP), from Eastman; Polyvinylpyrrolidone polyvinyl acetate (Plasdone S630), from Ashland; Hydroxypropylmethylcellulose acetate succinate (HPMCAS-M), AquaSolve, from Ashland.

### Procedure:

For each dispersion, 22 g of Compound I were weighed into a 1 L Pyrex bottle. Then 66 g of the polymer were added, followed by 880 mL tetrahydrofuran (THF) and the mixtures stirred until all solids were fully dissolved.

Each of the solutions was spray dried with the following conditions: inlet temperature 100°C; outlet temp ~ 40-45°C based on the inlet temp; aspirator 100 %; pump 35%; used standard cyclone and collection vessel.

The spray dried dispersion of amorphous Compound I produced for each batch was collected and evaluated by XRPD.
**Dispersion 14:** 25:75 Compound I:CAP
**Dispersion 15:** 25:75 Compound I:Plasdone S630
**Dispersion 16:** 25:75 Compound I:HPMCAS-M

The spray dried dispersions of amorphous Compound I produced above were dried further in a vacuum oven at 10-15 in Hg at 40°C over four nights until the LODs (Loss on drying levels) were sufficiently low (see values below).

Dispersions were analyzed by XRPD, mDSC, and microscopy. The same conditions as those described in Example 3 were used for XRPD and microscopy.

For mDSC the slightly modified conditions below were used:
Modulated DSC; Pan: Hermetic sealed pan with a pinhole added; Equilibrate at 0.00 °C;
Modulate ± 1.00 °C every 60 s; Isothermal for 5.00 min; Ramp 1.50 °C/min to 240.00 °C.

Samples of the dispersions were placed in 30 cc HDPE bottles with and without 1 g desiccants and placed in Stability Chamber 1 and Stability Chamber 2.

Results for the dispersions analyses at time 0:
Dispersion 14: LOD of 1.66%, Tg of 129°C, amorphous by XRPD, spherical particles without any signs of crystallization.
Dispersion 15: LOD of 1.40%, Tg of 101°C, amorphous by XRPD, spherical particles without any signs of crystallization.
Dispersion 16: LOD of 1.05%, Tg of 91°C, amorphous by XRPD, spherical particles without any signs of crystallization.

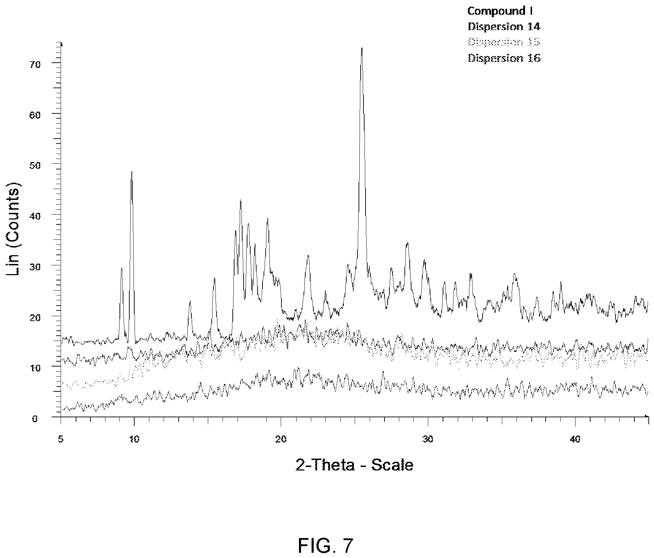

XRPD results for Dispersions 14, 15 and 16 after production are shown in Figure 7.

### Example 6: Stability of spray dried dispersions of amorphous Compound I under different conditions and using different polymers

A number of dispersions previously produced as indicated in Examples 2, 4 and 5 that had been kept in Stability Chamber 2 (40 °C/75 %) for different lengths of time were analyzed by XRPD, mDSC and Microscopy, the results are summarized below in Table 1.

**Table 1. Stability of dispersions generated in Examples 2, 4 and 5.**

| **dispersion** | **Polymer** | **Compound I:Polymer** | **Storage Condition** | **DSC Initial Tg** | **Crystallization Detection Timepoint (mo)** | **Method Detected By** |
|---|---|---|---|---|---|---|
| Dispersion 2 | CAP | 50 : 50 | 40°C/75°/ RH Open | NA | 6 | Microscopy |
| Dispersion 4 | HPMCAS | 50: 50 | 40°C/75°/ RH Open | NA | 3 | Microscopy |
| Dispersion 5 | CAP | 50 : 50 | 40°C/75%RH Open | 78 | 2 | Microscopy |
| Dispersion 6 | CAP | 25 : 75 | 40°C/75%RH Open | 99 | 6 | DSC |
| Dispersion 9 | HPMCAS | 50: 50 | 40°C/75%RH Open | 73 | 6 | DSC |
| Dispersion 10 | HPMCAS | 25 : 75 | 40°C/75%RH Open | 93 | Not detected after 6 months | NA |
| Dispersion 11 | HPMCP | 25 : 75 | 40°C/75%RH Open | 85 | Not detected after 6 months | NA |
| Dispersion 12 | PVP K29/32 | 25 : 75 | 40°C/75°/ RH Open | NA | Not detected after 6 months | NA |
| Dispersion 13 | Plasdone S630 | 25 : 75 | 40°C/75%RH Open | 90 | Not detected after 6 months | NA |
| Dispersion 14 | CAP | 25 : 75 | 40°C/75%RH Open | 129 | 6 | Microscopy |
| Dispersion 16 | HPMCAS | 25 : 75 | 40°C/75%RH Open | 91 | Not detected after 12 months | NA |

### Example 7: Spray dried dispersions of Compound I with HPMCAS-H polymer

In previous examples, dispersions of Compound I with HPMCAS-M (medium pH) were produced. Here, dispersions with HPMCAS-H (high pH, a different grade of the same polymer) were produced.

Weighed 22 g of Compound I into a 1 L Pyrex bottle then added 66 g of the polymer. Added 880 mL of tetrahydrafuran (THF) and stirred until all solids were fully dissolved. This took a few hours.

The solution was spray dried with the following conditions: Inlet temp: 100°C; Outlet temp: ~ 40-45°C based on the inlet temp; Aspirator: 100%; Pump: 35%; used standard cyclone and collection vessel.

The above protocol generated a new dispersion:
**Dispersion 17:** Spray dried dispersion (25:75) Compound I:HPMCAS-H

This dispersion was dried further in a vacuum oven at 10-15 in Hg at 40°C over four nights until the LOD was sufficiently low. The dispersion was analyzed by XRPD, mDSC, and microscopy at time zero. The following conditions were used: XRPD Method: scan 5-45° 2-theta, 0.02° step size, 1 sec per step on low background holder; DSC Method: Modulated DSC (mDSC); Pan: Hermetic sealed pan with a pinhole; Equilibrate at 0.00 °C; Modulate ± 1.00 °C every 60 s; Isothermal for 5.00 min; Ramp 1.50 °C/min to 200.00 °C; Microscopy Method: Images taken with 20X objective lens under polarized light.

Characterization at time zero: LOD of 1.05%, Tg of 91°C, amorphous by XRPD, spherical particles without any signs of crystallization.

### Example 8A: Spray dried dispersions of Compound I with HPMCAS-M polymer and a range of ratios for stability evaluation

Previously, Compound I spray dried dispersions were produced at 25:75 and 50:50 Compound I:HPMCAS-M (Dispersions 10, and 4 and 9) levels. Dispersions at 25:75 level were found to have greater stability. In this study, dispersions using HPMCAS-M were prepared at different ratios and placed under stability conditions in Stability Chamber 1 and Stability Chamber 2. In addition, additional dispersions were produced at the 25:75 level for use in subsequent tableting experiments.

### Materials:

Compound I; Hydroxypropylmethylcellulose acetate succinate (HPMCAS-M, MG Grade), from Shin Etsu.

### Procedure: Production of spray dried dispersions

For each of the dispersions weighed Compound I and HPMCAS-M into 250 mL Pyrex bottles or a 2 L Pyrex bottle and added the methanol amount shown in Table 2. Stirred the solutions until Compound I and HPMCAS-M were fully dissolved.

**Table 2. Preparation of dispersions 18 to 24.**

| Dispersion # | Compound I: HPMCAS-M | Compound I (g) | HPMCAS-M (g) | Methanol (mL) |
|---|---|---|---|---|
| **Dispersion 18** | 10:90 | 0.99 | 9.01 | 100 |
| **Dispersion 19** | 15:85 | 1.51 | 8.49 | 100 |
| **Dispersion 20** | 20:80 | 2.02 | 7.98 | 100 |
| **Dispersion 21** | 30:70 | 3.02 | 7.03 | 100 |
| **Dispersion 22** | 40:60 | 4.03 | 6.03 | 100 |
| **Dispersion 23** | 25:75 | 26.66 | 79.90 | 1066 |
| **Dispersion 24** | 25:75 | 37.51 | 112.52 | 1500 |

Each of the solutions was spray dried with the following conditions: Inlet temp: 100°C; Outlet temp: ~ 35-45°C based on the inlet temp; Aspirator: 100%; Pump: 35%; the high efficiency cyclone and collection vessel were used for Dispersions 18 to 22; the standard cyclone and collection vessel were used for Dispersions 23 and 24.

Each of the dispersions was dried in the vacuum oven over 4 nights. The LOD and Tg value for the batches were measured after production, giving the results indicated in Table 3:

**Table 3. DSC results for Dispersions 18 to 24.**

| Dispersion # | LOD (%) | Tg (°C) |
|---|---|---|
| **Dispersion 18** | 2.13 | 106 |
| **Dispersion 19** | 1.44 | 101 |
| **Dispersion 20** | 0.56 | 96 |
| **Dispersion 21** | 1.95 | 88 |
| **Dispersion 22** | 1.79 | 80 |
| **Dispersion 23** | 1.49 | 91 |
| **Dispersion 24** | 1.97 | 88 |

The spray dried product for each batch was collected and evaluated by XRPD, DSC, and Microscopy using the following methods.

XRPD Method: scan 5-45° 2-theta, 0.02° step size, 1 sec per step on low background holder.

DSC Method: Modulated DSC; Pan: Hermetic sealed pan with a pinhole added; Equilibrate at 0.00 °C; Modulate ± 1.00 °C every 60 s; Isothermal for 5.00 min; Ramp 1.50 °C/min to 200.00 °C.

Microscopy Method: Images taken with 20X objective lens under polarized light.

Once generated, samples of the dispersions were placed in HDPE bottles (unsealed) in the 40°C/75%RH stability chamber (Stability Chamber 2) for stability evaluation.

| **dispersion** | **Polymer** | **Compound I: Polymer** | **Storage Condition** | **DSC Initial Tg** | **Crystallization Detection Timepoint (mo)** | **Method Detected By** |
|---|---|---|---|---|---|---|
| Dispersion 18 | HPMCAS-M | 10 : 90 | 40°C/75%RH Open | 106 | Not detected after 12 months | NA |
| Dispersion 19 | HPMCAS-M | 15 : 85 | 40°C/75°/ RH Open | 101 | Not detected after 12months | NA |
| Dispersion 20 | HPMCAS-M | 20 : 80 | 40°C/75%RH Open | 96 | Not detected after 12 months | NA |
| Dispersion 21 | HPMCAS-M | 30 : 70 | 40°C/75°/ RH Open | 88 | Not detected after 12 months | NA |
| Dispersion 22 | HPMCAS-M | 40 : 60 | 40°C/75%RH Open | 80 | Not detected after 12 months | NA |
| Dispersion 23 | HPMCAS-M | 25 : 75 | 40°C/75%RH Open | 91 | Not detected after 12 months | NA |
| Dispersion 24 | HPMCAS-M | 25 : 75 | 40°C/75%RH Open | 88 | Not detected after 12 months | NA |
| Dispersion 25 | HPMCAS-M | 25 : 75 | 40°C/75%RH Open | 90 | Not detected after 12 months | NA |

A summary of the LOD and Tg values is shown in Table 3 above. XRPD indicated that all dispersions were amorphous and microscopy showed the presence of spherical particles and no indication of the crystalline form (needles/rods) at time zero.

None of the above prepared dispersions showed signs of crystallization after 12 months of storage at 40 °C/75 % RH in unsealed bottles.

### Example 8B. Preparation of Dispersion 25.

### General Procedure for preparation of 25: 75 Compound I:HPMCAS-M dispersions in large scale:

Transferred known weight of methanol into a container. Stirred the methanol. While stirring, added Compound I and allowed to dissolve. Continued stirring, slowly added HPMCAS-M and allowed to dissolve. The total solids concentration used was 10%, with 90% of the weight of the solution from the methanol. Spray dried the solution using an appropriately sized spray dryer and appropriate nozzle using an appropriate inlet temperature and solution flow rate. Once spray drying was complete, transferred the material to a tray dryer or fluid bed dryer dry until the loss on drying (LOD) was below 2 %.

### Specific procedure for Dispersion 25

Transferred 1500 mL of methanol into a 2 L bottle. Stirred the methanol. While stirring, added 37.5 g of Compound I and allowed to dissolve. Continued stirring, slowly added 112.5 g of HPMCAS-M and allowed to dissolve. Spray dried using a Buchi B290 spray dryer with a two fluid nozzle using the following conditions:
Inlet temp: 100°C; Aspirator: 100%; Pump: 35%

The powder collected in the collection vessel below the cyclone of the spray dryer was transferred into an oven and tray dried over 4 nights until a loss on drying (LOD) below 2% was achieved.

### Example 9. Tablet preparation from dispersions prepared in Example 2, using dry granulation by slugging at 20 mg dosage.

### Materials:

Dispersion 2 prepared in Example 2; Dispersion 4 prepared in Example 2; Avicel DG (co-processed MCC with anhydrous dicalcium phosphate), from FMC; Croscarmellose Sodium (CCS), Ac-Di-Sol, from FMC; Mg Stearate, from Fisher.

### Procedure:

Weighed the intragranular blend components, except Mg Stearate (see Table 4 and Table 5) into a HDPE bottle then blended on the Turbula Shaker for 5 minutes. Added Mg Stearate and blended for an additional 3 minutes for each dispersion.

Weighed out approximately 1150 mg aliquots of the above blends and tableted them using 0.6250" round, flat tooling at a pressure of 1500 psi (= 10.342 MPa) to target a hardness of 12 kP.

Milled the slugs on the comill using a screen with a 0.040" hole size at 2000 rpm.

Based on the yields, added the extragranular components, except Mg Stearate, and blended for 5 minutes on the Turbula Shaker. Added Mg Stearate and blended for an additional 3 minutes for each dispersion.

Weighed 200 mg aliquots of each blend (corresponds to a 20 mg dose of Compound I) and compressed into tablets using 0.3125" round tooling at a pressure of 1300 psi (= 8.936 MPa) to target a hardness of 9-10 kP.

**Tablets T2/200/20** were obtained from Dispersion 2.

**Tablets T4/200/20** were obtained from Dispersion 4.

**Table 4. Preparation of Tablets T2/200/20**

| | **%** | |
|---|---|---|
| | **Intragranular** | **Extragranular** |
| **Dispersion 2** | 20 | |
| MCC DG | 56.5 | 20 |
| CCS | 3 | |
| Mg Stearate | 0.25 | 0.25 |

**Table 5. Preparation of Tablets T4/200/20.**

| | **%** | |
|---|---|---|
| | **Intragranular** | **Extragranular** |
| **Dispersion 4** | 20 | |
| MCC DG | 56.5 | 20 |
| CCS | 3 | |
| Mg Stearate | 0.25 | 0.25 |

### Example 10. Tablet preparation from dispersions prepared in Example 2, using dry granulation by slugging at 40 mg and 80 mg dosages

### Materials:

The blends previously used in Example 9 to prepare tablets from Dispersion 2 were used again to prepare 400 mg tablets with a dose of 40 mg and 800 mg tablets with a dose of 80 mg.

### Procedure:

Tooling: For 40 mg dose (400 mg tablet weight), 0.2677"x0.5787" modified oval tooling was used; for 80 mg dose (800 mg tablet weight), 0.4000"x0.7500" modified oval tooling was used.

Weighed blends and evaluated compression settings. These were selected to achieve the following target hardness levels: 40 mg tablets - 12-13 kP; 80 mg tablets - 15-16 kP

**Tablets T2/400/40** were obtained from Dispersion 2 and a 40 mg final dose of Compound I per tablet; tablets were compressed at 1250 psi (= 8.618 MPa).

**Tablets T2/800/80** were obtained from Dispersion 2 and an 80 mg final dose of Compound I per tablet; tablets were compressed at 2000 psi (= 13.79 MPa).

**Example 11. Tablet preparation from dispersions prepared in Example 5, using dry granulation by roller compaction at 10 mg dosages**

### Materials

Dispersion 14 from Example 5, Dispersion 15 from Example 5, Dispersion 16 from Example 5; Avicel DG (MCC co-processed with dicalcium phosphate), from FMC; Croscarmellose Sodium (CCS), Ac-Di-Sol, from FMC; Mg Stearate, from Fisher; MCC (Avicel PH-102), from FMC; Opadry II, 8518422 White, from Colorcon - WP731466; Milli Q Laboratory Water; Desiccant, Sorb-IT.

### Production of dry granulations (by roller compaction):

Dispersions 14, 15 and 16, obtained in Example 5 were weighed and blended in a 1 qt V-shell on the V-blender for 10 minutes with Avicel DG and CCS that had been screened through a 20-mesh screen. Mg Stearate was screened through 20-mesh and added for an additional 3 minutes of blending.

The blends were then roller compacted with a roll pressure of 10 MPa and roll speed of 1 rpm. The screw speed was varied from 40 rpm to 70 rpm to achieve desirable ribbons.
Dispersion 14: screw speed ~ 50 rpm, ribbon thickness 1.8 - 2.0 mm
Dispersion 15: screw speed ~ 70 rpm, ribbon thickness 1.2 - 1.5 mm
Dispersion 16: screw speed ~ 50 rpm, ribbon thickness 1.2 - 1.5 mm

The ribbons were milled using the CoMil at a speed of 2000 rpm using a screen with a 0.062" hole size to produce the final granulation.

### Production of tablets:

The extragranular components were screened through a 20-mesh screen. Based on the yield, the extragranular components, except Mg Stearate, were added and blended for 10 minutes in a 1 qt V-shell on the V-blender. Added Mg Stearate and blended for an additional 3 minutes.

Tableting was performed using the force feeder with the automated tablet press. Tableting was performed using 0.3150" round tooling and a target a hardness of 9-10 kP.

The main compression and ejection forces were monitored and were 7-8 kN and 70-80 N, respectively, for Dispersions 14 and 16 and 7-8 kN and 80-100 N, respectively, for Dispersion 15. *Coating of tablets:*

The target weight gain during coating was 3.0%. Coating solution used for Dispersion 14 and 15: prepared Opadry II coating solution at 20 % solids by weighing 40.03 g and added to 200 mL of purified water while stirring with an overhead stirrer. The solution was stirred for > 45 minutes prior to initiating the coating process.

Coating solution used for Dispersion 16: prepared Opadry II coating solution at 20 % solids by weighing 40.06 g and added to 200 mL of purified water while stirring with an overhead stirrer. The solution was stirred for > 45 minutes prior to initiating the coating process. The ingredients and amounts utilized for each batch are described in Tables 6, 7 and 8 below.

**Table 6. Preparation of Tablets T14/200/10/coated. From Dispersion 14.**

| | | **Intragranular** | **Extragranular** |
|---|---|---|---|
| **Dispersion 14** (25:75) CAP | | 20 | |
| MCC/dicalcium phosphate (Avicel DG) | | 56.5 | |
| MCC (Avicel PH102) | | | 20 |
| CCS | | 3 | |
| Mg Stearate | | 0.25 | 0.25 |

**Table 7. Preparation of Tablets T15/200/10/coated. From dispersion 15.**

| | | **%** | |
|---|---|---|---|
| | | **Intragranular** | **Extragranular** |
| **Dispersion 15** (25:75) Plasdone S630 | | 20 | |
| MCC/dicalcium phosphate (Avicel DG) | | 56.5 | |
| MCC (Avicel PH102) | | | 20 |
| CCS | | 3 | |
| Mg Stearate | | 0.25 | 0.25 |

**Table 8. Preparation of Tablets T16/200/10/coated. From dispersion 16.**

| | | **%** | |
|---|---|---|---|
| | | **Intragranular** | **Extragranular** |
| **Dispersion 16** (25:75) HPMCAS | | 20 | |
| MCC/dicalcium phosphate (Avicel DG) | | 56.5 | |
| MCC (Avicel PH102) | | | 20 |
| CCS | | 3 | |
| Mg Stearate | | 0.25 | 0.25 |

### Stability of tablets:

Tablets of each lot were aliquoted into 60 cc HDPE bottles for placing into stability chambers 1 and 2.

Coated tablets obtained from Dispersion 14 (Tablets T14/200/10/coated) were used for initial testing, placed in Stability Chamber 1 at 25°C/60%RH and placed in Stability Chamber 2 at 40°C/75%RH.

Coated tablets obtained from Dispersion 15 (Tablets T15/200/10/coated) were used for initial testing, placed in Stability Chamber 1 at 25°C/60%RH and placed in Stability Chamber 2 at 40°C/75%RH.

Coated tablets obtained from Dispersion 16 (Tablets T16/200/10/coated) were used for initial testing, placed in Stability Chamber 1 at 25°C/60%RH and placed in Stability Chamber 2 at 40°C/75%RH.

1 g of desiccant was added to all bottles which were then induction sealed. The bottles for initial testing were placed in a foil pouch with 6 1-g desiccants, sealed and placed at 5°C.

### Example 12. Tablet preparation from dispersions prepared in Example 5 and 7, using dry granulation by roller compaction at 20 mg dosages

### Materials:

Dispersion 14 (25:75 Compound I:CAP), obtained in Example 5; Dispersion 16 (25:75 Compound I:HPMCAS-M, obtained in Example 5; Dispersion 17 (25:75 Compound I: HPMCAS-H), obtained in Example 7, MCC/dicalcium phosphate (Avicel DG), from FMC; Croscarmellose Sodium (CCS), Ac-Di-Sol, from FMC; Mg Stearate, from Fisher; MCC (Avicel PH-102), from FMC; Desiccant, Sorb-IT.

Blends that had been previously used in Example 11, also using Dispersions 14 and 16, were made for a 10 mg dose in a 200 mg tablet, therefore a 400 mg tablet was produced to achieve a 20 mg dose.

25:75 Compound I:HPMCAS-M Tablets, 20 mg dose were produced from Dispersion 16 (Tablets T16/400/20). The following protocol was used:

Weighed aliquots of approximately 400 mg of final blend for Dispersion 16, and transferred to the die for 0.2677"x0.5787" modified oval tooling setup on the manual press. Target hardness was 12 kP. See Table 10B.

**Table 10B. Preparation of Tablets T16/400/20.**

| | | **Intragranular** | **Extragranular** |
|---|---|---|---|
| **Tablets T16/400/20 (25:75) HPMCAS-M Dispersion** | | 20 | |
| MCC/dicalcium phosphate (Avicel DG) | | 56.5 | |
| MCC (Avicel PH102) | | | 20 |
| CCS | | 3 | |
| Mg Stearate | | 0.25 | 0.25 |

25:75 Compound I:CAP Tablets, 20 mg dose were produced from Dispersion 14 (Tablets T14/400/20). The following protocol was used:
Weighed aliquots of approximately 400 mg of final blend for Dispersion 14, and transferred to the die for 0.2677"x0.5787" modified oval tooling setup on the manual press. Target hardness was 12 kP. See Table 10C.

**Table 10C. Preparation of Tablets T16/400/20.**

| | | **Intragranular** | **Extragranular** |
|---|---|---|---|
| **Tablets T14/400/20 (25:75) CAP Dispersion** | | 20 | |
| MCC/dicalcium phosphate (Avicel DG) | | 56.5 | |
| MCC (Avicel PH102) | | | 20 |
| CCS | | 3 | |
| Mg Stearate | | 0.25 | 0.25 |

### Production of dry granulation from Dispersion 17

The dispersions were weighed and blended in a 1 qt V-shell for 10 minutes with Avicel DG and CCS that had been screened through a 20-mesh screen. Mg Stearate was screened through 20-mesh and added for an additional 3 minutes of blending.

The blends were then roller compacted with a roll pressure of 10 MPa and roll speed of 1 rpm. The screw speed was varied from 40 rpm to 70 rpm to achieve desirable ribbons.

For the production of dry granulation from Dispersion 17: screw speed ~ 70 rpm, ribbon thickness 1.1 - 1.2 mm. The ribbons were milled using the CoMil at a speed of 2000 rpm using a screen with 0.062" hole size to produce the final granulation.

### Production of 10 mg tablets from Dispersion 17 (Tablets 17/200/10)

An extragranular blend for 10 mg tablets was prepared. Screened the extragranular components through a 20-mesh screen. Based on the yield, added the extragranular components, except Mg Stearate, and blended for 10 minutes in a 1 qt V-shell on the V-blender. Added Mg Stearate and blended for an additional 3 minutes.

Tableting was performed using the force feeder with the automated tablet press. Tableting was performed using 0.3150" round tooling and a target a hardness of 9-10 kP. The main compression and ejection forces were monitored with the Director software.

### Production of tablets at a dose of 20 mg of Compound with HPMCAS-H (from dispersion 17)

25:75 Compound I:HPMCAS-H Tablets were produced at a 20 mg dose (Tablets T17/400/20).

Aliquots of approximately 400 mg of final blend that had been used for the production of the 10 mg dose tablets above were weighed and transferred to the die for 0.2677"x0.5787" modified oval tooling setup on the manual press. Target hardness was 12 kP. (Tablets 17/400/20). Details of the formulation are collected in Table 11.

**Table 11. Preparation of Tablets T17/400/20.**

| | | **Intragranular** | **Extragranular** |
|---|---|---|---|
| **Tablets T17/400/20 (25:75) HPMCAS-H Dispersion** | | 20 | |
| MCC/dicalcium phosphate (Avicel DG) | | 56.5 | |
| MCC (Avicel PH102) | | | 20 |
| CCS | | 3 | |
| Mg Stearate | | 0.25 | 0.25 |

### Example 13. Kinetic solubility of solid dispersions in simulated intestinal fluids

The solubilities of some of the spray dried dispersions previously produced in Example 4 were evaluated to determine if there is a polymer that inhibits crystallization of Compound I from solution for a longer duration. Solutions with a Compound I:polymer 25:75 were used in this study. Solubility was determined in simulated intestinal fluids. Two fluids were utilized: FeSSIF (Fed State Stimulated Intestinal Fluid) and FaSSIF (Fasted State Intestinal Fluid).

Based on the dissolution data in the simulated fluids, cellulose acetate phthalate (CAP), HPMCAS, and Plasdone S630 appeared to have the most potential for stabilization of the amorphous form of Compound I.

### Materials:

Compound I; Hydroxypropylmethyl Cellulose, Methocel E5, from Dow; Polyvinylpyrrolidone (PVP), Plasdone K29/32, from Ashland; Copolymer of polyvinylpyrrolidone and polyvinylacetate (Plasdone), Plasdone S630, from Ashland; 25:75 Compound I:CAP spray dried dispersion (Dispersion 6), 25:75 Compound I:HPMCAS spray dried dispersion (Dispersion 10); 25:75 Compound I:HPMCP spray dried dispersion (Dispersion 11); 25:75 Compound I:PVP spray dried dispersion (Dispersion 12); 25:75 Compound I:Plasdone S630 spray dried dispersion (Dispersion 13); Sodium Taurocholate, from Spectrum; Lecithin, from Spectrum; Pepsin, from Fisher Scientific; Sodium chloride, from Sigma-Aldrich; Sodium hydroxide, from Fisher Scientific; Maleic acid, from Sigma-Aldrich; FaSSIF-V2 powder, from Biorelevant; FeSSIF-V2 powder, from Biorelevant.

### Procedure:

1 L of each of the simulated fluids was prepared. For FaSSIF and FeSSIF, the powders and procedures from Biorelevant were used. The instructions for the preparation of FaSSIF can be found on line at http://biorelevant.com/fassf-v2/how-to-make/. The instructions for the preparation of FeSSIF can be found on line at http://biorelevant.com/fessif-v2-dissolution-medium/fed-state-stimulated-intestinal-fluid-version-two-how-to-make/.

Maleate buffers were prepared at pH levels of 6.5 and 5.8 for FaSSIF and FeSSIF, respectively, using maleic acid, sodium hydroxide, and sodium chloride. FaSSIF-V2 and FeSSIF-V2 powders were then added to produce the FaSSIF and FeSSIF dissolution media. All prepared media were stored refrigerated until the day of use on which it was removed and placed at lab conditions for a minimum of 30 minutes to equilibrate to room temperature prior to adding to the dispersions.

For each 25:75 Compound I:polymer dispersion generated in Example 4, approximately 100 mg of the dispersion was weighed into each of two 100 mL glass bottles labeled FaSSIF or FeSSIF. 50 mL of the FaSSIF or FeSSIF media was then added to the bottles

As soon as these samples were prepared, a 1.5 mL aliquot was transferred into centrifuge tubes. All bottles were then placed on the shaker which was set at 37°C and 115 RPM. The tubes were then centrifuged for 15 minutes at 13000 RPM. 0.5 mL aliquots of the supernatant were transferred into HPLC vials. 0.5 mL of acetonitrile was added to each of the vials and the vials were vortexed. This process was repeated for samples at 15, 30, 45, 60, 90, and 120 minutes and 4, 6, and 24 hrs.

### Standards preparation

One set of standards was prepared as follows and re-injected with each sample set: weighed approximately 10 mg of Compound I into a 20 mL scintillation vial. Added 2.5 mL acetonitrile and vortexed and sonicated to dissolve. Added 2.5 mL of water to this solution and vortexed/sonicated. Transferred 0.5 mL of this solution into a HPLC vial labeled (STDH). Added 0.5 mL of 1:1 acetonitrile:water to this vial. Performed a 1:10 dilution to obtain the mid standard (STDM). Then performed a 1:10 dilution of STDM to obtain the low standard (STDL).

Estimates of the concentrations were obtained by determining the relative peak area to that of the nearest standard (either STDL or STDM) or by analyzing against the standard curve.

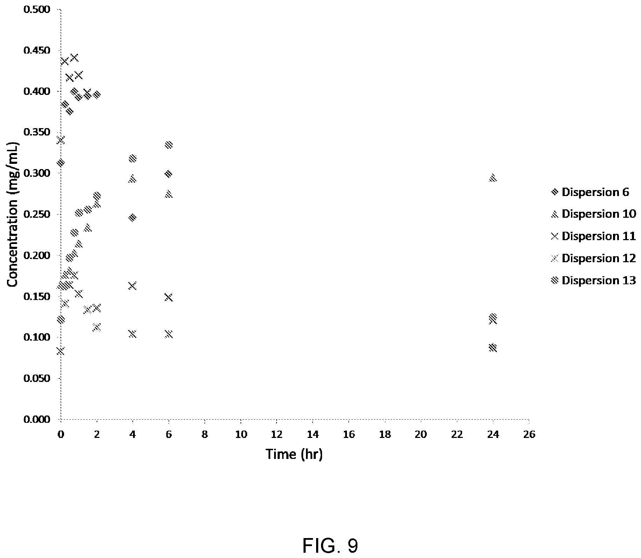

Plots of concentration of Compound I versus time over a period of 24 hours for each of the dispersions in each of the simulated media are shown in Figure 8 [kinetic solubility of dispersions in FaSSIF (Example 13)] and Figure 9 [kinetic solubility of dispersions in FeSSIF (Example 13)].

### UPLC method:

Mobile Phase A: 0.1% TFA in Water
Mobile Phase B: 0.1% TFA in Acetonitrile
Column: Acquity BEH Shield RP18, 1.7 um, 2.1 × 50 mm, Part No. 186002853, SN. 021439007153 14
Injection Volume: 3 uL
Autosampler Temperature: Ambient
Column Temperature: 25°C
Run Time: 10 minutes
Flow Rate: 0.45 mL/min
Collected Wavelengths: 220 nm, 245 nm, 254 nm, 280 nm, PDA Spectrum (190-400 nm)
Wavelength Used for Analysis: 254 nm
Gradient:

| **Time (min)** | **% MPA** | **% MPB** |
|---|---|---|
| 0.00 | 90.0 | 10.0 |
| 1.00 | 90.0 | 10.0 |
| 7.50 | 20.0 | 80.0 |
| 8.50 | 20.0 | 80.0 |
| 8.60 | 90.0 | 10.0 |
| 10.00 | 90.0 | 10.0 |

### Example 14. Dissolution of Tablets generated in Example 11 in FaSSIF

Evaluated the dissolution of tablets produced with granulations of a solid dispersion previously described in Example 11. Based on the dissolution of these tablets, dispersions produced with CAP or HPMCAS appear to show a faster dissolution profile than the dispersion produced with Plasdone S630.

### Materials:

**Tablets 14/200/10/coated, Tablets 15/200/10/coated, Tablets 16/200/10/coated;** Sodium chloride, from Sigma-Aldrich; Sodium hydroxide, from Fisher Scientific; Maleic acid, from Sigma-Aldrich; FaSSIF-V2 powder, from Biorelevant.

### Procedure, dissolution:

Prepared 6 L of FaSSIF (see previous example).

Using USP Type II paddles with a rotation speed of 50 RPM at 37°C with 500 mL of FaSSIF, the dissolution was evaluated for each dosage form.

Samples were collected at 0, 15, 30, 45, and 60 minutes.

After the 60 minute sample collection, the dissolution apparatus was set to increase to a paddle speed of 200 RPM for 20 minutes and samples were obtained into Eppendorf tubes. The tubes were centrifuged for 15 minutes at 13,000 RPM.

0.5 mL of each sample was added to 0.5 mL of acetonitrile and vortexed for analysis by UPLC

### Standard preparation:

Weighed 10.00 mg Compound I into a 20 mL vial and added 5 mL acetonitrile. Vortexed to dissolve. Added 5 mL water to achieve a 1 mg/mL concentration. Performed a 1:20 dilution at a 2 mL volume to achieve a concentration of 0.05 mg/mL. 1 mL of this solution was used as standard 1. Added 0.4 mL of standard 1 to 0.6 mL of 1: 1 acetonitrile:water to achieve standard 2 at a concentration of 0.02 mg/mL. Added 0.2 mL of standard 1 to 0.8 mL of 1:1 acetonitrile:water to achieve standard 3 at a concentration of 0.01 mg/mL. Added 0.1 mL of standard 1 to 0.9 mL of 1: 1 acetonitrile:water to achieve standard 4 at a concentration of 0.005 mg/mL. Added 0.05 mL of standard 1 to 0.95 mL of 1: 1 acetonitrile:water to achieve standard 5 at a concentration of 0.0025 mg/mL. Added 0.1 mL of standard 3 to 0.9 mL of 1: 1 acetonitrile:water to achieve standard 6 at a concentration of 0.001 mg/mL.

The standards and samples were analyzed by UPLC using the method below
Mobile Phase A: 0.1% TFA in Water
Mobile Phase B: 0.1% TFA in Acetonitrile
Column: Acquity BEH Shield RP18, 1.7 um, 2.1 × 50 mm, Part No. 186002853, SN. 021439007153 14
Injection Volume: 3 uL
Autosampler Temperature: Ambient
Column Temperature: 25°C
Run Time: 10 minutes
Flow Rate: 0.45 mL/min
Collected Wavelengths: 220 nm, 245 nm, 254 nm, 280 nm, PDA Spectrum (190-400 nm)
Wavelength Used for Analysis: 254 nm
**Gradient:**

| **Time (min)** | **% MPA** | **% MPB** |
|---|---|---|
| 0.00 | 90.0 | 10.0 |
| 1.00 | 90.0 | 10.0 |
| 7.50 | 20.0 | 80.0 |
| 8.50 | 20.0 | 80.0 |
| 8.60 | 90.0 | 10.0 |
| 10.00 | 90.0 | 10.0 |

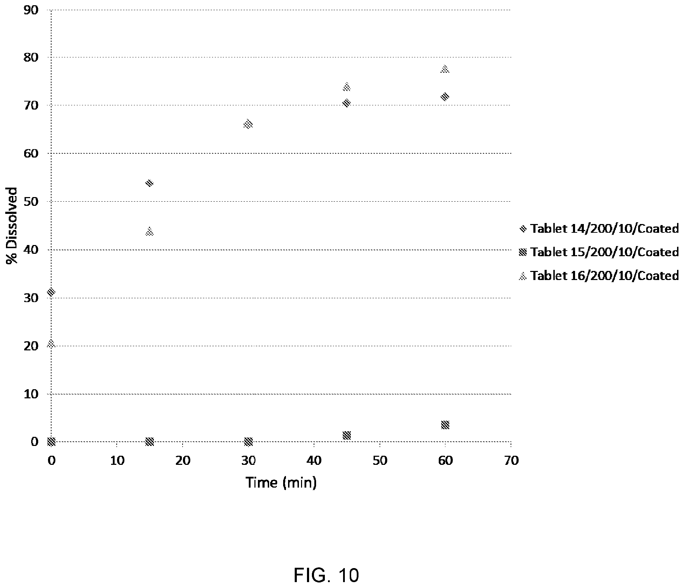

A plot of the Tablet dissolution rates is shown in Figure 10 [dissolution of tablets of amorphous spray dried dispersions of Compound I in FaSSIF (Example 14)].

### Example 15A. Tablet preparation from dispersions prepared in Example 5, using dry granulation by roller compaction at 5 mg dosages.

### Materials

Dispersion 16 from Example 5; MCC/dicalcium phosphate (Avicel DG), from FMC, Croscarmellose Sodium (CCS), Ac-Di-Sol, from FMC; Mg Stearate, from Mallinckrodt; MCC (Avicel PH-102), from FMC; Opadry II, 8518422 White, from Colorcon; Milli Q purified Laboratory Water; Desiccant, Molecular Sieve from Dessicare.

### Production of dry granulations (by roller compaction):

Dispersion 16, obtained in Example 5 was weighed and blended in a 16 qt V-blender for 10 minutes with Avicel DG and CCS that had been screened through a 20-mesh screen. Mg Stearate was screened through 20-mesh and added for an additional 3 minutes of blending. The theoretical batch size was 5 kg.

The blend was then roller compacted with a roll pressure of 1400 psi (= 9.65 MPa) and roll speed of 0.75 - 1.25 rpm. The screw speed was varied from 30 rpm to 50 rpm to achieve desirable ribbons.
Dispersion 16: screw speed ~ 30 rpm, ribbon thickness 1.4 - 1.6 mm

The ribbons were milled using the CoMil at a speed of 2000 rpm using a screen with a 0.050" hole size and round impeller to produce the final granulation.

### Production of tablets:

Screened the extragranular components through a 20-mesh screen. Based on the yield, added the extragranular components, except Mg Stearate, and blended for 10 minutes in a 16 qt V-shell on the V-blender. Added Mg Stearate and blended for an additional 3 minutes.

Tableting was performed using the force feeder with the Piccola tablet press. Tableting was performed using 0.3125" round tooling and a target a hardness of 8 kP.

The main compression was 10-11 kN. The turret speed for the tablet press was 30 rpm.

### Coating of tablets:

Coating solution used for Dispersion 16: prepared Opadry II coating solution at 20 % solids by weighing 800 g and added to 3200 mL of purified water while stirring with an overhead stirrer. The solution was stirred for > 45 minutes prior to initiating the coating process.

The ingredients and amounts utilized are described in Table 15 below. Compression details are collected in Table 16. The tablets were compressed to a target hardness of 12 kP. The main compression and ejection forces were 7-8 kN and 70-80 N, respectively. The tablets were coated to target weight gain of 3.0%. Coating process details are collected in Table 16.

**Table 15. Preparation of Tablets T16/200/5/coated. From dispersion 16.**

| | | **% weight** | |
|---|---|---|---|
| | | **Intragranular** | **Extragranular** |
| **Dispersion 16** (25:75) HPMCAS | | 10 | |
| MCC/dicalcium phosphate (Avicel DG) | | 56.5 | |
| MCC (Avicel PH102) | | | 30 |
| CCS | | 3 | |
| Mg Stearate | | 0.25 | 0.25 |

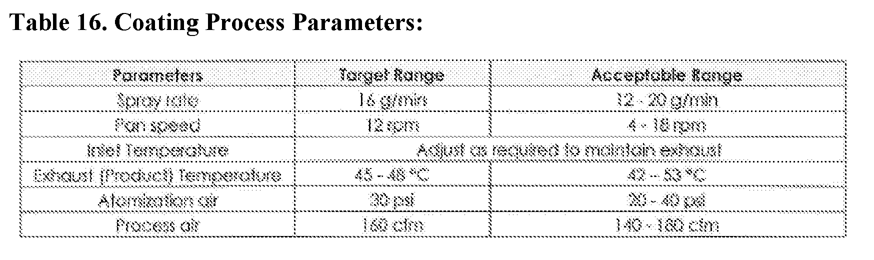

### Stability of tablets:

Tablets were aliquoted into 60 cc HDPE bottles for placing into stability chambers 1 and 2. 1 g of desiccant was added to all bottles which were then induction sealed.

These tablets, kept in sealed bottles with 1 g desiccant at 25C/60%RH and 40C/75%RH were tested for stability (presence of crystallinity) at different time points. Up to 12 months of storage, crystallinity was not observed by using the techniques described above. Moisture increased over time even in the presence of desiccant, however up to 12 months, the increase in moisture did appear to result in an increase in crystallinity.

### Example 15B. Dissolution of Tablets generated in Example 15A in FaSSIF

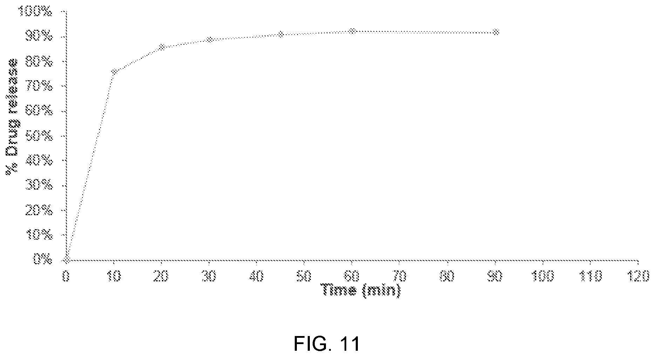

Using the same protocol as that described in Example 14 above, we evaluated the dissolution of a batch of 5 mg tablets produced as described in Example 15B. The results are summarized in FIG. 11.

### Example 16. Dog PK studies

The table below (Table 17) summarizes the tablets that were tested in dogs to determine PK parameters.

**Table 17. Dog PK study design.**

| **Group** | **No of males** | **Test article** | **Dose (mg)** | **Conc. (mg/tablet)** | **Tablets /dog** | **Tablet type** | **Route** | **Feeding status** |
|---|---|---|---|---|---|---|---|---|
| **1** | **5** | **Compound I** | **40** | **40** | **1** | **Tablets T2/400/40 (50:50 CAP)** | **PO Tablet** | **Fasted** |
| **2** | **5** | **Compound I** | **40** | **40** | **1** | **Wet granulation of native Compound I** | **PO Tablet** | **Fasted** |
| **3a** | **5** | **Compound I** | **20** | **20** | **1** | **T2/200/20 (50:50 CAP)** | **PO Tablet** | **Fed** |
| **4a** | **5** | **Compound I** | **20** | **20** | **1** | **Wet granulation of native Compound I** | **PO Tablet** | **Fed** |
| **3b** | **5** | **Compound I** | **20** | **20** | **1** | **T2/200/20 (50:50 CAP)** | **PO Tablet** | **Fasted** |
| **4b** | **5** | **Compound I** | **20** | **20** | **1** | **Wet granulation of native Compound I** | **PO Tablet** | **Fasted** |
| **5** | **5** | **Compound I** | **80** | **80** | **1** | **T2/800/80 (50:50 CAP)** | **PO Tablet** | **Fasted** |
| **6** | **5** | **Compound I** | **80** | **80** | **1** | **Wet granulation of native Compound I** | **PO Tablet** | **Fasted** |
| **7** | **6** | **Compound I** | **20** | **20** | **1** | **T14/400/20 (25:75 CAP)** | **PO Tablet** | **Fasted** |
| **8** | **6** | **Compound I** | **20** | **20** | **1** | **T14/400/20 (25:75 CAP)** | **PO Tablet** | **Fasted** |
| **9** | **6** | **Compound I** | **20** | **20** | **1** | **T16/400/20 (25:75 HPMCAS-M)** | **PO Tablet** | **Fasted** |
| **10** | **6** | **Compound I** | **20** | **20** | **1** | **T17/400/20 (25:75 HPMCAS-H)** | **PO Cap Tablet** | **Fasted** |

### Feeding Conditions:

Male Beagle dogs were used for this study. Groups 1, 2, 3b, 4b, 5, 6, 7, 8, 9 and 10: All dogs were fasted overnight. All dogs received their daily ration at 4 h postdose. Groups 3a and 4a: All dogs were fasted overnight and within 10 minutes prior to dosing, the dogs were gavaged with 50 g of a homogenate prepared from a Human FDA diet (2 eggs, 2 bacon strips, 2 pieces of toast with butter/jelly, hash brown potatoes, and 8 oz (=237 ml) of whole milk). All dogs received their daily ration at 4 h postdose.

All animals were treated with pentagastrin (6 mg/kg; 0.024 mL/kg) 30 min prior to test article administration. The same animals were dosed several times in several groups with at least a 7 day washout period in each case.

### Dose Administration:

The tablet was placed in the back of the mouth via pill gun or by hand. Following administration each animal was given 5-10 mL of water to facilitate swallowing of the tablet. Blood/Plasma:

Blood samples (2 mL) were collected from the jugular, cephalic and saphenous veins at the following times: 0.25h, 0.5 h, 1h, 2h, 3h, 4h, 6h, 8h, 24h, 32h and 48 h post-dose. Blood samples were kept on ice until processed for plasma. Blood samples were centrifuged at 3200 RPM for 10 minutes at ~ 5°C within 1 h of collection. Plasma was split into two approximately equal aliquots, and directly transferred to a 96-well plate tube (1.1 mL). Plug caps were placed on the tubes. Plasma samples were stored at approximately - 70 °C until used. Compound I concentration in plasma was assayed using an HPLC assay.

In dogs that had been fasted, when administered Tablet T2/400/40 (40 mg dose of a 50:50 CAP:Compound I dispersion) the average Cmax/dose (Cmax in ng/mL divided by the dose in mg/kg) was about 93. The Cmax/dose for dogs that had been administered an equivalent dose of native Compound I as a wet granulation tablet was about 48.

In dogs that had been fed, when administered Tablet T2/200/20 (20 mg dose of a 50:50 CAP:Compound I dispersion) the average Cmax/dose (Cmax in ng/mL divided by the dose in mg/kg) was about 245. The Cmax/dose for dogs that had been administered an equivalent dose of native Compound I as a wet granulation tablet was about 145.

In dogs that had been fasted, when administered Tablet T2/200/20 (20 mg dose of a 50:50 CAP:Compound I dispersion) the average Cmax/dose (Cmax in ng/mL divided by the dose in mg/kg) was about 153. The Cmax/dose for dogs that had been administered an equivalent dose of native Compound I as a wet granulation tablet was about 40.

In dogs that had been fasted, when administered Tablet T2/800/80 (80 mg dose of a 50:50 CAP:Compound I dispersion) the average Cmax/dose (Cmax in ng/mL divided by the dose in mg/kg) was about 82. The Cmax/dose for dogs that had been administered an equivalent dose of native Compound I as a wet granulation tablet was about 30.

In dogs that had been fasted, when administered Tablet T2/400/40 (40 mg dose of a 50:50 CAP:Compound I dispersion) the average AUC/dose (AUC in (h x ng)/mL divided by the dose in mg/kg) was about 1710. The AUC/dose for dogs that had been administered an equivalent dose of native Compound I as a wet granulation tablet was about 950.

In dogs that had been fed, when administered Tablet T2/200/20 (20 mg dose of a 50:50 CAP:Compound I dispersion) the average AUC/dose (AUC in (h x ng)/mL divided by the dose in mg/kg) was about 3392. The AUC/dose for dogs that had been administered an equivalent dose of native Compound I as a wet granulation tablet was about 2324.

In dogs that had been fasted, when administered Tablet T2/200/20 (20 mg dose of a 50:50 CAP:Compound I dispersion) the average AUC/dose (AUC in (h x ng)/mL divided by the dose in mg/kg) was about 1869. The AUC/dose for dogs that had been administered an equivalent dose of native Compound I as a wet granulation tablet was about 691.

In dogs that had been fasted, when administered Tablet T2/800/80 (80 mg dose of a 50:50 CAP:Compound I dispersion) the average AUC/dose (AUC in (h x ng)/mL divided by the dose in mg/kg) was about 1273. The AUC/dose for dogs that had been administered an equivalent dose of native Compound I as a wet granulation tablet was about 486.

In dogs that had been fasted, when administered Tablet T2/400/40 (40 mg dose of a 50:50 CAP:Compound I dispersion) the average estimated Bioavailability (% F) was about 59. The estimated bioavailability for dogs that had been administered an equivalent dose of native Compound I as a wet granulation tablet was about 31.

In dogs that had been fed, when administered Tablet T2/200/20 (20 mg dose of a 50:50 CAP:Compound I dispersion) the average bioavailability (% F) was about 118. The bioavailability for dogs that have been administered an equivalent dose of native Compound I as a wet granulation tablet was about 73.

In dogs that had been fasted, when administered Tablet T2/200/20 (20 mg dose of a 50:50 CAP:Compound I dispersion) the average estimated bioavailability (% F) was about 60. The bioavailability for dogs that have been administered an equivalent dose of native Compound I as a wet granulation tablet was about 22.

In dogs that had been fasted, when administered Tablet T2/800/80 (80 mg dose of a 50:50 CAP:Compound I dispersion) the estimated bioavailability (% F) was about 44. The estimated bioavailability for dogs that have been administered an equivalent dose of native Compound I as a wet granulation tablet was about 16.

In dogs that had been fasted, when administered Tablet T14/400/20 (20 mg dose of a 75:25 CAP:Compound I dispersion) the average Cmax/dose (Cmax in ng/mL divided by the dose in mg/kg) was about 204.

In dogs that had been fasted, when administered Tablet T16/400/20 (20 mg dose of a 75:25 HPMCAS-M:Compound I dispersion) the average Cmax/dose (Cmax in ng/mL divided by the dose in mg/kg) was about 244.

In dogs that had been fasted, when administered Tablet T17/400/20 (20 mg dose of a 75:25 HPMCAS-H:Compound I dispersion) the average Cmax/dose (Cmax in ng/mL divided by the dose in mg/kg) was about 92.

In dogs that had been fasted, when administered Tablet T14/400/20 (20 mg dose of a 75:25 CAP:Compound I dispersion) the average AUC/dose (AUC in (h x ng)/mL divided by the dose in mg/kg) was about 2762.

In dogs that had been fasted, when administered Tablet T16/400/20 (20 mg dose of a 75:25 HPMCAS-M:Compound I dispersion) the average AUC/dose (AUC in (h x ng)/mL divided by the dose in mg/kg) was about 2798.

In dogs that had been fasted, when administered Tablet T17/400/20 (20 mg dose of a 75:25 HPMCAS-H:Compound I dispersion) the average AUC/dose (AUC in (h x ng)/mL divided by the dose in mg/kg) was about 1416.

In dogs that had been fasted, when administered Tablet T14/400/20 (20 mg dose of a 50:50 CAP:Compound I dispersion) the average estimated bioavailability (% F) was about 87.

In dogs that had been fasted, when administered Tablet T14/400/20 (20 mg dose of a 75:25 HPMCAS-M:Compound I dispersion) the average estimated bioavailability (% F) was about 99.

In dogs that had been fasted, when administered Tablet T14/400/20 (20 mg dose of a 75:25 HPMCAS-H:Compound I dispersion) the average estimated bioavailability (% F) was about 44.

### Example 17. Preparation of Tablets from Dispersion 25

Using protocols equivalent to those described in Example 15, two other strengths of Tablets (10 and 20 mg) with slightly different compositions have also been prepared. See Tables below:

| 20 mg Formulation | %/100 | |
|---|---|---|
| | Intragranular | Extragranular |
| 25:75 Compound I:HPMCAS (Dispersion 25) | 0.4 | 0 |
| MCC (Avicel PH102) | 0.255 | 0.145 |
| Lactose | 0.13 | 0 |
| CCS | 0.025 | 0.025 |
| Aerosil 200 | 0.005 | 0.005 |
| Mg Stearate | 0.005 | 0.005 |
| Total Intra/Extragranular | 0.82 | 0.18 |
| Total | | 1 |

10 mg formulation:

| | %100 | |
|---|---|---|
| | Intragranular | Extragranular |
| 25:75 Compound I:HPMCAS (Dispersion 25) | 0.2 | 0 |
| MCC (Avicel PH102) | 0.395 | 0.145 |
| Lactose | 0.19 | 0 |
| CCS | 0.025 | 0.025 |
| Aerosil 200 | 0.005 | 0.005 |
| Mg Stearate | 0.005 | 0.005 |
| Total Intra/Extragranular | 0.82 | 0.18 |
| Total | | 1 |

As explained *supra,* it is to be understood that pharmaceutically acceptable salts are included in these embodiments, even though the phrase "pharmaceutically acceptable salt" is not written.

## Claims

1. A solid dispersion of amorphous 1,1,1,3,3,3-hexafluoro-2-(((5-fluoro-2-(1-(2-fluorobenzyl)-5-(isoxazol-3-yl)-1H-pyrazol-3-yl)pyrimidin-4-yl)amino)methyl)propan-2-ol (Compound I) and a polymer carrier selected from hydroxypropylmethylcellulose acetate succinate (HPMCAS), hydroxypropylmethylcellulose phthalate (HPMCP), cellulose acetate phthalate (CAP), polyvinyl pyrrolidone (PVP), or a co-polymer of polyvinyl pyrrolidone; wherein the polymer is present in an amount of between about 60 % and about 95 % of the total weight of the solid dispersion.

2. The solid dispersion of claim 1 wherein the polymer carrier is HPMCAS.

3. The solid dispersion of claim 1 wherein the polymer carrier is CAP.

4. The solid dispersion of any one of claims 1 to 3, wherein the polymer is present in an amount of between 60 % and 90 % of the total weight of the solid dispersion; optionally wherein the polymer is present in an amount of (i) between about 70 % and about 90 % of the total weight of the solid dispersion; or (ii) between about 75 % and about 90 % of the total weight of the solid dispersion.

5. The solid dispersion of any one of claims 1 to 4, wherein said solid dispersion is obtained by spray-drying.

6. The solid dispersion of any one of claims 1-5, wherein Compound I is present in an amount of from about 5% by weight to about 40% by weight, optionally wherein Compound I is present in an amount of (i) from about 10 % by weight to about 30 % by weight; (ii) from about 10 % by weight to about 25 % by weight; (iii) from about 15% by weight to about 40% by weight; or (v) from about 20% by weight to about 30 % by weight.

7. The solid dispersion of any one of claims 1 to 6, wherein said solid dispersion of Compound I comprises less than 20 % crystalline Compound I, optionally wherein said solid dispersion of Compound I comprises less than 10 % crystalline Compound I or less than 5 % crystalline Compound I.

8. The solid dispersion of any one of claims 1 to 7, wherein the weight to weight ratio of Compound I to polymer is between 20:80 and 40:60, optionally wherein the weight to weight ratio of Compound I to polymer is 25:75.

9. A pharmaceutical composition comprising at least one pharmaceutically acceptable excipient and a solid dispersion according to any one of claims 1 to 8.

10. An oral dosage unit form, comprising a solid dispersion of any one of claims 1-8 or a pharmaceutical composition of claim 9.

11. The dosage unit form according to claim 10, wherein said dosage unit form is a tablet.

12. A solid dispersion of any one of claims 1 to 8, or a pharmaceutical composition of claim 9, or a dosage unit form of claims 10 or 11, for use in treating a disease, health condition or disorder, wherein the disease, health condition or disorder is selected from: renal disease, diabetic nephropathy, diabetic retinopathy, non-alcoholic steatohepatitis (NASH), hypertension, or heart failure.

13. The dispersion, composition or dosage unit form for use of claim 12, wherein the disease or disorder is diabetic nephropathy or diabetic retinopathy.

14. The dispersion, composition or dosage unit form for use according to claim 12, wherein the renal disease is selected from renal fibrosis, ischemic renal disease, renal failure, renal insufficiency, chronic kidney disease, optionally, wherein the renal failure is due to accumulation or deposition and tissue injury, progressive sclerosis or glomerulonephritis.

15. The dispersion, composition or dosage unit form for use according to claim 12, wherein the disease or disorder is hypertension, optionally wherein said hypertension is resistant hypertension.

## Patentansprüche

1. Feste Dispersion von amorphem 1,1,1,3,3,3-Hexafluor-2-(((5-fluor-2-(1-(2-fluorbenzyl)-5-(isoxazol-3-yl)-1H-pyrazol-3-yl)pyrimidin-4-yl)amino)methyl)propan-2-ol (Verbindung I) und einem Polymerträger, ausgewählt aus Hydroxypropylmethylcelluloseacetatsuccinat (HPMCAS), Hydroxypropylmethylcellulosephthalat (HPMCP), Celluloseacetatphthalat (CAP), Polyvinylpyrrolidon (PVP) oder einem Co-Polymer von Polyvinylpyrrolidon; wobei das Polymer in einer Menge zwischen etwa 60 % und etwa 95 % des Gesamtgewichts der festen Dispersion vorhanden ist.

2. Feste Dispersion nach Anspruch 1, wobei der Polymerträger HPMCAS ist.

3. Feste Dispersion nach Anspruch 1, wobei der Polymerträger CAP ist.

4. Feste Dispersion nach einem der Ansprüche 1 bis 3, wobei das Polymer in einer Menge zwischen 60 % und 90 % des Gesamtgewichts der festen Dispersion vorhanden ist; optional wobei das Polymer in einer Menge von (i) zwischen etwa 70 % und etwa 90 % des Gesamtgewichts der festen Dispersion; oder (ii) zwischen etwa 75 % und etwa 90 % des Gesamtgewichts der festen Dispersion vorhanden ist.

5. Feste Dispersion nach einem der Ansprüche 1 bis 4, wobei die feste Dispersion durch Sprühtrocknen erlangt wird.

6. Feste Dispersion nach einem der Ansprüche 1-5, wobei Verbindung I in einer Menge von etwa 5 Gewichtsprozent bis etwa 40 Gewichtsprozent vorliegt, optional wobei die Verbindung I in einer Menge (i) von etwa 10 Gewichtsprozent bis etwa 30 Gewichtsprozent; (ii) von etwa 10 Gewichtsprozent bis etwa 25 Gewichtsprozent; (iii) von etwa 15 Gewichtsprozent bis etwa 40 Gewichtsprozent; oder (v) von etwa 20 Gewichtsprozent bis etwa 30 Gewichtsprozent vorhanden ist.

7. Feste Dispersion nach einem der Ansprüche 1 bis 6, wobei die feste Dispersion von Verbindung I weniger als 20 % kristalline Verbindung I umfasst, optional wobei die feste Dispersion von Verbindung I weniger als 10 % kristalline Verbindung I oder weniger als 5 % kristalline Verbindung I umfasst.

8. Feste Dispersion nach einem der Ansprüche 1 bis 7, wobei das Gewichtsverhältnis von Verbindung I zu Polymer zwischen 20:80 und 40:60 ist, optional wobei das Gewichtsverhältnis von Verbindung I zu Polymer 25:75 ist.

9. Pharmazeutische Zusammensetzung, umfassend mindestens einen pharmazeutisch annehmbaren Hilfsstoff und eine feste Dispersion nach einem der Ansprüche 1 bis 8.

10. Orale Einheitsdarreichungsform, umfassend eine feste Dispersion nach einem der Ansprüche 1-8 oder eine pharmazeutische Zusammensetzung nach Anspruch 9.

11. Einheitsdarreichungsform nach Anspruch 10, wobei die Einheitsdarreichungsform eine Tablette ist.

12. Feste Dispersion nach einem der Ansprüche 1 bis 8 oder pharmazeutische Zusammensetzung nach Anspruch 9 oder Einheitsdarreichungsform nach Anspruch 10 oder 11 zur Verwendung beim Behandeln einer Krankheit, eines Gesundheitszustands oder einer Störung, wobei die Krankheit, der Gesundheitszustand oder die Störung ausgewählt ist aus: Nierenkrankheit, diabetische Nephropathie, diabetische Retinopathie, nichtalkoholische Steatohepatitis (NASH), Bluthochdruck oder Herzversagen.

13. Dispersion, Zusammensetzung oder Einheitsdarreichungsform zur Verwendung nach Anspruch 12, wobei die Krankheit oder Störung diabetische Nephropathie oder diabetische Retinopathie ist.

14. Dispersion, Zusammensetzung oder Einheitsdarreichungsform zur Verwendung nach Anspruch 12, wobei die Nierenkrankheit ausgewählt ist aus Nierenfibrose, ischämischer Nierenerkrankung, Nierenversagen, Niereninsuffizienz, chronischer Nierenkrankheit, optional wobei das Nierenversagen auf Akkumulation oder Ablagerung und Gewebeschädigung, fortschreitende Sklerose oder Glomerulonephritis zurückzuführen ist.

15. Dispersion, Zusammensetzung oder Einheitsdarreichungsform zur Verwendung nach Anspruch 12, wobei die Krankheit oder Störung Bluthochdruck ist, optional wobei der Bluthochdruck resistenter Bluthochdruck ist.

## Revendications

1. Dispersion solide de 1,1,1,3,3,3-hexafluoro-2-(((5-fluoro-2-(1-(2-fluorobenzyl)-5-(isoxazol-3-yl)-1H-pyrazol-3-yl)pyrimidin-4-yl)amino)méthyl)propan-2-ol amorphe (composé I) et d'un support polymère choisi parmi le succinate d'acétate d'hydroxypropylméthylcellulose (HPMCAS), le phtalate d'hydroxypropylméthylcellulose (HPMCP), le phtalate d'acétate de cellulose (CAP), la polyvinylpyrrolidone (PVP) ou un copolymère de polyvinylpyrrolidone ; dans laquelle le polymère est présent en une quantité comprise entre environ 60 % et environ 95 % du poids total de la dispersion solide.

2. Dispersion solide selon la revendication 1, dans laquelle le support polymère est le HPMCAS.

3. Dispersion solide selon la revendication 1, dans laquelle le support polymère est le CAP.

4. Dispersion solide selon l'une quelconque des revendications 1 à 3, dans laquelle le polymère est présent en une quantité comprise entre 60 % et 90 % du poids total de la dispersion solide ; éventuellement, dans laquelle le polymère est présent en une quantité (i) comprise entre environ 70 % et environ 90 % du poids total de la dispersion solide ; ou (ii) comprise entre environ 75 % et environ 90 % du poids total de la dispersion solide.

5. Dispersion solide selon l'une quelconque des revendications 1 à 4, dans laquelle ladite dispersion solide est obtenue par séchage par atomisation.

6. Dispersion solide selon l'une quelconque des revendications 1 à 5, dans laquelle le composé I est présent en une quantité allant d'environ 5 % en poids à environ 40 % en poids, éventuellement dans laquelle le composé I est présent en une quantité allant (i) d'environ 10 % en poids à environ 30 % en poids ; (ii) d'environ 10 % en poids à environ 25 % en poids ; (iii) d'environ 15 % en poids à environ 40 % en poids ; ou (v) d'environ 20 % en poids à environ 30 % en poids.

7. Dispersion solide selon l'une quelconque des revendications 1 à 6, dans laquelle ladite dispersion solide du composé I comprend moins de 20 % de composé I cristallin, éventuellement dans laquelle ladite dispersion solide du composé I comprend moins de 10 % de composé I cristallin ou moins de 5 % de composé I cristallin.

8. Dispersion solide selon l'une quelconque des revendications 1 à 7, dans laquelle le rapport poids/poids entre le composé I et le polymère est compris entre 20:80 et 40:60, éventuellement dans laquelle le rapport poids/poids entre le composé I et le polymère est de 25:75.

9. Composition pharmaceutique comprenant au moins un excipient pharmaceutiquement acceptable et une dispersion solide selon l'une quelconque des revendications 1 à 8.

10. Forme posologique orale unitaire, comprenant une dispersion solide selon l'une quelconque des revendications 1 à 8 ou une composition pharmaceutique selon la revendication 9.

11. Forme d'unité de dosage selon la revendication 10, dans laquelle ladite forme d'unité de dosage est un comprimé.

12. Dispersion solide selon l'une quelconque des revendications 1 à 8, ou composition pharmaceutique selon la revendication 9, ou forme d'unité de dosage selon les revendications 10 ou 11, pour une utilisation dans le traitement d'une maladie, d'un état de santé ou d'un trouble, la maladie, l'état de santé ou le trouble étant choisi parmi : la maladie rénale, la néphropathie diabétique, la rétinopathie diabétique, la stéatohépatite non alcoolique (NASH), l'hypertension, ou l'insuffisance cardiaque.

13. Dispersion, composition ou forme d'unité de dosage pour une utilisation selon la revendication 12, la maladie ou le trouble étant la néphropathie diabétique ou la rétinopathie diabétique.

14. Dispersion, composition ou unité de dosage pour une utilisation selon la revendication 12, la maladie rénale étant choisie parmi la fibrose rénale, la maladie rénale ischémique, la défaillance rénale, l'insuffisance rénale, la maladie rénale chronique, éventuellement, la défaillance rénale étant due à une accumulation ou à un dépôt et à une lésion tissulaire, à une sclérose progressive ou à une glomérulonéphrite.

15. Dispersion, composition ou forme d'unité de dosage pour une utilisation selon la revendication 12, la maladie ou le trouble étant l'hypertension, éventuellement l'hypertension résistante.
